(19)

Europäisches Patentamt
European Patent Office
Office européen des brevets

(11)     **EP 4 126 088 B1**

(12)                **EUROPEAN PATENT SPECIFICATION**

(45) Date of publication and mention
of the grant of the patent:
**30.07.2025  Bulletin 2025/31**

(51) International Patent Classification (IPC):
*A61L 27/00* *(2006.01)*     *A61F 2/02* *(2006.01)*
*B33Y 80/00* *(2015.01)*     *A61L 27/24* *(2006.01)*
*A61L 27/36* *(2006.01)*     *A61L 27/22* *(2006.01)*

(21) Application number: **21776713.6**

(22) Date of filing: **22.03.2021**

(52) Cooperative Patent Classification (CPC):
(C-Sets available)
**A61L 27/56; A61F 2/12; A61L 27/24; A61L 27/26;
A61L 27/3604; A61L 27/3633; A61L 27/3804;
B33Y 80/00;** A61L 2400/06; A61L 2430/04;
A61L 2430/34                                    (Cont.)

(86) International application number:
**PCT/IL2021/050321**

(87) International publication number:
**WO 2021/191897 (30.09.2021 Gazette 2021/39)**

(54) **COLLAGEN-BASED FORMULATIONS USABLE AS SOFT TISSUE FILLERS AND/OR IMPLANTS**

FORMULIERUNGEN AUF KOLLAGENBASIS ZUR VERWENDUNG ALS WEICHGEWEBEFÜLLER
UND/ODER IMPLANTATE

FORMULATIONS À BASE DE COLLAGÈNE UTILISABLES EN TANT QUE PRODUITS DE
COMBLEMENT ET/OU IMPLANTS POUR TISSU MOU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **22.03.2020   US 202062992998 P**

(43) Date of publication of application:
**08.02.2023   Bulletin 2023/06**

(73) Proprietor: **CollPlant Ltd.
7670104 Rehovot (IL)**

(72) Inventors:
• **ORR, Nadav
7680400 Mazkeret Batya (IL)**
• **STERN, Miriam
7175915 Modiln (IL)**
• **ZARKA, Revital
7680400 Mazkeret Batya (IL)**

(74) Representative: **Finetti, Claudia
Bugnion S.p.A.
Viale Lancetti, 17
20158 Milano (IT)**

(56) References cited:
**WO-A1-2018/225076      WO-A1-2018/225076
US-A1- 2017 021 058**

• **CHHAYA MOHIT P. ET AL: "Transformation of
Breast Reconstruction via Additive
Biomanufacturing", SCIENTIFIC REPORTS, vol.
6, no. 1, 1 September 2016 (2016-09-01), pages
28030, XP055862746, Retrieved from the Internet
<URL:https://www.nature.com/articles/
srep28030.pdf> DOI: 10.1038/srep28030**
• **MOHIT P. CHHAYA ET AL.: "Transformation of
Breast Reconstruction via Additive
Biomanufacturing", SCIENTIFIC REPORTS, vol.
6, no. 1, 15 June 2016 (2016-06-15), pages 28030,
XP055862746, Retrieved from the Internet
<URL:www.nature.com/scientificreports/> DOI:
10.1038/srep28030**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**(Cont. next page)**

- **A. LEE ET AL.: "3D bioprinting of collagen to rebuild components of the human heart", SCIENCE, vol. 365, no. 6452, 2 August 2019 (2019-08-02), pages 482 - 487, XP055862758, DOI: 10.1126/ science.aav9051**
- **M. MOHSENIA ET AL.: "Additive biomanufacturing of scaffolds for breast reconstruction", ADDITIVE MANUFACTURING, vol. 30, no. 2019, 4 September 2019 (2019-09-04), pages 100845, XP055862761, DOI: 10.1016/ j.addma.2019.00845**
- **A. LODE ET AL.: "Additive manufacturing of collagen scaffolds by three-dimensional plotting of highly viscous dispersions", BIOFABRICATION, vol. 8, no. 1, 1 March 2016 (2016-03-01), pages 015015, XP055862764, DOI: 10.1088/1758-5090/8/1/015015**

Remarks:

The complete document including Reference Table(s) and the Sequence Listing(s) can be downloaded from the EPO website

(52) Cooperative Patent Classification (CPC): (Cont.)

C-Sets
**A61L 27/26, C08L 29/04;**
**A61L 27/26, C08L 67/04;**
**A61L 27/26, C08L 71/02;**
**A61L 27/26, C08L 89/06**

**Description**

RELATED APPLICATION/S

This application claims the benefit of priority under 35 USC §119(e) of U.S. Provisional Patent Application No. 62/992,998 filed on March 22, 2020.

SEQUENCE LISTING STATEMENT

The ASCII file, entitled 86700SequenceListing.txt, created on March 22, 2021, comprising 49,152 bytes, submitted concurrently with the filing of this application is incorporated herein by reference.

FIELD AND BACKGROUND OF THE INVENTION

The present invention, in some embodiments thereof, relates to soft tissue repair and/or augmentation and, more particularly to collagen-based formulations usable for forming degradable scaffolds and soft tissue implants comprising same, and to collagen-based formulations which are usable as soft tissue fillers in combination with the degradable scaffolds.

Soft tissue implants of the generic type are used for example in the form of breast implants in the field of plastic surgery after breast amputations or in cosmetic surgery for breast augmentation. Further applications of soft tissue implants include, for example, calf muscle prostheses or cheek, nose, gluteal muscle, testicular or brachial muscle implants. Other examples of soft tissue augmentation opportunities include the face, the buttocks, depressed scar contours, or any other body deformity or area that is desirably augmented.

Elevated requirements, for example good biocompatibility, are placed on materials which are to be introduced into a human body. Particularly elevated requirements are placed on materials which are intended to remain (permanently or transiently) in the human body as implants. Medical implants have the function of supporting or replacing body functions, while in the case of plastic implants, the shape of body parts which may be destroyed is to be restored or changed.

Prosthetic implants have been developed for insertion below the skin. However, the severity of the potential complications including scarring, implant rupture, capsular contracture, necrosis and implant migration as well as the recent adverse publicity thereof have significantly reduced the desirability of these implants. While scientific evidence is not conclusive, there is some indication that breast implants might contribute to secondary diseases. Thus, there is a societal need for other means to obtain breast augmentation.

Mammoplasty (breast cosmetic surgery) includes augmentation, for increasing the size, form, and feel of breasts, or reconstruction following damage to breast tissues by trauma, disease (breast cancer), and anatomic deformations. In the United States breast augmentation is the top cosmetic surgical procedure performed and has been since 2006 (American Society of Plastic Surgeons, 2018 Plastic Surgery Statistics Report). The main types of breast implants in use are saline-filled and silicone gel-filled implants. The shell for both types of implants is manufactured from polysiloxane silicone rubber. Complications following breast augmentation include breast pain, altered sensation, impeded breast-feeding function, visible wrinkling, asymmetry, and thinning of the breast tissue.

One of the main safety concerns associated with breast implants is implant rupture. A rupture is a tear or hole in the outer shell of the breast implant and is not always noticeable. In saline filled breast implants, the breast deflates as the saline is absorbed by the body. In silicone-gel filled breast implants, the rupture is "silent", the gel can remain in the shell or within the scar tissue that forms around the implant, or the gel can move outside the scar tissue, and in some cases, may migrate outside the vicinity of the augmented or reconstructed breast.

Clinical complications from the leaked silicone filler-gel are usually manifested as granulomas (inflammatory nodules) and axillary lymphadenopathy (enlarged lymph glands in the armpit area) (Hölmich et al., (2004). Untreated Silicone Breast Implant Rupture Plastic and Reconstructive Surgery. 114 (1): 204-214, Katzin et al., (2005). Pathology of Lymph Nodes from Patients with Breast Implants: A Histologic and Spectroscopic Evaluation American Journal of Surgical Pathology. 29 (4): 506-11, FDA Breast Implant Consumer Handbook - *Study of Rupture of Silicone Gel-filled Breast Implants (MRI Component)* - 2004). Rupture rates increase the longer implants are in place. Overall, rupture rates are generally less than 5% before Year 4 and then increase around 4-6 years post-implanting. After Year 6, the rupture rates continue to increase at variable rates. Rupture is resolved by explantation (surgical removal) of the implant.

In 2016, the World Health Organization (WHO) designated breast implant-associated anaplastic large cell lymphoma (BIA-ALCL) as a T-cell lymphoma that can develop from breast implants (Swerdlow et al., (2016) The 2016 revision of the World Health Organization classification of lymphoid neoplasms. Blood, 127(20), 2375-2390). Clinically, BIA-ALCL typically originates in the capsule around breast implants and presents as a fluid collection or tumor adjacent to the implant surface (FDA Executive Summary Breast Implant Special Topics Prepared for the Meeting of the General and Plastic Surgery Devices Advisory Panel, March 25-26, 2019). As of July 6, 2019, the Food and Drug Administration (FDA) has received a total of 573 US and global medical device reports (MDRs) of BIA-ALCL, including 33 deaths ("Medical Device Reports of Breast Implant-Associated Anaplastic Large Cell Lymphoma", www(dot)fda(dot)gov(dot) as of 24 July 2019).

Collagen comprises the main component of connective tissue and is the most abundant protein in mammals, comprising approximately 30 % of the proteins found in the body. Collagen serves as the predominant component and primary structural-mechanical determinant of most tissue extracellular matrix (ECM) [see, for example, Kadler K. Birth Defects Res C Embryo Today. 2004; 72:1-11; Kadler KE, Baldock C, Bella J, Boot-Handford RP. J Cell Sci. 2007; 120:1955-1958.; Kreger ST. Biopolymers. 2010 93(8): 690-707].

Due to its unique characteristics and diverse profile in human body functions, collagen is frequently selected from a variety of biocompatible materials for use in tissue repair to support structural integrity, induce cellular infiltration and promote tissue regeneration. Among the 5 major collagen types, Type I collagen is the most abundant form of in the human body. Collagen's unique properties make it a favorite choice for regenerative medicine products.

Additive manufacturing (AM) is generally a process in which a three-dimensional (3D) object is manufactured utilizing a computer model of the objects. The basic operation of any AM system consists of slicing a three-dimensional computer model into thin cross sections, translating the result into two-dimensional position data and feeding the data to control equipment which manufacture a three-dimensional structure in a layerwise manner.

Various AM technologies exist, amongst which are stereolithography, digital light processing (DLP), and three-dimensional (3D) printing such as 3D inkjet printing. Such techniques are generally performed by layer by layer deposition and hardening (e.g., solidification) of one or more building materials, which typically include photopolymerizable (photocurable) materials.

Stereolithography, for example, is an additive manufacturing process which employs a liquid ultraviolet (UV)-curable building material and a UV laser. In such a process, for each dispensed layer of the building material, the laser beam traces a cross-section of the part pattern on the surface of the dispensed liquid building material. Exposure to the UV laser light cures and solidifies the pattern traced on the building material and joins it to the layer below. After being built, the formed parts are immersed in a chemical bath in order to be cleaned of excess building material and are subsequently cured in an UV oven.

In three-dimensional printing processes, for example, a building material is dispensed from a dispensing head having a set of nozzles to deposit layers on a supporting structure. Depending on the building material, the layers may then be cured or solidify using a suitable device.

The building materials may include modeling material formulation(s) and support material formulation(s), which form, upon hardening, the object and the temporary support constructions supporting the object as it is being built, respectively.

The modeling material formulation(s) is/are deposited to produce the desired object and the support material formulation(s) is/are used, with or without modeling material elements, to provide support structures for specific areas of the object during building and assure adequate vertical placement of subsequent object layers, e.g., in cases where objects include overhanging features or shapes such as curved geometries, negative angles, voids, and so on.

Both the modeling and support materials are preferably liquid at the working temperature at which they are dispensed, and subsequently hardened, typically upon exposure to hardening or curing condition such as curing energy (e.g., UV curing), to form the required layer shape. After printing completion, support structures, if present, are removed to reveal the final shape of the fabricated 3D object. The hardening (curing) of the dispensed materials typically involves polymerization (e.g., photopolymerization) and/or crosslinking (e.g., photocrosslinking).

Additive manufacturing has been first used in biological applications for forming three-dimensional sacrificial resin molds in which 3D scaffolds from biological materials were created.

3D bioprinting is an additive manufacturing methodology which uses biological materials, optionally in combination with chemicals and/or cells, that are printed layer-by-layer with a precise positioning and a tight control of functional components placement to create a 3D structure.

Three dimensional (3D) bioprinting is gaining momentum in many medicinal applications, especially in regenerative medicine, to address the need for complex scaffolds, tissues and organs suitable for transplantation.

Inherent to 3D printing in general is that the mechanical properties of the printing media (the dispensed building material) are very different from the post-printed cured (hardened) material.

To allow tight control on the curing (e.g., polymerization) after printing, the building material commonly includes polymerizable (e.g., photopolymerizable) moieties or groups that polymerize (e.g., by chain elongation and/or cross-linking) upon being dispensed, to preserve the geometric shape and provide the necessary physical and/or mechanical properties of the final product.

Different technologies have been developed for 3D bioprinting, including 3D Inkjet printing, Extrusion printing, Laser-assisted printing and Projection stereolithography [see, for example, Murphy SV, Atala A, Nature Biotechnology. 2014 32(8).; Miller JS, Burdick J. ACS Biomater. Sci. Eng. 2016, 2, 1658-1661]. Each technology has its different requirements for the dispensed building material (also referred to herein as printing media), which is derived from the specific application mechanism and the curing/gelation process required to maintain the 3D structure of the scaffold post printing.

For all technologies, the most important parameter determining the accuracy and efficiency of the printing is the static and dynamic physical properties of the dispensed building material, including viscosity, shear thinning and thixotropic properties. The static and dynamic properties of the building material are important not only for the printing technology but also when considering cell-laden printing, i.e. including cells in the building material dispensed during printing. In this case, the shearing forces applied to the building material during printing (dispensing) have a significant effect on the survival of the cells. Therefore, it is desirable to have good control on the specific properties of the printing media over a wide range of conditions, i.e. concentration, temperature, ionic strength and pH.

Type I collagen is considered a good candidate for use as a major component of a building material in 3D-bioprinting.

Collagen methacrylate can be used as a rapidly self-assembling type I collagen to form cross-linked hydrogels for tissue engineering [see, for example, Isaacson et al., Experimental Eye Research 173, 188-193 (2018)]. It has been used with mesenchymal stem cells [Kathryn E. Drzewiecki et al., A thermoreversible, photocrosslinkable collagen bio-ink for free-form fabrication of scaffolds for regenerative medicine, Technology (2017)], fibroblasts, adipose derived stem cells, epithelial cells, and many more. Collagen methacrylate is useful for forming scaffolds with varying degree of stiffness, by altering collagen concentration or the curing conditions (e.g., intensity and duration of irradiation).

Collagen methacrylate extracted from tissues has been characterized for its usefulness in 3D-bioprinting (extrusion, inkjet, and photolithographic [Drzewiecki, K. E. et al. Langmuir 30, 11204-11211 (2014); Gaudet, I. D. & Shreiber, D. I. Biointerphases 7, 25 (2012)].

Despite the significant advantages offered by this natural polymer, a number of factors hinder its use for 3D bioprinting. The use of tissue extracted collagen for this purpose is limited due to its sensitivity to temperature and ionic strength, which leads to spontaneous gel formation at temperatures higher than 20 °C, under physiological conditions [see, for example, PureCol, Advanced BioMatrix, Inc.]. The typical temperature-dependent formation of gel of tissue extracted-collagens hampers significantly the precise fluidity during printing. Keeping the printing media at low temperature until application is a possible solution for this phenomena but implies a serious technical limitation. Another solution is the use of gelatin, the denatured form of collagen which does not become gel-like under these conditions. However, gelatin lacks the genuine tissue and cell interactions of native collagen and thus crucial biological functions are lost.

The present assignee has developed a technology that allows the purification of naive human Type I collagen (rhCollagen) by introducing into tobacco plants, five human genes encoding heterotrimeric type I collagen [see, for example, Stein H. (2009) Biomacromolecules; 10:2640-5]. The protein is purified to homogeneity through a cost-effective industrial process taking advantage of collagen's unique properties. See also WO 2006/035442, WO 2009/053985, WO 2011/064773, WO 2013/093921, WO 2014/147622.

WO 2018/225076, by the present assignee, describes curable recombinant human collagen, and kits comprising same, which are usable in preparing modeling material formulations for additive manufacturing (e.g., 3D bioprinting) of 3D objects, and methods utilizing such modeling material formulations in additive manufacturing of 3D objects having a collagen-based material in at least a portion thereof. The formulations feature a desired viscosity at a temperature higher than 10 °C (e.g., room temperature or 37 °C) and allow performing the additive manufacturing without cooling the system or a part thereof.

WO 2019/211854, by the present assignee, describes photocurable (photoinitiated) dermal fillers, hyaluronic acid-rhCollagen double cross-linked dermal fillers and hyaluronic acid-rhCollagen semi interpenetrated network, each comprising plant-derived human collagen, as well as methods of using the same.

Additional background art includes U.S. Patent Nos. 5,591,444, 7,723,108, 7,745,105, 7,919,112, 8,025,869, 8,038,665, 8,066,691, 8,124,120, 8,142,815, 8,192,487, 8,435,600, 8,546,142, 8,702,684, 8,641,775, 8,642,735, 8,697,059, 8,992,551, 8,758,781, 8,778,333, 8,778,909, 8,871,267, 9,101,692, 9,074,190, 9,173,975, 9,150,668, 9,289,533, 9,956,317, 9,681,941, 9,752,138, 9,744,260, 9,782,517, 9,801,976, 9,901,440, 9,913,705, 9,956,072, 10,011,820, 10,258,588, 10,117,822, 10,039,633, 10,300,169, 10,335,190, 10,327,884, 10,449,034, 10,471,181; DE Patent No. 102011121982; RU Patent No. 2675019; EP Patent Application No. 2995278; EP Patent Nos. 3013379, 2231061, 1280562, 3247413, 1814606, 2550028, 2841115, 1734894, 3191020, 1546307, 3357519; and U.S. Patent Applications Publication Nos. 2017/0274052, 2011/0274666, 2018/0064854, 2020/0078411, 2020/0030495, 2019/0321158, 2018/0177917, 2019/0184064, 2017/0071725, 2020/0030496, 2019/0060516, 2020/0016191, 2019/0374457, 2018/0193522, 2018/0098836, 2017/0224896, 2019/0134265, 2017/0087273, 2018/0289860, 2018/0015204, 2020/0268503 and 2021/0030528.

CHHAYA MOHIT P. ET AL: "Transformation of Breast Reconstruction via Additive Biomanufacturing", SCIENTIFIC REPORTS, vol. 6, no. 1, 1 September 2016 (2016-09-01), page 28030, DOI: 10.1038/ srep28030, URL:https://www. nature.com/articles/srep28030.pdf discloses a 3D soft tissue implant comprising a porous scaffold and an inner cavity that is formed by additive biomanufacturing using medical-grade polylcaprolactone.

SUMMARY OF THE INVENTION

The present inventors have now devised formulations containing rhCollagen usable for forming degradable implants, which are suitable for use in soft tissue augmentation, reconstruction and/or regeneration procedures. The disclosed implants are be designed while controlling their mechanical, physical and/or biological properties so as to suit the intended use, and can be utilized in a wide variety of applications as described in further detail hereinbelow, including reconstruction or augmentation of a breast tissue in a subject. The scope of this invention is defined by the claims. Any references in the description to methods of treatment refer to the products of the present invention for use in a method for treatment.

According to an aspect of some embodiments of the present invention there is provided a three-dimensional (3D) biocompatible, degradable soft tissue implant according to claim 1.

According to some of any of the embodiments described herein, the scaffold further comprises at least one printed vascular network path that connects the outer most surface of the scaffold with the inner cavity of the scaffold, wherein the vascular network path is sized to permit entry of vascular cells and tissues.

According to some of any of the embodiments described herein, the scaffold comprises between about 1 to 1,000 printed vascular network paths.

According to some of any of the embodiments described herein, a total volume of the inner cavity is from about 5 ml to about 300 ml, or from about 10 ml to about 300 ml, or from about 50 ml to about 300 ml.

According to some of any of the embodiments described herein, the inner cavity comprises at least one chamber.

According to some of any of the embodiments described herein, the inner cavity comprises from 2 to 30 chambers.

According to some of any of the embodiments described herein, at least two of the chambers are interconnected to one another.

According to some of any of the embodiments described herein, the scaffold comprises between 1 to 15 injection ports.

The bio-printed composite scaffold is formed by bio-printing a curable formulation in a configured pattern which corresponds to a desired shape and dimension of the soft tissue implant, wherein the curable formulation comprises a recombinant human collagen (rhCollagen) that features a curable moiety.

The curable formulation further comprises a biocompatible synthetic polymer that features a curable moiety.

According to some of any of the embodiments described herein, a curable moiety of the rhCollagen and a curable moiety of the synthetic polymer are curable when subjected to the same curing condition.

According to some of any of the embodiments described herein, each of the curable moieties is a photocurable moiety.

According to some of any of the embodiments described herein, the biocompatible synthetic polymer comprises polylactic acid (PLA), polyglycolic acid (PGA), polycaprolactone (PCL), poly (lactic-co-glycolic acid) (PLGA), polyethylene glycol (PEG), polyvinyl alcohol (PVA), poly(N-isopropylacrylamide) (PNIPAAm), poly-4-hydroxybutyrate (P4HB) or any copolymer thereof.

According to some of any of the embodiments described herein, the rhCollagen comprises a plant-derived recombinant human collagen.

According to some of any of the embodiments described herein, a ratio of the rhCollagen to the biocompatible synthetic polymer is from about 1:1 to about 1:20, or from about 1:1 to 1:10, or from about 1:2 to 1:10.

According to some of any of the embodiments described herein, the scaffold further comprises at least one extracellular matrix (ECM) component.

According to some of any of the embodiments described herein, the formulation comprises at least one ECM component that features a curable moiety.

According to some of any of the embodiments described herein, the at least one ECM component comprises at least one of hyaluronic acid, fibronectin, heparin, elastin, laminin, and any combination thereof.

According to some of any of the embodiments described herein, the scaffold further comprises an integrin-binding material.

According to some of any of the embodiments described herein, the integrin-binding material is an RGD-containing material.

According to some of any of the embodiments described herein, the formulation comprises an integrin-binding moiety that features a curable moiety.

The implant further comprises a matrix at least within the inner cavity of the scaffold, the matrix comprising rhCollagen optionally in combination with one or more additional components. and cells or adipose tissue.

According to some of any of the embodiments described herein, the at least one additional ECM component comprises hyaluronic acid (HA), fibronectin, heparin, elastin, or laminin, or any combination thereof.

According to some of any of the embodiments described herein, the rhCollagen is comprises at least one of a cross-linked fibrillar rhCollagen and rhCollagen-derived nanoparticles.

According to some of any of the embodiments described herein, the matrix further comprises an integrin-binding material.

According to some of any of the embodiments described herein, a weight ratio between the ECM component and the cells or adipose tissue in the matrix ranges from 1:1 to 1:5.

According to some of any of the embodiments described herein, the cells comprise pericytes, adipose derived stem cells, pre-adipocytes, endothelial cells, progenitor cells, hematopoietic cells, adipocytes, or any combination thereof.

According to some of any of the embodiments described herein, the cells comprise a stromal vascular fraction (SVF) isolated from a fat tissue.

According to some of any of the embodiments described herein, the cells comprise a minimally processed extract from a fat tissue.

According to some of any of the embodiments described herein, a volume of the matrix is from about 5 ml to about 300 ml.

According to some of any of the embodiments described herein, the soft tissue implant is a breast implant.

According to some of any of the embodiments described herein, the soft tissue is selected from a breast tissue, a facial tissue, a neck tissue, a muscle tissue, a joint tissue, a jaw tissue, a buttock tissue, a hand tissue, and a chest tissue.

According to an aspect of some embodiments of the present invention there is provided a method of preparing the implant as described herein in any of the respective embodiments and any combination thereof, the method comprising dispensing at least one formulation to sequentially form a plurality of layers in the configured pattern of the scaffold, wherein for at least a portion of the layers, the dispensing is of a formulation that comprises the recombinant human collagen featuring at least one curable group, the synthetic polymer featuring at least one curable group, and rhCollagen featuring the curable group and optionally the integrin-binding material featuring at least one curable material.

The method further comprises injecting to at least within the inner cavity of the scaffold, via the injection port, a matrix as defined herein in any of the respective embodiments.

According to an aspect of some embodiments of the present invention there is provided a kit comprising at least one formulation for forming as scaffold as described herein any of the respective embodiments; and an injectable matrix formulation for forming a matrix as described herein in any of the respective embodiments, the kit being identified for use in preparing a soft tissue implant.

According to an aspect of some embodiments of the present invention there is provided a soft tissue implant as described herein in any of the respective embodiments, for use in augmenting and/or reconstructing and/or regenerating a soft tissue in a subject in need thereof.

According to some of any of the embodiments described herein, injecting the matrix is performed subsequent to the implanting, and is optionally performed repetitively.

According to some of any of the embodiments described herein, a volume of the matrix is from about 5 ml to about 300 ml.

According to some of any of the embodiments described herein, following the implanting, the printed vascular network path is anastomosed with at least one of the subject's blood vessels.

According to an aspect of some embodiments ( not according to the claimed invention) there is provided an injectable matrix formulation comprising: at least one extracellular matrix (ECM) component; and cells or adipose tissue, or a combination thereof, wherein the at least one ECM component comprises rhCollagen; and the cells comprise pericytes, adipose derived stem cells, pre-adipocytes, endothelial cells, progenitor cells, hematopoietic cells, or adipocytes, or any combination thereof, and/or a fat fraction isolated from a fat tissue.

According to some of any of the embodiments described herein, the cells comprise a stromal vascular fraction (SVF) isolated from a fat tissue.

According to some of any of the embodiments described herein, the cells comprise a minimally processed fraction isolated from a fat tissue.

According to some of any of the embodiments described herein, the rhCollagen comprises a plant-derived human collagen.

According to some of any of the embodiments described herein, a weight ratio of the ECM component to the cells or adipose tissue is from about 5:1 to about 1:5.

According to some of any of the embodiments described herein, the formulation further comprises an integrin-binding material.

According to some of any of the embodiments described herein, the integrin binding material is an RGD-containing material.

According to some of any of the embodiments described herein, the soft tissue comprises the face, nose, jaw, breast, chin, buttocks, hands, muscle, joint, legs, feet, chest, lip, or cheek tissue, or any combination thereof.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

BRIEF DESCRIPTION OF THE SEVERAL VIEWS OF THE DRAWING(S)

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

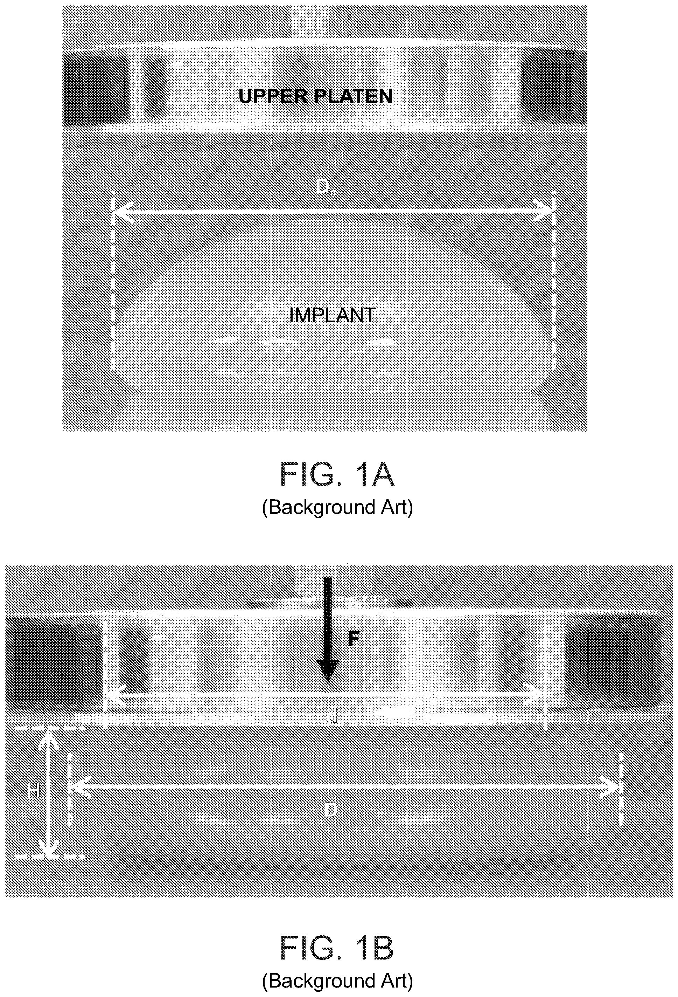

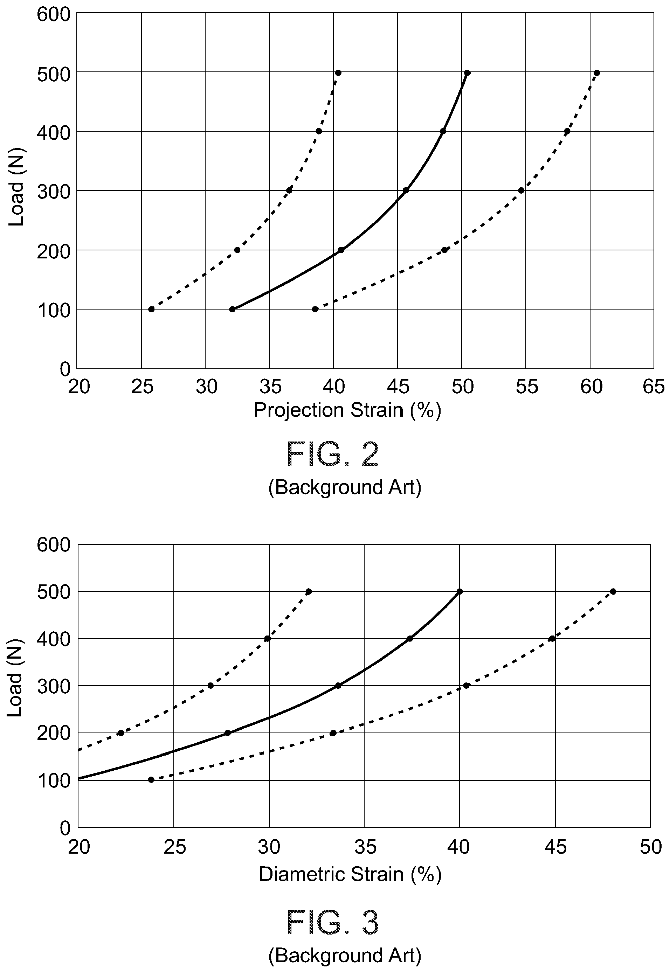

FIGs. 1A-B (Background Art) show an example of a compression test between two parallel plates used for characterizing implant mechanical properties (adopted from Brandon et al. (2019) Bioengineering (Basel).6(2):43, Figures 2A and 2B). FIG. 1A shows images of an implant between plates prior to the compression testing. FIG. 1B shows the same implant undergoing compression testing. ($D_o$ = Diameter (cm) of the implant prior to compression at time 0; D = Diameter of the implant; d = Contact diameter; H = Implant projection (plate spacing) between the plates; F = Applied force).

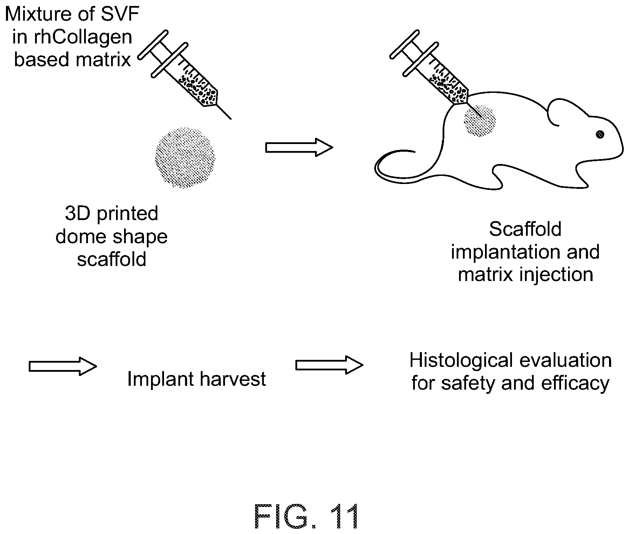

FIG. 2 (Background Art) shows an exemplary graphic presentation of an analysis testing load as a function of projection strain (adopted from Brandon et al. (2019) Bioengineering (Basel).6(2):43, Figure 11). The change in implant projection, the displacement of the plates, is measured in response to compression load.

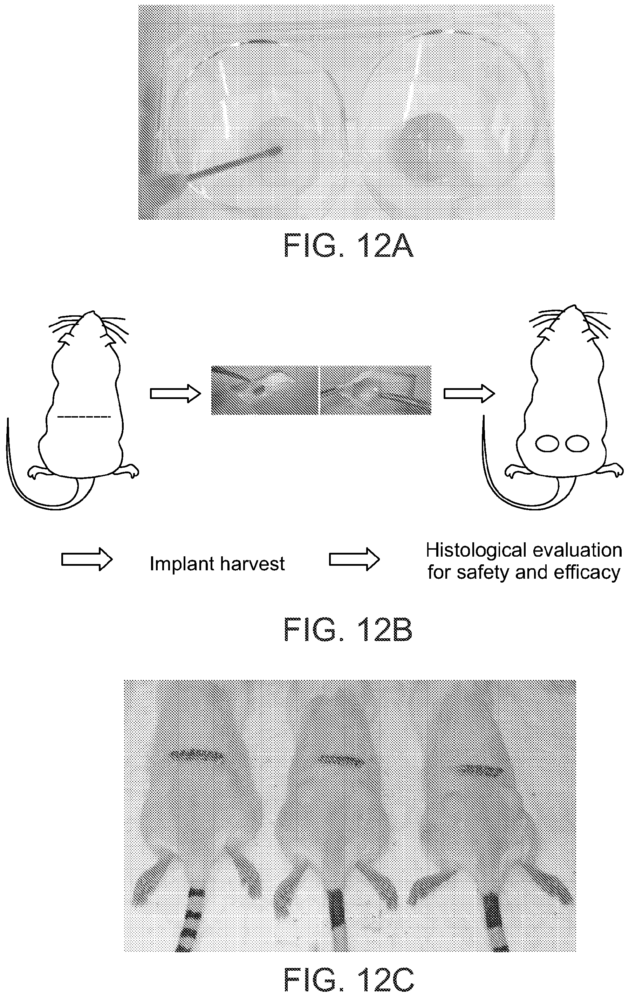

FIG. 3 (Background Art) shows an exemplary graphic presentation of an analysis testing load as a function of diametric strain (adopted from Brandon et al. (2019) Bioengineering (Basel).6(2):43, Figure 12). The change in implant diameter is measured in response to compressive load.

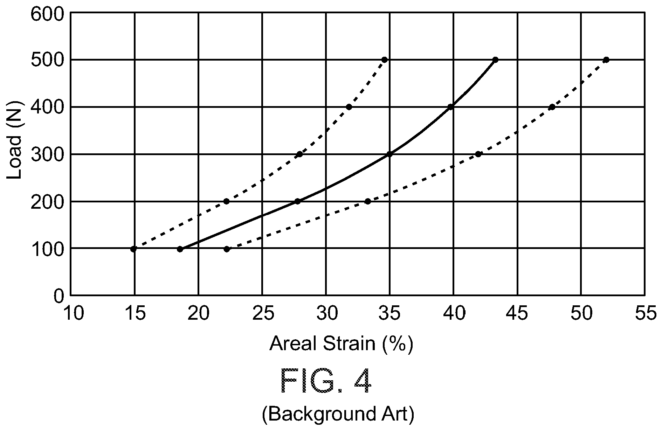

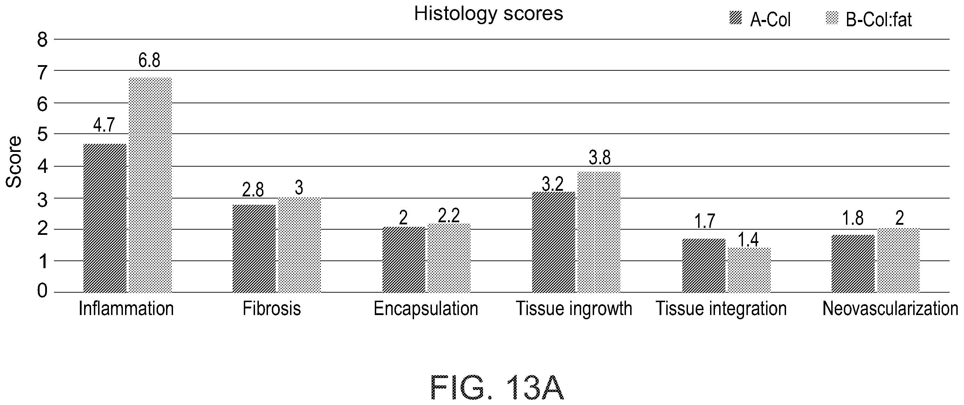

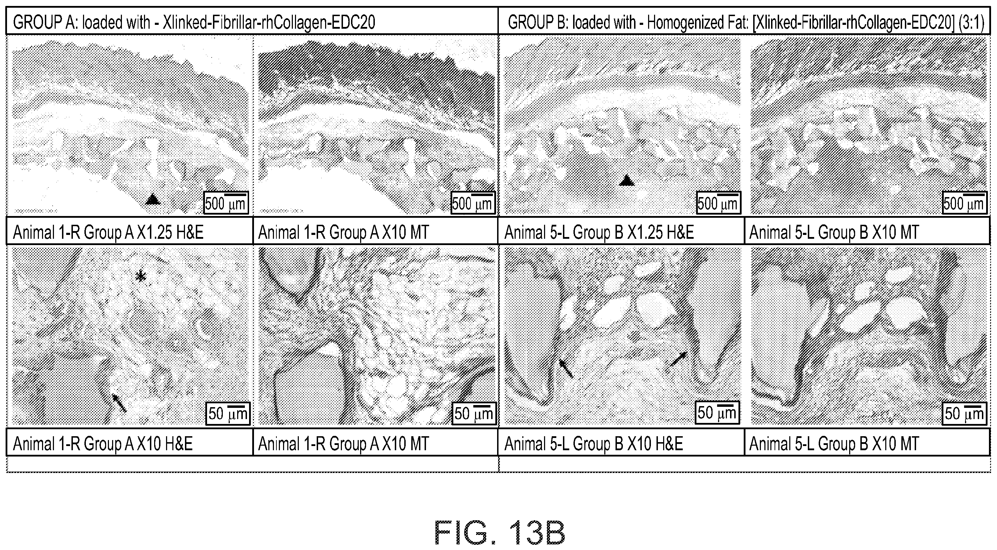

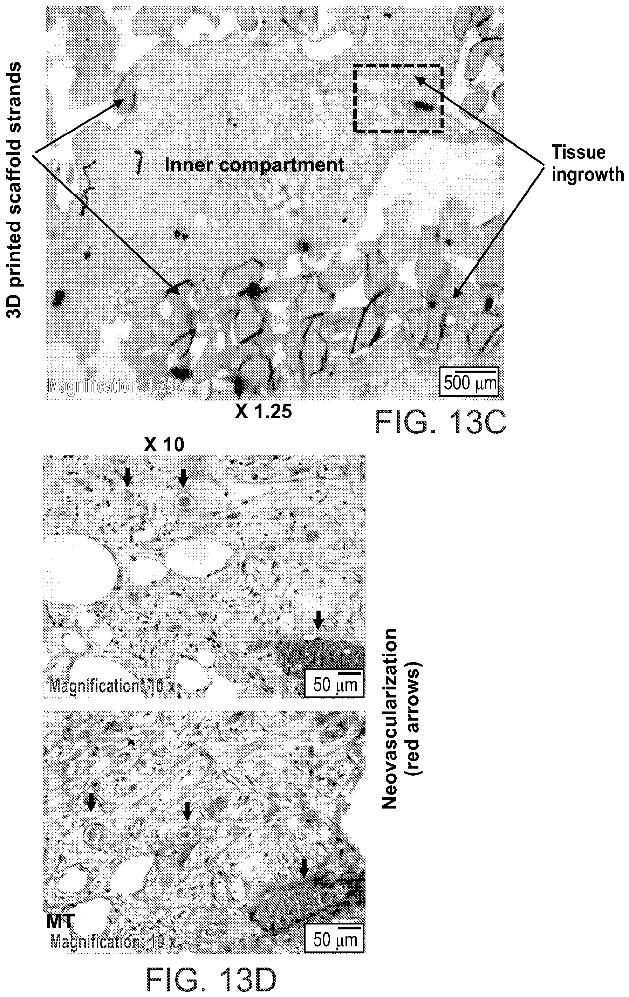

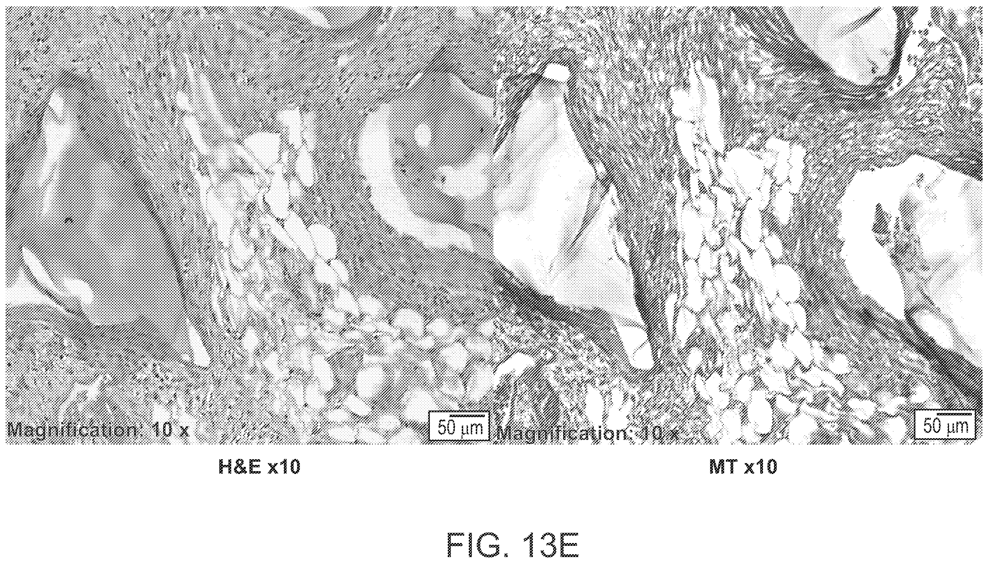

FIG. 4 (Background Art) shows an exemplary graphic presentation of an analysis testing load as a function of areal strain (adopted from Brandon et al. (2019) Bioengineering (Basel).6(2):43, Figure 13). The change in implant surface area is measured in response to compressive load.

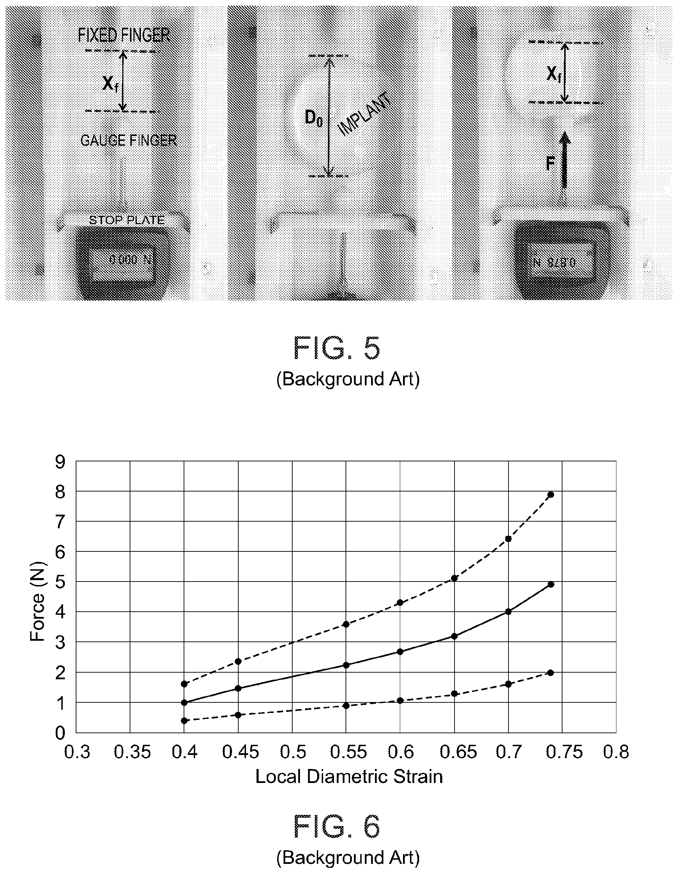

FIG. 5 (Background Art) presents images of an exemplary set up to analyze localized strain at very low compressive loads ("Pinch" tests (adopted from Brandon et al. (2019) Bioengineering (Basel).6(2):43, Figure 17). Implant deformation due to unconfined, low, and localized compressive forces is applied at the perimeter of the implant. The left-most image shows the set up for $X_f$. The middle image shows implant placement within the setup, while the right most image shows an implant pinched for force measurement. (Stop = a pre-defined stop; Fixed Finger and Gauge Finger are silicon probes simulating fingertips; $D_0$ = the initial implant diameter; $X_f$ = final pinch distance; F = pinch force).

FIG. 6 (Background Art) shows an exemplary graphic presentation of results obtained in "Pinch" tests (adopted from Brandon et al. (2019) Bioengineering (Basel).6(2):43, Figure 18). The results show implant deformation due to unconfined, low, and localized compressive forces applied at the perimeter of the implant by the "fingers".

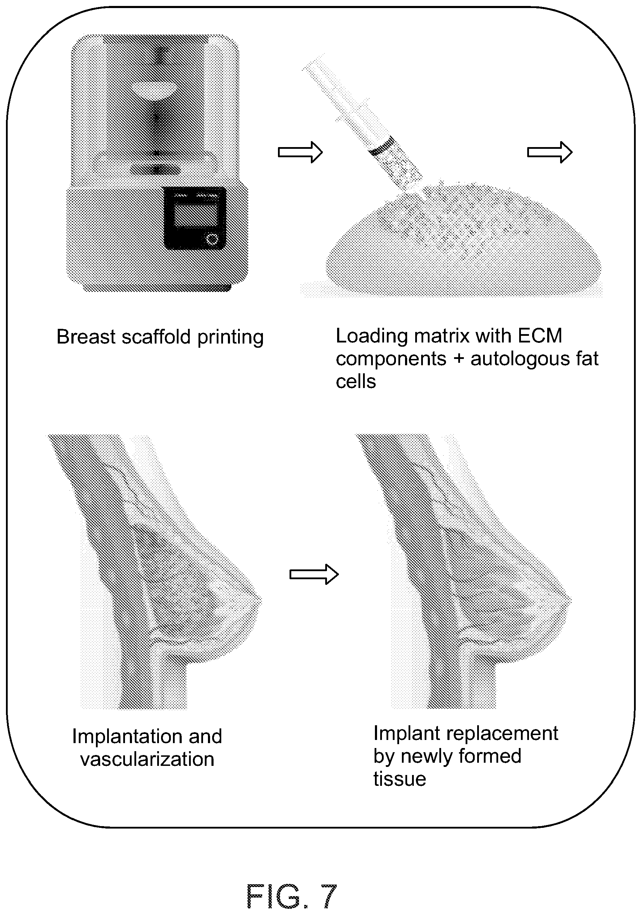

FIG. 7 presents a schematic illustration of producing and using a 3D bio-printed, breast implant comprising a degradable scaffold according to exemplary embodiments of the present invention. A degradable breast implant scaffold is prepared by bio-printing (upper left object), and a matrix comprising an ECM component, such as rhCollagen, optionally combined with stromal vascular fraction (SVF), homogenized fat extract and/or cellular components, is injected into the degradable scaffold (upper right object). The ECM components may, in some embodiments, comprise hyaluronic acid (HA), fibronectin, heparin, or laminin, or any combination thereof. The matric-loaded scaffold in then implanted and vascularized in the body (lower left object). A gradual degradation of the implant scaffold together with the gradual replacement by a new tissue, comprising new tissue regeneration and vascularization, is thereafter effect (lower right object). At this final stage, the scaffold disappears, leaving a fully functional vascularized tissue.

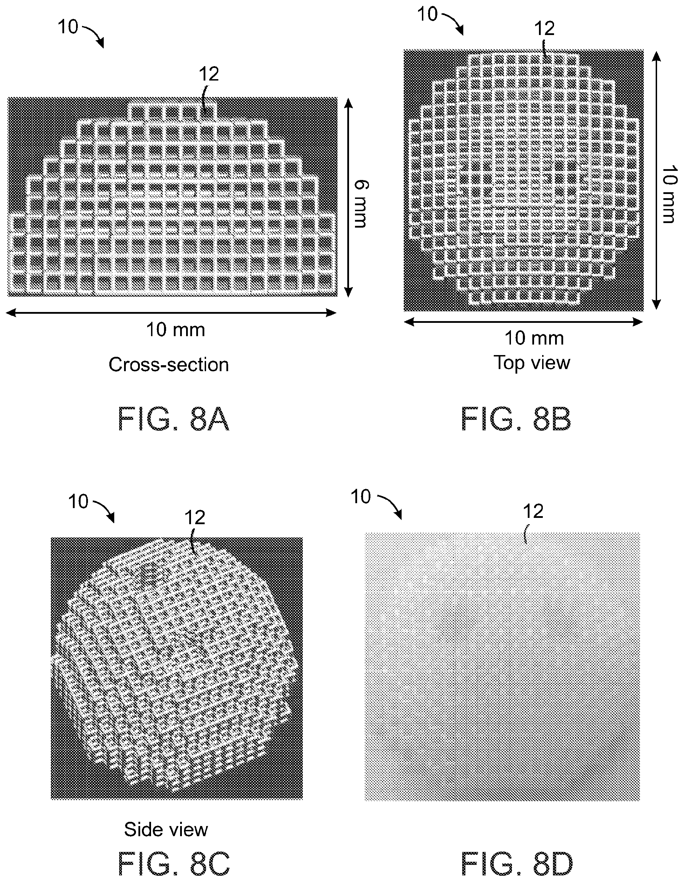

FIGs. 8A-D present images of a cross-section (FIG. 8A), top-view (FIG. 8B), and side-view (FIG. 8C) of a dome-shaped, 10 mm x 10 mm x 6 mm, 3D-printed degradable scaffold according to exemplary embodiments of the present invention, comprising 0.5 mm x 0.5 mm pores. The two depressions observed in the top-view and side-view are injection ports where a matrix as described herein may be loaded, prior to or post implanting or both before and after implanting. FIG. 8D shows a 3D bio-printed degradable scaffold comprising methacrylated rhCollagen (0.5 %), PEG-DA (0.5-2 %), SR9035 (1.0-2.5 %), HEAA (12.0-40.0 %).

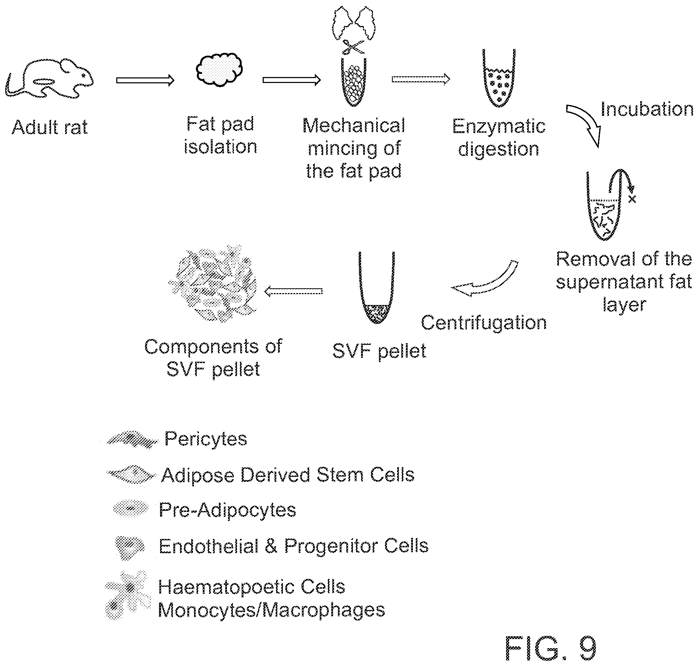

FIG. 9 presents a schematic illustration of an exemplary method of isolation of functional cells of the stromal vascular fraction (SVF) that may be combined with an ECM matric (e.g., rhCollagen-containing matrix) and loaded into the degradable scaffold. The steps shown include isolating fat pads from a subject (an adult rat), mechanically mincing the fat pad and enzymatic digestion of the tissue to remove the fat component, and then collecting the SVF that may include pericytes, adipose derived stem cells, pre-adipocytes, endothelial and progenitor cells, and/or haemopoietic cells including monocytes and macrophages. In some embodiments, the isolation procedure is stopped by mechanical mincing/homogenization of the fat pad, without enzymatic digestion, to obtain a minimally processed homogenized fat extract.

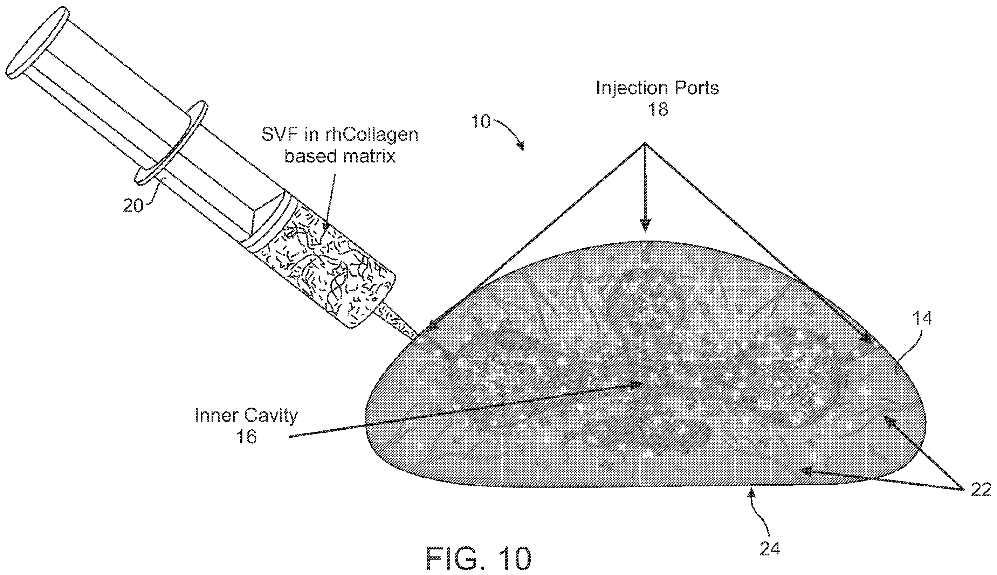

FIG. 10 presents a schematic depiction of an exemplary 3D bio-printed scaffold loaded with SVF and rhCollagen based matrix, according to exemplary embodiments of the present invention, prior to being implanted in a subject in need for down-stream regeneration of breast tissue. Loading the scaffold is effected by injection at an injection port as shown. The exemplary scaffold comprises 3 injection ports in (e.g., fluid) communication with the inner cavity. The bio-printed scaffold additionally comprises printed vascular network paths distributed around the scaffold, with openings at the scaffolds edge.

FIG. 11 presents a schematic presentation of exemplary animal (rat/mouse) studies, for evaluating the safety and efficacy of an implanted scaffold loaded with a matrix comprising at least one ECM component, such as rhCollagen, and a fat extract (VSF). A 3D printed dome shaped scaffold is implanted in the animal, a syringe is loaded with the matrix and the matrix injected to the implanted scaffold. The implant is harvested at pre-determined times post-implantation, and histologically evaluated for safety and efficacy.

FIGs. 12A-C present a schematic illustration and photographs showing another schematic presentation of exemplary animal (rat/mouse) studies, for evaluating the safety and efficacy of an implanted scaffold loaded with a matrix comprising at least one ECM component, such as rhCollagen, and a fat extract (minimally processed homogenized fat extract). The scaffold is loaded into the matrix (FIG. 12A) and is thereafter implanted (FIG. 12B), as shown in photographs of representative animals post-implantation (FIG. 12C).

FIGs. 13A-B present a bar graph showing the histological scoring of the tested implants, 4-weeks after implantation in rats (FIG. 13A), and representative histological images of both implant types with examples of tissue response (FIG. 13B). Neovascularization is shown by wide arrows, connective tissue proliferation around the printed strands is shown by regular arrows, triangles represent tissue ingrowth and fatty infiltration is shown by asterisk.

FIGs. 13C-E present additional representative histological images of both implant types with examples of tissue response. FIG. 13C demonstrate the tissue ingrowth observed in the inner, previously empty, compartment of the scaffold and between the scaffold strands. FIG. 13D further show the neovascularization wide arrows. FIG. 13E presents representative images showing neovascularized tissue ingrowth and fatty infiltration in-between the scaffold strands.

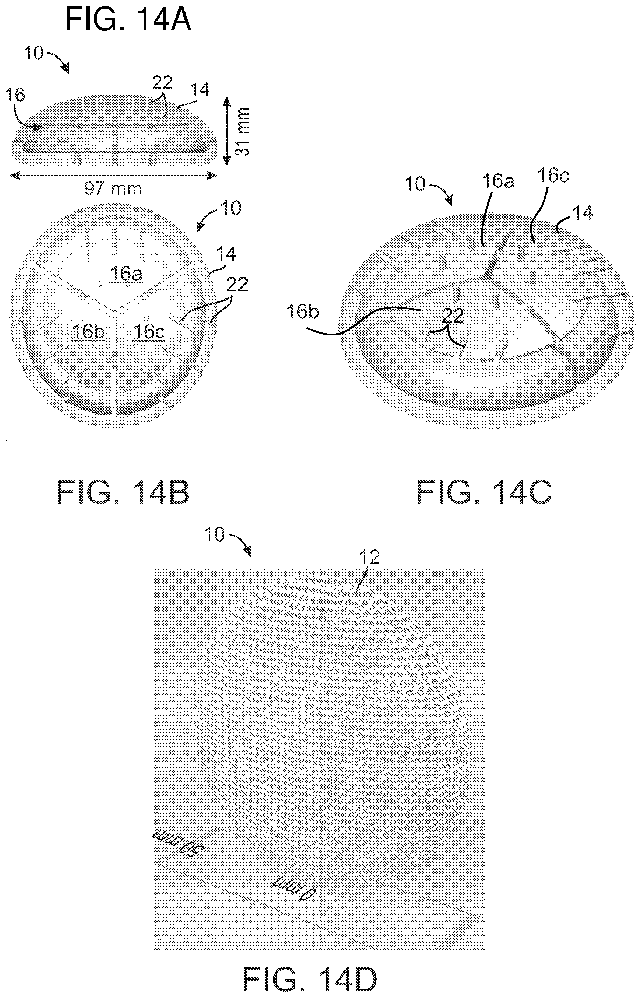

FIGs. 14A-D present images of an exemplary design of the scaffold according to some embodiments of the present invention, designed to enhance tissue ingrowth. The 3D scaffold features several interconnected inner compartments, and thin channels connecting the inner space to the surface.

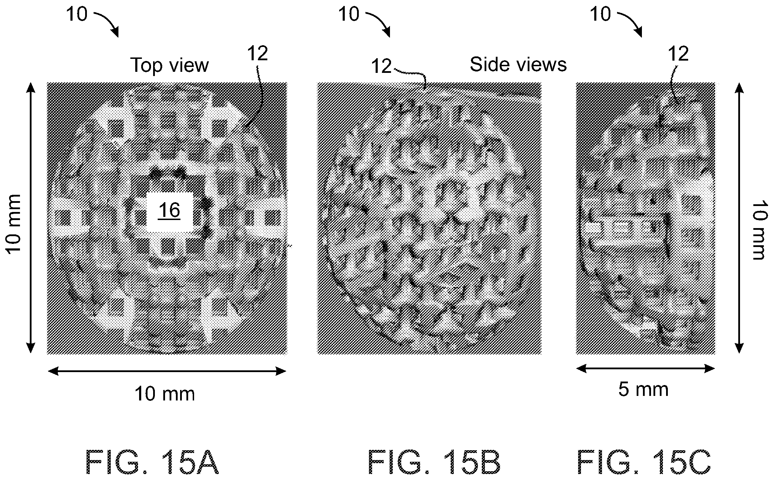

FIGs. 15A-C present images of a top view (FIG. 15A), and side views (FIGs. 15B-C) of an exemplary small size 3D scaffold with central compartment and folds to increase surface area for enhancement of tissue ingrowth.

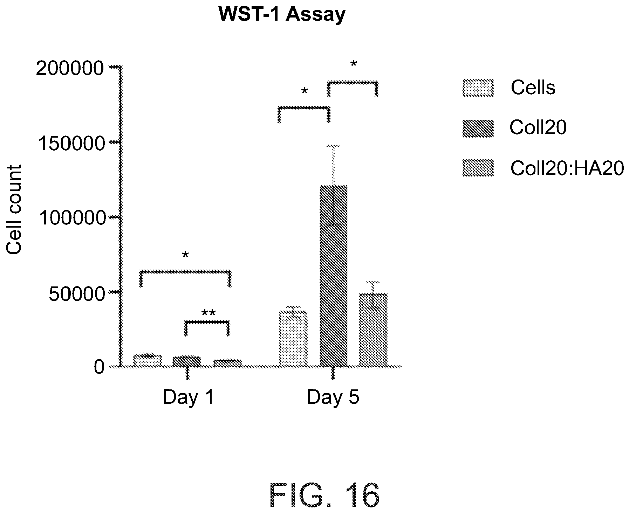

FIG. 16 is a bar graph showing the effect rhCollagen-based injectable matrix on the cell growth.

## DESCRIPTION OF SPECIFIC EMBODIMENTS OF THE INVENTION

The present invention relates to soft tissue repair and/or augmentation and, more particularly to collagen-based formulations usable for forming degradable scaffolds and soft tissue implants comprising same, and to collagen-based formulations which are usable as soft tissue fillers in combination with the degradable scaffolds.

Before explaining at least one embodiment of the invention in detail, it is to be understood that the invention is not necessarily limited in its application to the details of construction and the arrangement of the components and/or methods set forth in the following description and/or illustrated in the drawings and/or exemplified in the Examples.

The present inventors have designed and successfully practiced a methodology in which a degradable (e.g., biodegradable) composite scaffold is formed by an additive manufacturing process (e.g., 3D-bioprinting) which utilizes curable rhCollagen in combination with a curable synthetic material that hardens into a synthetic polymer, preferably a biocompatible synthetic polymer. The scaffold in designed to feature a porous wall, an inner cavity, and one or more injection ports and optionally a vascular network path that connects the outer most surface of the scaffold with the inner cavity of the scaffold, as described in further detail hereinbelow. The composite structure forms a part of a soft tissue implant and is designed so as to feature mechanical, physical and/or biological properties that allow and/or promote soft tissue growth at the implantation site, such that a newly formed soft tissue replaces the degradable implant.

The present inventors have further designed and successfully practiced an injectable matrix, which comprises rhCollagen, optionally in combination with one or more additional ECM components, and optionally a biological material such as cells and/or adipose tissue, which is injected (loaded) into the composite scaffold of the present embodiments as part of the degradable implant.

FIGs. 1A-6 present Background art methods and desirable parameters usable for controlling and determining mechanical and/or physical properties of an implant and/or matrix according to some embodiments of the present invention, and which can be used to devise implants and/or matrices that feature desirable properties (e.g., by controlling the chemical nature and relative amounts of the components composing the composite structure and/or matrix as described herein in any of the respective embodiments.

FIG. 7 presents a schematic illustration of producing and using an exemplary 3D bio-printed, breast implant comprising a degradable scaffold according to exemplary embodiments of the present invention. A degradable breast implant scaffold is prepared by bio-printing (upper left object), and a matrix comprising rhCollagen, optionally combined with stromal vascular fraction (SVF), homogenized fat extract and/or cellular components, is injected into the degradable scaffold (upper right object). The ECM components may, in some embodiments, comprise hyaluronic acid (HA), fibronectin, heparin, or laminin, or any combination thereof. The matrix-loaded scaffold in then implanted and vascularized in the body (lower left object). A gradual degradation of the implant scaffold together with the gradual replacement by a new tissue, comprising new tissue regeneration and vascularization, is thereafter effected (lower right object). At this final stage, the scaffold disappears, leaving a fully functional vascularized tissue.

FIGs. 8A-D, 10, 14A-B and 15 present images of exemplary designs of a 3D bio-printed degradable scaffold according to exemplary embodiments of the present invention.

FIG. 9 presents an exemplary production of a biological material - a fat extract - which can be included in an injectable matrix according to some embodiments of the present invention.

As shown in FIGs. 11-13E, the present inventors have demonstrated in *in vivo* studies that upon implantation of a degradable implant as described herein, neovascularization and tissue ingrowth occur at the site of implantation.

As shown in FIG. 16, the present inventors have further demonstrated that an injectable matrix as described herein promotes tissue growth.

Embodiments of the present invention therefore relate to a 3D bio-printed degradable composite scaffold, to curable formulations and an additive manufacturing process for preparing same, to a soft tissue implant comprising same, and to an injectable matrix are used in combination with the scaffold for promoting tissue growth. Embodiments of the present invention further relate to a use of the composite scaffold and the injectable matrix for reconstructing, augmenting, replacing and/or regenerating a soft tissue in a subject in need thereof.

The implant, composite scaffold and injectable matrix make use of a recombinant human collagen (rhCollagen).

Biocompatible, degradable soft tissue (e.g., breast) implants according to some of the present embodiments comprise composite scaffolds, which may be bio-printed to incorporate components including recombinant human collagen (rhCollagen) and biocompatible synthetic polymers. Composite scaffold components could further include at least one ECM component, for example, but not limited hyaluronic acid (HA), fibronectin, heparin, elastin, or laminin, or any combination thereof. Implant scaffolds comprise a porous lattice, an inner cavity, and at least one injection port that is sized to permit insertion of a cannula for injection of cells or tissue. Implant scaffolds may further comprise at least one printed vascular network path that permits entry of vascular cells and tissues into the implant.

The soft tissue (e.g., breast) implants disclosed herein may be used in methods of implanting in a subject in need, for example, but not limited to, to reconstruct or augment soft tissue (e.g., breast tissue) in a subject, where the implant degrades over time and is replaced by a new tissue. Further disclosed are methods of preparing the degradable soft tissue (e.g., breast) implants.

Disclosed herein are matrices comprising rhCollagen which does not include curable groups and cells or adipose tissue or both. In some embodiments, a matrix is injected into a soft tissue (e.g., breast) implant prior to implanting and in other embodiments is injected into the implant post implanting in a subject.

*Recombinant Human Collagen (rhCollagen):*

Collagen is a key structural protein of the extracellular matrix (ECM) of various connective tissues in the body.

Collagen is the most abundant protein in mammals and consists of poly-amino acid strands bound together to form triple-helices of elongated fibrils. Bone, tendons, and cartilage comprise collagen, as well as such disparate structures as corneas, blood vessels, intestines, and teeth. Type I collagen is the most abundant collagen of the human body.

A skilled artisan would appreciate that the term "collagen chain" may encompass a collagen subunit such as the alpha 1 or 2 chains of collagen fibers, which may be type I fibers. A skilled artisan would appreciate that the term "collagen" may encompass an assembled collagen trimer, which in the case of type I collagen, includes alpha 1 chains or two alpha 1 chains and one alpha 2 chain. A collagen fiber is collagen which is devoid of terminal propeptides C and N.

One distinguishing feature of collagen is the regular arrangement of amino acids in each of the three chains of the triple helix. The amino acid sequence often includes Gly-Pro-X or Gly-X-hydroxyproline repeats, where X may be any of various other amino acid residues. Typically, proline or hydroxyproline residues account for approximately one-sixth of the total sequence and glycine residues for one-third of the total sequence. Hydroxylation of proline and lysine residues by prolyl-4-hydroxylase (P4H) occurs within the Gly-X-Y repeat region as the polypeptide chain is co-translationally translocated across the endoplasmic reticulum (ER) membrane. P4H is an enzyme composed of two subunits, alpha and beta. Both are needed to form an active enzyme. Stability of the final triple-helical structure of collagen is highly dependent on the P4H-mediated hydroxylation of collagen chains. Lysyl hydroxylase (LH, EC 1.14.11.4), galactosyltransferase (EC 2.4.1.50) and glucosyltransferase (EC 2.4.1.66) are enzymes involved in posttranslational modifications of collagens. They sequentially modify lysyl residues in specific positions to hydroxylysyl, galactosylhydroxylysyl and glucosylgalactosyl hydroxylysyl residues. A single human enzyme, lysyl hydroxylase 3 (LH3) can catalyze all three consecutive steps in hydroxylysine linked carbohydrate formation.

Collagen used herein comprises collagen selected from the group consisting of recombinant human collagen (rhCollagen), or a plant-derived recombinant human collagen.

In some of any of the embodiments described herein, rhCollagen comprises plant-derived recombinant human collagen. In some of any of the embodiments described herein, a plant-derived human collagen is obtained from a genetically modified plant. A skilled artisan would appreciate that the term "genetically modified plant" may encompass any lower (e.g. moss) or higher (vascular) plant or a tissue or an isolated cell thereof (e.g., of a cell suspension) which is stably or transiently transformed with at least one exogenous polynucleotide sequence. In some embodiments, a genetically modified plant is selected from the group consisting of tobacco, maize, alfalfa, rice, potato, soybean, tomato, wheat, barley, canola, carrot, and cotton. In some of any of the embodiments described herein, a genetically modified plant comprises a tobacco plant such as described in WO 2006/035442, WO 2009/053985, WO 2011/064773, WO 2013/093921, WO 2014/147622 and WO 2018/225076.

In some embodiments, any type of collagen chain can be expressed by the genetically modified plant. In some embodiments, collagen chains comprise fibril-forming collagens (types I, II, III, V, and XI), networks forming collagens (types IV, VIII, and X), collagens associated with fibril surfaces (types IX, XII, and XIV), collagens which occur as transmembrane proteins (types XIII and XVII) or form 11-nm periodic beaded filaments (type VI). In some embodiments, a collagen chain expressed is an alpha 1 and/or alpha 2 chain of type I collagen.

In some embodiments, the expressed collagen alpha 1 chain can be encoded by any polynucleotide sequences derived from any mammal. In some embodiments, nucleic acid sequences encoding collagen alpha chains 1 and 2 are human and are set forth in SEQ ID NOs: 1 and 2, respectively. In some embodiments, a genetically modified plant comprises a sequence encoding a human collagen alpha-1 chain as set forth in SEQ ID NO: 1. In some embodiments, a genetically modified plant comprises a sequence encoding a human collagen alpha-2 chain as set forth in SEQ ID NO: 2.

In some embodiments, a genetically modified plant produces an amino acid sequence comprising a modified human collagen alpha-1 chain as set forth in SEQ ID NO: 3. In some embodiments, a genetically modified plant produces an amino acid sequence encoding a modified human collagen alpha-2 chain as set forth in SEQ ID NO: 4.

In some embodiments, a genetically modified plant comprises one or more sequences encoding P4H-alpha and P4H-beta. In some embodiments, exogenous P4H expressed by the genetically modified plant comprises mammalian P4H. In some embodiments, exogenous P4H comprises human P4H. In some embodiments, a genetically modified plant comprises one or more sequences encoding human P4H as set forth in SEQ ID NOs: 5 and 6.

In some embodiments, a genetically modified plant comprises a sequence encoding mammalian LH3. In some embodiments, a genetically modified plant comprises a sequence encoding LH3 as set forth in SEQ ID NO: 7.

In some embodiments, alpha collagen chains expressed in plants may or may not include their terminal propeptides (i.e., propeptide C and propeptide N). In some embodiments, a genetically modified plant comprises a sequence encoding protease N, protease C, or both.

In some other embodiments, the plant-derived human collagen may be a procollagen molecule which comprises partially digested propeptides. In yet some further embodiments, the plant-derived human collagen comprises atelocollagen. A skilled artisan would appreciate that the term "atelocollagen" may encompass collagen molecules lacking both the N- and C-terminal propeptides. In some embodiments, a plant-derived human collagen is atelocollagen having an amino acid (AA) sequence derived from sequences encoding collagen alpha chains as set forth in SEQ ID NO: 1 and SEQ ID NO: 2. Atelocollagen is derived by enzymatic digestion (for example, with ficin) of procollagen, which is the product of SEQ ID NO: 1 and SEQ ID NO: 2.

In some embodiments, polynucleotide sequences encoding the alpha chains and/or modifying enzymes (e.g. P4H and LH3) can be modified in a way which alters their cellular localization when expressed in plants.

One of ordinary skill in the art would be familiar with various methods of introducing nucleic acid constructs into genetically modified plants, including, for example, agrobacterium-mediated gene transfer or direct DNA transfer, such as, electroporation. One of ordinary skill in the art would also be knowledgeable of various plant breeding techniques and therefore no further description of such techniques is provided herein. In some embodiments, plant tissues/cells are harvested at maturity, and the collagen fibers are isolated using well know extraction methods.

The recombinant human collagen is a recombinant human type I collagen.

In some embodiments, type I collagen is considered a major component of a building material in 3D-bioprinting. In some embodiments, a soft tissue (e.g., breast) implant composite scaffold comprises a recombinant type I collagen. In some embodiments, a soft tissue (e.g., breast) implant composite scaffold comprises a recombinant human type I collagen. In some embodiments, a soft tissue (e.g., breast) implant composite scaffold comprises a recombinant human type I collagen produced in plants.

In some embodiments, a matrix as described herein comprises a recombinant human type I collagen. In some embodiments, a matrix as described herein comprises a recombinant human type I collagen produced in plants.

In some of any of the embodiments described herein, the recombinant human collagen is a plant-derived recombinant human collagen and in some embodiments the plant is tobacco.

In some of any of the embodiments described herein, a plant-derived human collagen is prepared using a system for the purification of type I recombinant human collagen (rhCollagen), which includes introducing five human genes encoding heterotrimeric type I collagen into tobacco plants (COLLPLANT™, Israel). The protein is purified to homogeneity through a cost-effective industrial process taking advantage of collagen's unique properties. See also WO 2006/035442, WO 2009/053985.

In some of any of the embodiments described herein, the human recombinant collagen (rhCollagen) as described herein in any of the respective embodiments is a monomeric rhCollagen.

By "monomeric" it is meant a rhCollagen as described herein which is soluble in an aqueous solution and does not form fibrillar aggregates.

In some of any of the embodiments described herein, the human recombinant collagen (rhCollagen) as described herein in any of the respective embodiments is a fibrillar rhCollagen, which is also referred to herein as "cross-linked thCollagen" or as "cross-linked fibrillar rhCollagen".

By "fibrillar" it is meant rhCollagen as described herein which is in a form of fibrillar aggregates in an aqueous solution containing same. Typically, but not obligatory, fibrillar rhCollagen is formed by subjecting monomeric rhCollagen to a fibrillogenesis buffer, typically featuring a basic pH. An exemplary procedure for forming fibrillar rhCollagen, is described in the Examples section that follows.

In some of any of the embodiments described herein, the rhCollagen is a particulate rhCollagen, for example, in a form of rhCollagen-derived nanoparticles, which can comprise, for example, rhCollagen nanoparticles and/or rhGellatin nanoparticles which are formed as a degradation product during the preparation of the nanoparticles, as described in the Examples section that follows.

In some of any of the embodiments described herein, the rhCollagen is a curable rhCollagen, as described herein. In some of any of the embodiments described herein, the rhCollagen is formed of a curable rhCollagen upon subjecting it to a curing condition alone or in combination with other curable materials, as described herein in any of the respective embodiments.

Generally, but obligatory, a curable rhCollagen as described herein is used for forming a composite scaffold according to the present embodiments, and fibrillar and/or particulate rhCollagen is utilized for forming a soft tissue filler according to the present embodiments.

In some embodiments, a 3D degradable soft tissue (e.g., breast) implant comprising a composite scaffold is prepared while using a type I collagen. In some embodiments, a 3D degradable soft tissue (e.g., breast) implant comprising a scaffold is prepared while using a recombinant type I collagen. In some embodiments, a 3D degradable soft tissue (e.g., breast) implant comprising a scaffold is prepared while using a recombinant human type I collagen. In some embodiments, a 3D degradable soft tissue (e.g., breast) implant which comprises a scaffold is prepared while using a recombinant human type I collagen produced in plants. In some embodiments, a 3D degradable soft tissue (e.g., breast) implant which comprises a scaffold is prepared while using of a recombinant human cross-linked type I collagen produced in plants. In some embodiments, a 3D degradable soft tissue (e.g., breast) implant which comprises a scaffold comprises is prepared while using a recombinant human modified type I collagen produced in plants such as a curable recombinant human type I collagen produced in plants, as described herein.

### Bio-printed composite scaffold:

According to an aspect of some embodiments of the present invention there is provided a composite scaffold which comprises a recombinant human collagen (rhCollagen) and a biocompatible synthetic polymer, as described herein in any of the respective embodiments.

According to some of any of the embodiments described herein, the composite scaffold is a bio-printed composite scaffold that is formed by additive manufacturing, for example, 3D-bioprinting, in configured pattern that essentially corresponds to the shape of the implant comprising same, as described herein.

According to some of any of the embodiments described herein, the composite scaffold is a 3D bio-printed composite scaffold.

A skilled artisan would appreciate that the term "scaffold" as used herein may encompass a three-dimensional (3D) structure used to enhance or promote the growth of cells and the formation of tissue.

According to some of any of the embodiments described herein, the composite scaffold (e.g., a 3D bio-printed composite scaffold) features: a porous lattice, an inner cavity within the scaffold and at least one injection port that connects the inner cavity with an outermost surface of the scaffold. The scaffold comprises a porous lattice that forms a porous wall; an inner cavity at least partially enclosed within the porous wall; and at least one injection port that connects the inner cavity with an outermost surface of the scaffold.

At least one, or each of, the injection port has an opening sized to permit insertion of an injection device through the port.

According to some of any of the embodiments described herein, a scaffold as disclosed herein is composed of a highly porous, artificial, three-dimensional network of interconnected pores that is used *in vivo* as a framework to which cells may attach. The scaffold comprises a matrix (as described in further detail hereinafter), both cells included in the matrix and additional cells may grow on and within the scaffold to regenerate tissues as desired. In some embodiments, a scaffold as described herein may be considered a living scaffold that may be formed to include living cells. One or more species of living cells may be attached to the scaffold. The living cells may be allowed to proliferate for a time period, in which the cells may grow to form colonies, after which the colonies may fuse to form a network of cells, and subsequently form a living tissue within the scaffold. In some embodiments, a scaffold promotes cell growth in three-dimensions.

In some embodiments, a composite scaffold provides a surface suitable for adherence and proliferation of cells. A composite scaffold may further provide mechanical stability and support. A composite scaffold may be in a particular shape or form so as to influence or delimit a three-dimensional shape or form assumed by a population of proliferating cells. Such shapes or forms include, but are not limited to, domes, cubes, cones, spheres, rolls, rectangle, three-dimensional amorphous shapes, etc.

A skilled artisan would appreciate that the term "pores" may encompass the voids or spaces of a composite scaffold. In some embodiments, the pores comprise a homogenous and interconnected porous network. In some embodiments, the pores comprise a heterogenous and interconnected porous network. In some embodiments, the interconnected porous network provides a space for cells to grow and proliferate, wherein nutrients may reach them, and waste may be eliminated by diffusion. In some embodiments, the at least one printed vascular network path provides a space for vascular tissues and cells, wherein vascular tissues and cells may provide nutrients to cells within the porous network. In some embodiments, pore openings are sized to allow cell penetration into the porous network of a scaffold.

According to some of any of the embodiments described herein, the composite scaffold comprises a porous lattice, in which solid spaces define and surround a plurality of pores. In some embodiments, the solid spaces are designed to provide support, shape, and structure to the scaffold or implant comprising same.

In some embodiments, solid spaces may comprise a porous network, such that scaffold comprises a porous network. In some embodiments, solid spaces may comprise a lattice, such that scaffold comprises a porous lattice. In some embodiments, solid spaces may comprise a braided lattice, such that scaffold comprises a braided lattice. In some embodiments, solid spaces may comprise a woven lattice, such that the scaffold comprises a woven lattice. In some embodiments, solid spaces may comprise a series of struts. In some embodiments, solid spaces may comprise a series of filaments. In some embodiments, solid spaces may comprise a series of walls. In some embodiments, solid spaces may comprise a combination of filaments, struts, walls, woven or braided lattice. In some embodiments, the filaments, struts, walls, woven or braided lattice may comprise tubular, square, rectangular, triangular, or free-form shapes. In some embodiments, that filaments, struts, walls, woven or braided lattice may be curved. In some embodiments, that filaments, struts, walls, woven or braided lattice may be straight. In some embodiments, that filaments, struts, walls, woven or braided lattice may be regularly distributed within the scaffold. In some embodiments, that filaments, struts, walls, woven or braided lattice may be irregularly distributed within the scaffold. In some embodiments, that filaments, struts, walls, woven or braided lattice may in some regions be regularly distributed and in other regions irregularly distributed within the scaffold. In some embodiments, that filaments, struts, walls, woven or braided lattice of the composite scaffold define a porous wall or a plurality of porous walls and are not present in the inner cavity.

In some embodiments, the thickness of the filaments, struts, lattice, or walls of a composite scaffold is between

about 1 μm to about 5 μm, between about 1 μm to about 10 μm, between about 5 μm to about 10 μm, between about 5 μm to about 25 μm, about 5 μm to about 50 μm, about 10 μm to about 50 μm, about 10 μm to about 75 μm, about 10 μm to about 100 μm, about 25 μm to about 100 μm, or about 50 μm to about 100 μm. In some embodiments, the thickness of the filaments, struts, lattice, or walls of a composite scaffold is between about 1 μm to about 1000 μm, between about 1 μm to about 500 μm, between about 100 μm to about 1000 μm, between about 50 μm to about 500 μm, between about 500 μm to about 1000 μm, about 50 μm to about 250 μm, about 100 μm to about 500 μm, about 100 μm to about 750 μm, about 250 μm to about 750 μm, about 250 μm to about 1000 μm, or about 50 μm to about 750 μm, including any intermediate values and subranges therebetween.

In some embodiments, the thickness of the filaments, struts, lattice, or walls of a composite scaffold is at least 1 μm, at least 2 μm, at least 3 μm, at least 4 μm, at least 5 μm, at least 6 μm, at least 7 μm, at least 8 μm, at least 9 μm, at least 10 μm, at least 20 μm, at least 30 μm, at least 40 μm, at least 50 μm, at least 60 μm, at least 70 μm, at least 80 μm, at least 90 μm, or at least 100 μm. In some embodiments, the thickness of the filaments, struts, lattice, or walls of a composite scaffold is at least 100 μm, at least 200 μm, at least 300 μm, at least 400 μm, at least 500 μm, at least 600 μm, at least 700 μm, at least 800 μm, at least 900 μm, at least 1000 μm, at least 250 μm, at least 350 μm, at least 450 μm, at least 550 μm, at least 650 μm, at least 750 μm, at least 850 μm, at least 950 μm, or at least 150 μm.

In some embodiments, the pores of the porous network, lattice or wall of a scaffold are uniformly spaced. In some embodiments, the pores of the porous network, lattice or wall of a scaffold have a repeating pattern. In some embodiments, the pores of the porous network, lattice or wall of the scaffold vary in shape and size. In some embodiments, the pores of the porous network, lattice or wall are constant in size.

In some embodiments, the pores of the porous network, lattice or wall of a scaffold have the same shape. In some embodiments, the shape of the pores of the porous network, lattice or wall of a scaffold is not the same. In some embodiments, the shape of the pores is not limited and may be any number of 3D shapes. In some embodiments, pore shapes comprise polyhedrons. In some embodiments, pore shapes comprise cubes, prisms, or pyramids. In some embodiments, pore shapes include, without limitation, circular, square, rectangular, oval, parallelogram, triangle, dodecahedrons (such as pentagonal dodecahedrons), 3D-kagome, diamond, octahedral. In some embodiments, the shape of the pores promotes cell migration, cell proliferation, cell differentiation and tissue growth.

In some embodiments, where the pore shape is square or rectangular, the dimensions of the pores are about between 50 μm to 200 μm by about 50 μm to 1,000 μm. In some embodiments, pore dimensions are about 50 μm by 50 μm, or 50 μm by 100 μm, or 50 μm by 150 μm, or 50 μm by 200 μm, or 50 μm by 250 μm, or 50 μm by 300 μm, or 50 μm by 350 μm, or 50 μm by 400 μm, or 50 μm by 450 μm, or 50 μm by 500 μm, or 50 μm by 550 μm, or 50 μm by 600 μm, or 50 μm by 650 μm, or 50 μm by 700 μm, or 50 μm by 750 μm, or 50 μm by 800 μm, or 50 μm by 850 μm, or 50 μm by 900 μm, or 50 μm by 950 μm, or 50 μm by 1,000 μm. In some embodiments, pore dimensions are about 100 μm by 100 μm, or 100 μm by 150 μm, or 100 μm by 200 μm, or 100 μm by 250 μm, or 100 μm by 300 μm, or 100 μm by 350 μm, or 100 μm by 400 μm, or 100 μm by 450 μm, or 100 μm by 1000 μm, or 100 μm by 550 μm, or 100 μm by 600 μm, or 100 μm by 650 μm, or 100 μm by 700 μm, or 100 μm by 750 μm, or 100 μm by 800 μm, or 100 μm by 850 μm, or 100 μm by 900 μm, or 100 μm by 950 μm, or 100 μm by 1,000 μm. In some embodiments, pore dimensions are about 150 μm by 150 μm, or 150 μm by 200 μm, or 150 μm by 250 μm, or 150 μm by 300 μm, or 150 μm by 350 μm, or 150 μm by 400 μm, or 150 μm by 450 μm, or 150 μm by 500 μm, or 150 μm by 550 μm, or 150 μm by 600 μm, or 150 μm by 650 μm, or 150 μm by 700 μm, or 150 μm by 750 μm, or 150 μm by 800 μm, or 150 μm by 850 μm, or 150 μm by 900 μm, or 150 μm by 950 μm, or 150 μm by 1,000 μm. In some embodiments, pore dimensions are about 200 μm by 200 μm, or 200 μm by 250 μm, or 200 μm by 300 μm, or 200 μm by 350 μm, or 200 μm by 400 μm, or 200 μm by 450 μm, or 200 μm by 1000 μm.

In some embodiments, pore openings are sized to allow cell penetration into the porous network of a scaffold. In some embodiments, pore opening or pore diameter is from about 50 μm to 800 μm. In some embodiments, the diameter of pores is from about 10 μm to 100 μm. In some embodiments, the diameter of pores is from about 100 μm to 700 μm. In some embodiments, the diameter of pores is from about 150 μm to 600 μm. In some embodiments, the diameter of pores is from about 200 μm to 550 μm. In some embodiments, the diameter of pores is from about 250 μm to 500 μm. In some embodiments, the diameter of pores is from about 300 μm to 450 μm. In some embodiments, the diameter of pores is from about 350 μm to 400 μm.

In some embodiments, an average diameter of pores is about 10 μm, or 20 μm, or 30 μm, or 40 μm, or 50 μm, or 60 μm, or 70 μm, or 80 μm, or 90 μm, or 100 μm, or 120 μm, or 140 μm, or 160 μm, or 180 μm, or 200 μm, or 220 μm, or 240 μm, or 260 μm, or 280 μm, or 300 μm, or 320 μm, or 340 μm, or 360 μm, or 380 μm, or 400 μm, or 420 μm, or 440 μm, or 460 μm, or 480 μm, or 500 μm, or 520 μm, or 540 μm, or 560 μm, or 580 μm, or 600 μm, or 620 μm, or 660 μm, or 680 μm, or 700 μm, or 720 μm, or 740 μm, or 760 μm, or 780 μm, or 800 μm.

Herein throughout, "μm" means micrometer, and is also referred to herein interchangeably as "micron(s)".

According to some of any of the embodiments described herein, the composite scaffold comprises a porous lattice that forms a porous wall, or a plurality of walls, e.g., interconnected walls that define a porous network, each composed of a plurality of pores, as described herein in any of the respective embodiments and any combination thereof.

Herein, the terms "porous lattice", "porous wall" and "porous network" are used interchangeably to describe the portion of the scaffold that at least partially enclose the inner cavity as described herein.

According to some of any of the embodiments described herein, a scaffold has a rough surface. In some embodiments, the average surface roughness (Ra) surface is greater than or equal to about 0.025 micrometers, e.g., from about 0.025 to about 50, or to about 20, micrometers. The surface's roughness can be manipulated, for example, by means of a folded porous wall, as described in further detail hereinafter (see, FIGs. 15A-C).

In some other embodiments, a scaffold has a smooth surface.

In some embodiments, a scaffold has a dome shape. In some embodiments, a scaffold has a free form shape. In some embodiments, the scaffold's size and shape corresponds to its intended use, for example, the size and shape of the scaffold is determined by a desired size and shape of the implant that comprises the scaffold, which is determined, amongst others, by the anatomy of the implanted subject.

In some embodiments, a scaffold has a volume of from about 1 to about 5 ml, or from about 5 ml to about 20 ml, or from about 20 to about 100 ml, or from about 100 to about 500 ml, or from about 50 to about 300 ml, including any intermediate values and subranges therebetween.

In some embodiments, the open space within a scaffold comprises the at least one cavity, e.g., an inner cavity as described herein, and the pores. In some embodiments, the total open space within a scaffold has a volume of from about 1 to about 5 ml, or from about 5 ml to about 20 ml, or from about 20 to about 100 ml, or from about 100 to about 500 ml, including any intermediate values and subranges therebetween. In some embodiments, the total volume of the open space within a scaffold is greater than 500 ml.

In some embodiments, the total open space within a scaffold network (e.g., a total volume of the pores and the inner cavity) comprises a volume about 1 ml, 2 ml, 3 ml, 4 ml, 5 ml, 6 ml, 7 ml, 8 ml, 9 ml, 10 ml, 11 ml, 12 ml, 13 ml, 14 ml, 15 ml, 16 ml, 17 ml, 18 ml, 19 ml, 20 ml, 25 ml, 30 ml, 35 ml, 40 ml, 50 ml, 60 ml, 70 ml, 80 ml, 90 ml, 100 ml, 110 ml, 120 ml, 130 ml, 140 ml, 150 ml, 160 ml, 170 ml, 180 ml, 190 ml, 200 ml, 210 ml, 220 ml, 230 ml, 240 ml, 250 ml, 260 ml, 270 ml, 280 ml, 290 ml, 300 ml, 350 ml, 400 ml, 450 ml, or 500 ml.

In some embodiments, the volume of the scaffold is at least 1 ml, at least 2 ml, at least 3 ml, at least 4 ml, at least 5 ml, at least 6 ml, at least 7 ml, at least 8 ml, at least 9 ml, at least 10 ml, at least 11 ml, at least 12 ml, at least 13 ml, at least 14 ml, at least 15 ml, at least 16 ml, at least 17 ml, at least 18 ml, at least 19 ml, at least 20 ml, at least 25 ml, at least 30 ml, at least 35 ml, at least 40 ml, at least at least 50 ml, at least 60 ml, at least 70 ml, at least 80 ml, at least 90, at least 100 ml, at least 110 ml, at least 120 ml, at least 130 ml, at least 140 ml, at least 150 ml, at least 160 ml, at least 170 ml, at least 180 ml, at least 190 ml, at least 200 ml, at least 210 ml, at least 220 ml, at least 230 ml, at least 240 ml, at least 250 ml, at least 260 ml, at least 270 ml, at least 280 ml, at least 290 ml, at least 300 ml, at least 350 ml, at least 400 ml, at least 450 ml, or at least 500 ml.

The scaffold as described herein comprises an inner cavity therewithin, for example, an inner cavity which is at least partially enclosed by a porous wall, as described herein.

In some embodiments, the volume of the inner cavity is from about 5 ml to about 300 ml, or from about 50 to about 250 ml, or from about 100 to about 200 ml, including any intermediate values and subranges therebetween. In some embodiments, the volume of the inner cavity is from about 20 ml to about 50 ml. In some embodiments, the volume of the inner cavity is from about 50 ml to 150 ml. In some embodiments, the volume of the inner cavity is from about 150 ml to 300 ml.

In some embodiments, the volume of the inner cavity is about 50 ml, 60 ml, 70 ml, 80 ml, 90 ml, 100 ml, 110 ml, 120 ml, 130 ml, 140 ml, 150 ml, 160 ml, 170 ml, 180 ml, 190 ml, 200 ml, 210 ml, 220 ml, 230 ml, 240 ml, 250 ml, 260 ml, 270 ml, 280 ml, 290 ml, 300 ml, or 350 ml. In some embodiments, the volume of the inner cavity is at least 50 ml, at least 60 ml, at least 70 ml, at least 80 ml, at least 90 ml, at least 100 ml, at least 110 ml, at least 120 ml, at least 130 ml, at least 140 ml, at least 150 ml, at least 160 ml, at least 170 ml, at least 180 ml, at least 190 ml, at least 200 ml, at least 210 ml, at least 220 ml, at least 230 ml, at least 240 ml, at least 250 ml, at least 260 ml, at least 270 ml, at least 280 ml, at least 290 ml, at least 300 ml, or at least 350 ml.

According to some of any of the embodiments described herein, an inner cavity comprises at least one chamber. Herein, the term "chamber" encompasses a fully or partially enclosed space or compartment within the scaffold (e.g., within a porous lattice, network or wall(s)), which, for example, is capable of containing a matrix described herein. In some embodiments, an inner cavity comprises up to 20 chambers. In some embodiments, an inner cavity comprises up to 30 chambers. In some embodiments, an inner cavity comprises from 1 to 30 cambers, or from 2 to 30 chambers. In some embodiments, an inner cavity comprises from 2 to 25 chambers. In some embodiments, an inner cavity comprises from 2 to 15 chambers. In some embodiments, an inner cavity comprises from 10 to 30 chambers. In some embodiments, an inner cavity comprises from 10 to 20 chambers. In some embodiments, an inner cavity comprises from 15 to 30 chambers.

In some embodiments, an inner cavity comprises one chamber, or two chambers, or three chambers, or four chambers, or five chambers, or six chambers, or seven chambers, or eight chambers, or nine chambers, or ten chambers, or 11 chambers, or 12 chambers, or 13 chambers, or 14 chambers, or 15 chambers, or 16 chambers, or 17 chambers, or 18 chambers, or 19 chambers, or 20 chambers, or 21 chambers, or 22 chambers, or 23 chambers, or 24 chambers, or 25 chambers, or 26 chambers, or 27 chambers, or 28 chambers, or 29 chambers, or 30 chambers. In some embodiments, an inner cavity comprises at least one chamber, at least two chambers, at least three chambers, at least four chambers, at least five chambers, at least six chambers, at least seven chambers, at least eight chambers, at least nine chambers, at least ten chambers, at least 11 chambers, at least 12 chambers, at least 13 chambers, at least 14 chambers, at least 15 chambers, at least 16 chambers, at least 17 chambers, at least 18 chambers, at least 19 chambers, at least 20 chambers, at least 21 chambers, at least 22 chambers, at least 23 chambers, at least 24 chambers, at least 25 chambers, at least 26 chambers, at least 27 chambers, at least 28 chambers, at least 29 chambers, at least 30 chambers.

In some embodiments, whenever there are two or more chambers in the inner cavity, the chambers may vary in their size and/or shape from one another. Alternatively, all chambers have the same size and/or the same shape.

In some embodiments, whenever there are two or more chambers in the inner cavity, at least two and optionally and preferably all of the chambers are interconnected, for example, by means of one or more channels or tunnels or any other hollow structures that interconnect two (e.g., adjacent) chambers. In some other embodiments, chambers are not directly connected, but would be connected (e.g., to allow fluid communication therebetween) by the porous network around them. In some embodiments, some chambers of the scaffold's inner cavity are interconnected. In some of any of these embodiments, at least two, at least some, or all of the chambers are at least in fluid communication with one another, for example, by means of one or more channels or tunnels or any other hollow structures that interconnect two adjacent chambers and allow fluid flow therebetween (e.g., of blood and/or components thereof). In some embodiments, the two or more chambers are interconnected to allow therebetween flow of a fluid (e.g., liquid) that comprises cells and/or other biological components.

In some of any of the embodiments described herein, a total volume of the inner cavity is from about 10 to about 90 % of the total volume of the scaffold, including any intermediate values and subranges therebetween. In some embodiments, a total volume of the inner cavity is from about 10 to about 80, or from about 10 to about 70, or from about 10 to about 60, or from about 10 to about 50, or from about 10 to about 40, or from about 10 to about 30, or from about 10 to about 20, or from about 20 to about 90, or from about 20 to about 80, or from about 20 to about 70, or from about 20 to about 60, or from about 20 to about 50, or from about 20 to about 40, or from about 20 to about 30, or from about 30 to about 90, or from about 30 to about 90, or from about 30 to about 80, or from about 30 to about 70, or from about 30 to about 60, or from about 30 to about 50, or from about 30 to about 40, or from about 20 to about 90, or from about 20 to about 80, our from about 30 to about 70, or from about 30 to about 60, or from about 30 to about 50, or from about 30 to about 40, or from about 40 to about 90, or from about 40 to about 80, or from about 40 to about 70, or from about 40 to about 60, or from about 40 to about 50, or from about 50 to about 90, or from about 50 to about 80, or from about 50 to about 70, or from about 50 to about 60, or from about 60 to about 90, or from about 60 to about 80, or from about 60 to about 70, or from about 70 to about 90, or from about 70 to about 80, or from about 80 to about 90, % of the total volume of the scaffold.

The scaffold comprises at least one injection port that connects the inner cavity with the outer most surface of the scaffold (e.g., an outermost surface of a porous lattice, network or wall). A skilled artisan would appreciate that the term "injection port" means a hole, opening or orifice which allows injecting of material, for example, cells, tissue or a matrix, as described in detail herein.

The injection port openings are sized for injection of a matrix as described herein in any of the respective embodiments.

An injection port has a diameter which allows injection using a suitable injecting device. In some embodiments, such devices, include but are not limited to, needles, cannulas, pointed plastic tip applicators, reservoirs, stents, plungers, release systems and syringes. In an exemplary embodiments, the device has a needle of up to 20 or up to 18 Gauge and the diameter of the injection port is designed accordingly.

An injection port has a diameter which allows injection of a matrix as described in detail herein. In some embodiments, an injection port has a diameter which allows injection of ECM components, cells and/or tissue, as described herein.

In some embodiments, an injection port has a diameter of from about 0.3 mm to about 3 mm, including any intermediate values and subranges therebetween. In some embodiments, an injection port has a diameter of from about 0.2 mm to about 2.5 mm. In some embodiments, an injection port has a diameter of fro about 0.5 mm to about 1 mm. In some embodiments, an injection port has a diameter of from about 0.5 mm to about 2 mm.

In some embodiments, an injection port has a diameter of 0.2 mm, 0.3 mm, 0.4 mm, 0.5 mm, 0.6 mm, 0.7 mm, 0.8 mm, 0.9 mm, 1.0 mm, 1.2 mm, 1.3 mm, 1.4 mm, 1.5 mm, 1.6 mm, 1.7 mm, 1.8 mm, 1.9 mm, 2.0 mm, 2.1 mm, 2.2 mm, 2.3 mm, 2.4 mm, 2.5 mm, 2.6 mm, 2.7 mm, 2.8 mm, 2.9 mm, or 3 mm. In some embodiments, an injection port has a diameter of at least 0.2 mm, at least 0.3 mm, at least 0.4 mm, at least 0.5 mm, at least 0.6 mm, at least 0.7 mm, at least 0.8 mm, at least 0.9 mm, at least 1.0 mm, at least 1.2 mm, at least 1.3 mm, at least 1.4 mm, at least 1.5 mm, at least 1.6 mm, at least 1.7 mm, at least 1.8 mm, at least 1.9 mm, at least 2.0 mm, at least 2.1 mm, at least 2.2 mm, at least 2.3 mm, at least 2.4 mm, at least 2.5 mm, at least 2.6 mm, at least 2.7 mm, at least 2.8 mm, at least 2.9 mm, or at least 3 mm.

In some embodiments, a matrix is loaded into the scaffold prior to implanting in a subject, where injection is through the at least one injection port. In some embodiments, a matrix is injected into the scaffold, using at least one injection port of the scaffold, post implanting of the scaffold in a subject.

In some embodiments, a scaffold comprises up to 20 injection ports. In some embodiments, a scaffold comprises up to 30 injection ports. In some embodiments, a scaffold comprises from 1 to 20 injection ports, including any intermediate values and subranges therebetween. In some embodiments, a scaffold comprises from 10 to 30 injection ports. In some embodiments, a scaffold comprises between 1 to 30 injection ports, including any intermediate values and subranges therebetween. In some embodiments, a scaffold comprises from 5 to 25 injection ports. In some embodiments, a scaffold comprises from 1 to 15 injection ports. In some embodiments, a scaffold comprises from 20 to 30 injection ports. In some embodiments, a scaffold comprises from 10 to 20 injection ports. In some embodiments, a scaffold comprises from 15 to 30 injection ports.

In some embodiments, a scaffold comprises one injection port, two injection ports, three injection ports, four injection ports, five injection ports, six injection ports, seven injection ports, eight injection ports, nine injection ports, ten injection ports, eleven injection ports, twelve injection ports, thirteen injection ports, fourteen injection ports, fifteen injection ports, sixteen injection ports, seventeen injection ports, eighteen injection ports, nineteen injection ports, or twenty injection ports. In some embodiments, a scaffold comprises at least one injection port, at least two injection ports, at least three injection ports, at least four injection ports, at least five injection ports, at least six injection ports, at least seven injection ports, at least eight injection ports, at least nine injection ports, at least ten injection ports, at least eleven injection ports, at least twelve injection ports, at least thirteen injection ports, at least fourteen injection ports, at least fifteen injection ports, at least sixteen injection ports, at least seventeen injection ports, at least eighteen injection ports, at least nineteen injection ports, or at least twenty injection ports.

In some of any of the embodiments described herein, a scaffold comprises at least one vascular network path that connects at least a portion of the outermost surface of the scaffold with the inner cavity of the scaffold. According to some of these embodiments, the vascular network path is a printed vascular network path, that is, it is bio-printed or other manufactured by additive manufacturing as described herein, along with the composite scaffold as described herein. According to some of these embodiments, the vascular network path is sized to permit entry of vascular cells and tissues. In some embodiments, vascular network paths are configured to promote vascularization of the scaffold, when an implant comprising same is implanted in a subject. Specifically, the vascular network paths are configured to promote the growth of the subject's vascular network into the scaffold. Vascularization of the scaffold encourages growth of cells and tissue in the implant. The subject's vascular network provides an efficient supply of oxygen and other nutrients, to the cells and tissue within the scaffold, such as those described in detail herein. Further, the subject's vascular network provides an efficient egress point for cellular waste and debris, wherein the vascular network may efficiently remove the cellular waste and or debris from the area of the implant. In some embodiments, vascular network paths are designed and configured to allow anastomosis to the subject's blood vessels through a surgical procedure.

In some embodiments, upon implanting a 3D bio-printed degradable implant as described herein in a subject, the printed vascular network path may be surgically joined with the subject's blood vessels by anastomosis. In some embodiments, the subject's blood vessels are joined through at least one of the printed vascular network paths such that cells and tissue within the inner cavity of the scaffold receive a constant supply of oxygen and nutrients.

In some embodiments, a scaffold comprises up to 1,000 vascular network paths. In some embodiments, a scaffold comprises from 1 to about 1,000 printed vascular network paths, including any intermediate values and subranges therebetween. In some embodiments, a scaffold comprises from about 10 to about 1,000 vascular network paths. In some embodiments, a scaffold comprises from 1 to about 200 vascular network paths. In some embodiments, a scaffold comprises from 200 to 500 vascular network paths. In some embodiments, a scaffold comprises from 800 to 1,000 vascular network paths. In some embodiments, a scaffold comprises from 500 to 800 vascular network paths. In some embodiments, the number of vascular network paths is from 1 to about 100. In some embodiments, the number of vascular network paths is from about 100 to about 200. In some embodiments the number of vascular network paths is from about 200 to about 300. In some embodiments, the number of vascular network paths is from about 300 to about 400. In some embodiments the number of vascular network paths is from about 400 to about 500. In some embodiments the number of vascular network paths is from about 500 to about 600. In some embodiments the number of vascular network paths is from about 600 to about 700. In some embodiments the number of vascular network paths is from about 700 to about 800. In some embodiments the number of vascular network paths is from about 800 to about 900. In some embodiments the number of vascular network paths is from about 900 to about 1,000.

Reference is now made to FIG. 8B, which shows an exemplary 3D scaffold 10 according to some embodiments of the present disclosure. In the illustrated embodiment, which is not to be considered as limiting, scaffold 10 is dome-shaped, but other shapes are also contemplated. The illustrated dome shaped 3D scaffold **10** has lateral dimensions of about 10 mm by 10 mm and a height of about 6.0 mm, but other dimensions are also contemplated. Scaffold **10** is preferably porous. In the illustrated embodiment, which is not to be considered as limiting, the pores **12** of scaffold **10** are square shaped pores, but other shapes are also contemplated. In this exemplary scaffold, dimensions for pores **12** are about 0.5 mm x 0.5 mm, but other dimensions are also contemplated.

In some embodiments, scaffold **10** comprises a porous network, for example, but not limited to, a lattice, an inner cavity at least partially enclosed by the lattice, and at least one injection port that connects the inner cavity with the outer most surface of the scaffold, where the injection port openings are sized to permit insertion of an injection device, such as, but not limited to, a cannula for cell or tissue injection. In some embodiments, the scaffold comprises an inner cavity within the scaffold, as described herein in any of the respective embodiments.

A representative example of scaffold **10** in embodiments in which an inner cavity is employed is schematically illustrated in FIG. 10. Shown is a wall **14** of scaffold **10,** which wall **14** is optionally and preferably porous, and an inner cavity **16** which is at least partially enclosed within wall **14.** The inner cavity **16** illustrated in FIG. 10 is in the form of a plurality of connected lobes, but other shapes for inner cavity **16** are also contemplated according to some embodiments of the present invention. Also shown are several injection ports **18.** Three injection ports are illustrated in FIG. 10, but scaffold **10** can include any number of injections ports as described herein, including a configuration devoid of any port. At least one of injection ports **18** is sized to receive an injection device **20** for injecting a substance into cavity **16.** Injection device **20** is illustrated in FIG. 10 as a syringe, but any device suitable for injecting a substance, such as, but not limited to, a cannula or a pipette, as described herein, can be used. Typically, injection device **20** injects into cavity **16** a matrix as described herein. As a non-limiting example shown in FIG. 10, device **20** injects SVF in rhCollagen-based matrix.

In some embodiments of the present invention wall **14** of scaffold **10** comprises a vascular network **22,** as described herein in any of the respective embodiments and any combination thereof. Vascular network **22** topically connects the outer most surface **24** of wall **14** with the inner cavity **16.** Preferably, vascular network **22** is sized to permit entry of vascular components, cells and tissues into cavity **16.**

Another configuration suitable for the present embodiments is illustrated in FIGs. 14A-D, showing an illustrated side view (FIG. 14A), a top illustrated view (FIG. 14B), a perspective illustrated view (FIG. 14C), and a representative image (FIG. 14D)_of scaffold **10.** In this configuration, scaffold **10** comprises a plurality of inner cavities **16a, 16b, 16c** which are preferably interconnected thereamongst. The overall volume of the scaffold shown in FIGs. 14A-D is typically from about 160 ml to about 180 ml, e.g., about 168 ml. The total volume of the internal cavities **16a, 16b, 16c,** is typically from about 30 % to about 50 %, e.g., about 40 % of the overall volume of the scaffold. For example, when the overall volume of the scaffold is about 168 ml, the total volume of the internal cavities can be about 49 ml.

An additional example embodiment is shown in FIGs. 15A-C, which are schematic illustrations of a top view (FIG. 15A), and side views (FIGs. 15B-C) of scaffold **10.** In this embodiment, scaffold **10** comprises folds to increase the surface area of the scaffold, for example, for enhancement of tissue ingrowth (marked by dashed lines). In FIG. 15A, cavity **16** is illustrated as central, but embodiments in which cavity **16** is not central are also contemplated. The lateral dimensions of the scaffold shown in FIGs. 15A-C are about 10 mm by 10 mm, and the height is about 5 mm.

### Preparation of a bio-printed composite scaffold:

According to some of any of the embodiments described herein, the 3D composite scaffold as described herein is formed by additive manufacturing, for example, 3D-bioprinting. The 3D composite scaffold, which is also referred to herein throughout simply as a "scaffold", or as "composite scaffold" or as a bio-printed scaffold, or as a bio-printed composite scaffold, or as a 3D bio-printed composite scaffold, or as a 3D bio-printed scaffold, encompasses a composite scaffold formed in an additive manufacturing process such as bioprinting, as described herein.

According to some of any of the embodiments described herein the bio-printed composite scaffold is formed by bio-printing a curable formulation in a configured pattern which generally corresponds to a desired shape and dimension of the soft tissue implant comprising the composite scaffold, as described herein, and which comprises a porous lattice or wall, an inner cavity, one or more injection ports and optionally a network of vascular paths, as described herein in any of the respective embodiments and any combination thereof.

In some embodiments, bioprinting a 3D scaffold entails a manufacturing process using bio-ink formulations that comprise biological components via a manufacturing system such as a Digital Light Processing (DLP), stereolithography (SLA), Inkjet, Laser or Extrusion printer a bioprinter or a bioprinting system. In some embodiments, a 3D scaffold design is converted into a printable digital format such as stereolithography (STL) which is used for printing. A skilled artisan would be familiar with the available technology and commercial bio-printers which support bioprinting of the 3D scaffolds and implants disclosed herein.

A skilled artisan would appreciate that the bio-printed 3D composite scaffold is in the form comprising a porous lattice, an inner cavity within the scaffold, and at least one injection port that connects the inner cavity with the outer most surface of the scaffold, where the injection port is sized to permit insertion of a cannula for cell or tissue injection. The characteristics of a porous lattice, an inner cavity, vascular network paths and an at least one injection port have been described in detail above, wherein that description should be fully incorporated herein as well, including but not limited to shapes, sizes, bio-properties, the number of inner cavity chambers, cavity volume, the number of ports, and pore size.

According to an aspect of some embodiments of the present invention, there is provided a process (a method) of additive manufacturing (AM) of a three-dimensional composite scaffold as described herein in any of the respective embodiments and any combination thereof. According to embodiments of this aspect, the method is effected by sequentially forming a plurality of layers in a configured pattern corresponding to the size, shape and all other features of the composite scaffold, as described herein in any of the respective embodiments and any combination thereof, thereby forming the composite scaffold. According to embodiments of this aspect, formation of each layer is effected by dispensing at least one uncured building material, and exposing the dispensed building material to a curing condition to thereby form a hardened (cured) material, which constitutes at least a part of the composite scaffold.

A method of preparing a 3D bio-printed composite scaffold further comprises a step of subjecting the dispensed layers to a curing condition, e.g., by illumination of the composite scaffold with a light source, as described hereinafter in further detail.

Herein throughout, the phrase "building material" encompasses the phrases "uncured building material" or "uncured building material formulation" and collectively describes the materials that are dispensed by sequentially forming the layers, as described herein. This phrase encompasses uncured materials which form the final object (the composite scaffold), namely, one or more uncured modeling material formulation(s), and optionally also uncured materials used to form a support, namely uncured support material formulations. The building material can also include non-curable materials that preferably do not undergo (or are not intended to undergo) any change during the process, for example, biological materials or components (other than a curable collagen as described herein) and/or other agents or additives as described herein.

The building material that is dispensed to sequentially form the layers as described herein is also referred to herein interchangeably as "printing medium" or "bioprinting medium" or "bio-ink".

An uncured building material can comprise one or more modeling material formulations, and can be dispensed such that different parts of the object are made upon hardening (e.g., curing) of different modeling formulations, and hence are made of different hardened (e.g., cured) modeling materials or different mixtures of hardened (e.g., cured) modeling materials.

The method of the present embodiments manufactures three-dimensional composite scaffold in a layerwise manner by forming a plurality of layers in a configured pattern corresponding to the desired shape, size, and all other features of the scaffold, as described herein.

Each layer is formed by an additive manufacturing apparatus which scans a two-dimensional surface and patterns it. While scanning, the apparatus visits a plurality of target locations on the two-dimensional layer or surface, and decides, according to a pre-set algorithm, for each target location or a group of target locations, whether or not the target location or group of target locations is to be occupied by a building material, and which type of a building material is to be delivered thereto. The decision is made according to a computer image of the surface.

When the AM is by three-dimensional inkjet printing, an uncured building material, as defined herein, is dispensed from a dispensing head having a set of nozzles to deposit building material in layers on a supporting structure. The AM apparatus thus dispenses building material in target locations which are to be occupied and leaves other target locations void. The apparatus typically includes a plurality of dispensing heads, each of which can be configured to dispense a different building material (for example, different modeling material formulations, each containing a different biological component; or each containing a different curable material; or each containing a different concentration of a curable material, and/or different support material formulations). Thus, different target locations can be occupied by different building materials (e.g., a modeling formulation and/or a support formulation, as defined herein).

The final three-dimensional object (composite scaffold) is made of the hardened modeling material or a combination of hardened modeling materials or a combination of hardened modeling material/s and support material/s or modification thereof (e.g., following curing). All these operations are well-known to those skilled in the art of additive manufacturing (also known as solid freeform fabrication).

In some exemplary embodiments of the invention a composite scaffold is manufactured by dispensing a building material that comprises two or more different modeling material formulations, each modeling material formulation from a different dispensing head of the AM apparatus. The modeling material formulations are optionally and preferably deposited in layers during the same pass of the dispensing heads. The modeling material formulations and/or combination of formulations within the layer are selected according to the desired properties of the object (the composite scaffold).

An exemplary process according to some embodiments of the present invention starts by receiving 3D printing data corresponding to the shape, size and all other features of the composite scaffold, as described herein. The data can be received, for example, from a host computer which transmits digital data pertaining to fabrication instructions based on computer object data, *e.g.,* in a form of a Standard Tessellation Language (STL) or a StereoLithography Contour (SLC) format, Virtual Reality Modeling Language (VRML), Additive Manufacturing File (AMF) format, Drawing Exchange Format (DXF), Polygon File Format (PLY), Digital Imaging and Communications in Medicine (DICOM) or any other format suitable for Computer-Aided Design (CAD).

The process continues by dispensing the building material as described herein in layers, on a receiving medium, using one or more dispensing (e.g., printing) heads, according to the printing data.

The dispensing can be in a form of droplets, or a continuous stream, depending on the additive manufacturing methodology employed and the configuration of choice.

The receiving medium can be a tray of a printing system, or a supporting article or medium made of, or coated by, a biocompatible material, such as support media or articles commonly used in bioprinting, or a previously deposited layer.

In some embodiments, the receiving medium comprises a sacrificial hydrogel or other biocompatible material as a mold to embed the printed object, and is thereafter removed by chemical, mechanical or physical (e.g., heating or cooling) means. Such sacrificial hydrogels can be made of, for example, a Pluronic material or of Gelatin.

Once the uncured building material is dispensed on the receiving medium according to the 3D data, the method optionally and preferably continues by hardening the dispensed formulation(s). In some embodiments, the process continues by exposing the deposited layers to a curing condition. Preferably, the curing condition is applied to each individual layer following the deposition of the layer and prior to the deposition of the previous layer.

As used herein, the term "curing" describes a process in which a formulation is hardened. The hardening of a formulation typically involves an increase in a viscosity of the formulation and/or an increase in a storage modulus of the formulation (G'). In some embodiments, a formulation which is dispensed as a liquid becomes solid or semi-solid (e.g., gel) when hardened. A formulation which is dispensed as a semi-solid (e.g., soft gel) becomes solid or a harder or stronger semi-solid (e.g., strong gel) when hardened.

The term "curing" as used herein encompasses, for example, polymerization of monomeric and/or oligomeric materials and/or cross-linking of polymeric chains (either of a polymer present before curing or of a polymeric material formed in a polymerization of the monomers or oligomers). The product of a curing reaction is therefore typically a polymeric material and/or a cross-linked material. This term, as used herein, encompasses also partial curing, for example, curing of at least 20 % or at least 30 % or at least 40 % or at least 50 % or at least 60 % or at least 70 % of the formulation, as well as 100 % of the formulation.

Herein, the phrase "a condition that affects curing" or "a condition for inducing curing", which is also referred to herein interchangeably as "curing condition" or "curing inducing condition" describes a condition which, when applied to a formulation that contains a curable material, induces a curing as defined herein. Such a condition can include, for example, application of a curing energy, as described hereinafter to the curable material(s), and/or contacting the curable material(s) with chemically reactive components such as catalysts, co-catalysts, and activators.

When a condition that induces curing comprises application of a curing energy, the phrase "exposing to a curing condition" and grammatical diversions thereof means that the dispensed layers are exposed to the curing energy and the exposure is typically performed by applying a curing energy to the dispensed layers.

A "curing energy" typically includes application of radiation and/or application of heat.

The radiation can be electromagnetic radiation (e.g., ultraviolet or visible light), or electron beam radiation, or ultrasound radiation or microwave radiation, depending on the materials to be cured. The application of radiation (or irradiation) is effected by a suitable radiation source. For example, an ultraviolet or visible or infrared or Xenon or mercury or lamp, or LED source, can be employed, as described herein.

A curable material or system that undergoes curing upon exposure to radiation is referred to herein interchangeably as "photopolymerizable" or "photoactivatable" or "photocurable".

When the curing energy comprises heat, the curing is also referred to herein and in the art as "thermal curing" and comprises application of thermal energy. Applying thermal energy can be effected, for example, by heating a receiving medium onto which the layers are dispensed or a chamber hosting the receiving medium, as described herein. In some embodiments, the heating is effected using a resistive heater.

In some embodiments, the heating is effected by irradiating the dispensed layers by heat-inducing radiation. Such irradiation can be effected, for example, by means of an IR lamp or Xenon lamp, operated to emit radiation onto the deposited layer.

In some embodiments, heating is effected by infrared radiation applied by a ceramic lamp, for example, a ceramic lamp that produces infrared radiation of from about 3 $\mu$m to about 4 $\mu$m, *e.g.,* about 3.5 $\mu$m.

A curable material or system that undergoes curing upon exposure to heat is referred to herein as "thermally-curable" or "thermally-activatable" or "thermally-polymerizable".

In some of any of the embodiments described herein, hardening the dispensed formulation(s) comprises exposing the dispensed formulation to a curing condition as described herein in any of the respective embodiments, for example, to irradiation (illumination).

In some embodiments, the exposure to a curing condition is for a short time period, for example, a time period of less than 3 minutes, less than 300 seconds, for example, of from 10 seconds to 240 seconds, or from 10 seconds to 120 seconds, to from 10 seconds to 60 seconds, including an intermediate values and subranges therebetween.

In some of any of the embodiments described herein, the method further comprises exposing the cured modeling material formulation(s) either before or after removal of a support material formulation, if such has been included in the building material, to a post-treatment condition. The post-treatment condition is typically aimed at further hardening the cured modeling material(s). In some embodiments, the post-treatment hardens a partially-cured formulation to thereby obtain a completely cured formulation.

In some embodiments, the post-treatment is effected by exposure to heat or radiation, as described in any of the respective embodiments herein.

Some embodiments contemplate the fabrication of a composite scaffold by dispensing different formulations from different dispensing heads. These embodiments provide, *inter alia,* the ability to select formulations from a given number of formulations and define desired combinations of the selected formulations and their properties.

According to the present embodiments, the spatial locations of the deposition of each formulation with the layer are defined, either to effect occupation of different three-dimensional spatial locations by different formulations, or to effect occupation of substantially the same three-dimensional location or adjacent three-dimensional locations by two or more different formulations so as to allow post deposition spatial combination of the formulations within the layer.

The present embodiments thus enable the deposition of a broad range of material combinations, and the fabrication of an object which may consist of multiple different combinations of modeling material formulations, in different parts of the object, according to the properties desired to characterize each part of the object.

A system utilized in additive manufacturing may include a receiving medium and one or more dispensing heads. The receiving medium can be, for example, a fabrication tray that may include a horizontal surface to carry the material dispensed from the printing head. In some embodiments, the receiving medium is made of, or coated by, a biocompatible material, as described herein.

The dispensing head may be, for example, a printing head having a plurality of dispensing nozzles arranged in an array of one or more rows along the longitudinal axis of the dispensing head. The dispensing head may be located such that its longitudinal axis is substantially parallel to the indexing direction.

The additive manufacturing system may further include a controller, such as a microprocessor to control the AM process, for example, the movement of the dispensing head according to a pre-defined scanning plan (e.g., a CAD configuration converted to a Standard Tessellation Language (STL) format and programmed into the controller). The dispensing head may include a plurality of jetting nozzles. The jetting nozzles dispense material onto the receiving medium to create the layers representing cross sections of a 3D object.

In addition to the dispensing head, there may be a source of curing energy, for curing the dispensed building material. The curing energy is typically radiation, for example, UV radiation or heat radiation. Alternatively, there may be means for providing a curing condition other than electromagnetic or heat radiation, for example, means for cooling the dispensed building material of for contacting it with a reagent that promotes curing.

Additionally, the AM system may include a leveling device for leveling and/or establishing the height of each layer after deposition and at least partial solidification, prior to the deposition of a subsequent layer.

According to the present embodiments, the additive manufacturing method described herein is for bioprinting a biological object.

As used herein, "bioprinting" means practicing an additive manufacturing process while utilizing one or more curable (e.g., modeling) formulation(s) (bio-ink formulations) that comprise(s) biological components, as described herein, via methodology that is compatible with an automated or semi-automated, computer-aided, additive manufacturing system as described herein (e.g., a bioprinter or a bioprinting system).

Herein throughout, the phrase "modeling material formulation", which is also referred to herein interchangeably as "modeling formulation" or "modeling material composition" or "modeling composition", or simply as a "formulation", or a "composition", describes a part or all of the uncured building material (printing medium) which is dispensed so as to form the final object (the composite scaffold), as described herein. The modeling formulation is an uncured, curable modeling formulation, which, upon exposure to a curing condition, forms the object (the composite scaffold) or a part thereof.

In the context of bioprinting, an uncured building material comprises at least one modeling formulation that comprises one or more biological components or materials (e.g., a curable rhCollagen as described herein), and is also referred to herein and in the art as "bio-ink" or "bio-ink formulation".

In some embodiments, the bioprinting comprises sequential formation of a plurality of layers of the uncured building material in a configured pattern, preferably according to a three-dimensional printing data, as described herein. At least one, and preferably most or all, of the formed layers (before hardening or curing) comprise(s) one or more biological component(s) as described herein (e.g., a curable rhCollagen as described herein). Optionally, at least one of the formed layers (before hardening or curing) comprises one or more non-biological curable materials, and/or non-curable biological or non-biological components, preferably biocompatible materials which do not interfere (e.g., adversely affect) with the biological and/or structural features of the biological components (e.g., collagen) in the printing medium and/or bio-ink.

In some embodiments, the components in the bio-ink or the printing medium, e.g., non-curable and curable materials, and/or the curing condition applied to effect curing, are selected such that they do not significantly affect structural and/or functional properties of the biological components in the bio-ink or printing medium.

In some of any of the embodiments described herein, the building material (e.g., the printing medium) comprises modeling material formulation(s) (bio-ink) and optionally support material formulation(s), and all are selected to include materials or combination of materials that do not interfere with the biological and/or structural features of the biological components.

In some of any of the embodiments described herein, the bioprinting method is configured to effect formation of the layers under conditions that do not significantly affect structural and/or functional properties of the biological components in the bio-ink.

In some embodiments, a bioprinting system for effecting a bioprinting process/method as described herein is configured so as to allow formation of the layers under conditions that do not significantly affect structural and/or functional properties of the biological components in the bio-ink.

In some of any of the embodiments described herein, the additive manufacturing (e.g., bioprinting) process and system are configured such that the process parameters (e.g., temperature, shear forces, shear strain rate) do not interfere with (do not substantially affect) the functional and/or structural features of the biological components.

In some of any of the embodiments described herein, the additive manufacturing process (the bio-printing) is performed at a temperature of at least 10 °C, or of at least 20 °C, for example, at a temperature that ranges from about 10 to about 40 °C, preferably from about 10 °C to 37 °C, or from about 20 °C to 37 °C, or from about 20 °C to about 30 °C, or from about 20 °C to about 28 °C, or from about 20 °C to about 25 °C, including any intermediate values and subranges therebetween, or at room temperature, or at 37 °C.

In some of any of the embodiments described herein, the above-indicated temperatures/temperature ranges are the temperatures at which the building material (e.g., at least a modeling material formulation that comprises a biological component as described herein) are dispensed, that is, a temperature of a dispensing head in the AM system and/or a temperature at which the modeling material formulation is maintained prior to passing in the dispensing head.

In some of any of the embodiments described herein, the AM process is performed without cooling the AM system (e.g., without cooling the dispensing heads and/or a modeling material formulation), to a temperature below room temperature, e.g., a temperature lower than 20 °C or lower than 10 °C, or lower than 5 °C (e.g., 4 °C).

In some of any of the embodiments described herein, the AM system is devoid of means for cooling the system or a part thereof (e.g., means for cooling the dispensing heads and/or the modeling material formulation), to a temperature below room temperature, e.g., a temperature lower than 20 °C or lower than 10 °C, or lower than 5 °C (e.g., 4 °C).

In some of any of the embodiments described herein, the additive manufacturing process (bio-printing) is performed while applying a shear force that does not adversely affect structural and/or functional properties of biological components (e.g., cells). Applying the shear force can be effected by passing the building material (e.g., at least a modeling material formulation that comprises a biological component as described herein) through the dispensing head, and is to be regarded also as subjecting the building material to shear force.

According to some embodiments, the AM bioprinting processes is performed under conditions that do not affect the functional and/or structural features of biological components included in the bio-ink (e.g., at low shear force and room temperature or a physiological temperature), while maintaining the required fluidity (a viscosity that imparts fluidity, e.g., lower than 10,000 centipoises or lower than 5,000 centipoises, or lower than 2,000 centipoises), and while further maintaining the curability of the dispensed building material.

A bioprinting method and a corresponding system can be any of the methods and systems known in the art for performing additive manufacturing, and exemplary such systems and methods are described hereinabove. A suitable method and system can be selected upon considering its printing capabilities, which include resolution, deposition speed, scalability, bio-ink compatibility and ease-of-use.

Exemplary suitable bio-printing systems usually contain a dispensing system (either equipped with temperature control module or at ambient temperature), and stage (a receiving medium), and a movement along the x, y and z axes directed by a CAD-CAM software. A curing source (e.g., a light or heat source) which applies a curing energy (e.g., by applying light or heat radiation) or a curing condition to the deposition area (the receiving medium) so as to promote curing of the formed layers and/or a humidifier, can also be included in the system. There are printers that use multiple dispensing heads to facilitate a serial dispensing of several materials.

Generally, bio-printing can be effected using any of the known techniques for additive manufacturing. The following lists some exemplary additive manufacturing techniques, although any other technique is contemplated.

<u>3D Inkjet printing:</u>

3D Inkjet printing is a common type of 3D printer for both non-biological and biological (bioprinting) applications. Inkjet printers use thermal or acoustic forces to eject drops of liquid onto a substrate, which can support or form part of the final construct. In this technique, controlled volumes of liquid are delivered to predefined locations, and a high-resolution printing with precise control of (1) ink drops position, and (2) ink volume, which is beneficial in cases of microstructure-printing or when small amounts of bioreactive agents or drugs are added, is received. Inkjet printers can be used with several types of ink, for example, comprising multiple types of biological components and/or bioactive agents. Furthermore, the printing is fast and can be applied onto culture plates.

A bio-printing method that utilizes a 3D inkjet printing system can be operated using one or more bio-ink modeling material formulations as described herein, and dispensing droplets of the formulation(s) in layers, on the receiving medium, using one or more inkjet printing head(s), according to the 3D printing data.

Extrusion printing:

This technique uses continuous beads of material rather than liquid droplets. These beads of material are deposited in 2D, the stage (receiving medium) or extrusion head moves along the z axis, and the deposited layer serves as the basis for the next layer. The most common methods for biological materials extrusion for 3D bioprinting applications are pneumatic or mechanical dispensing systems

Stereolithography (SLA) and Digital Light Processing (DLP):

SLA and DLP are additive manufacturing technologies in which an uncured building material in a bath is converted into hardened material(s), layer by layer, by selective curing using a light source while the uncured material is later separated/washed from the hardened material. SLA is widely used to create models, prototypes, patterns, and production parts for a range of industries including for Bioprinting.

Laser-assisted printing:

Laser-assisted printing technique, in the version adopted for 3D bioprinting, and is based on the principle of laser-induced forward transfer (LIFT), which was developed to transfer metals and is now successfully applied to biological material. The device consists of a laser beam, a focusing system, an energy absorbing /converting layer and a biological material layer (e.g., cells and/or hydrogel) and a receiving substrate. A laser assisted printer operates by shooting a laser beam onto the absorbing layer which convert the energy into a mechanical force which drives tiny drops from the biological layer onto the substrate. A light source is then utilized to cure the material on the substrate.
Laser assisted printing is compatible with a series of viscosities and can print mammalian cells without affecting cell viability or cell function. Cells can be deposited at a density of up to $10^8$ cells/ml with microscale resolution of a single cell per drop.

Electrospinning:

Electrospinning is a fiber production technique, which uses electric force to draw charged threads of polymer solutions, or polymer melts.

***Curable formulations:***

According to some of any of the embodiments described herein, the composite scaffold is formed by bioprinting one or more curable modeling material formulations, and, if needed, support material formulations, according to the embodiments described hereinabove with respect to the additive manufacturing process.
For the sake of simplicity, the components of the one or more curable modeling formulations are described herein as components of a curable formulation, but it is to be understood that the components can be divided into two or more curable modeling material formulations, according to the selected configured pattern, as described herein.
According to some of any of the embodiments described herein, the curable formulation comprises a curable collagen, wherein the collagen is as described herein in any of the respective embodiments and any combination thereof.
The curable formulation comprises recombinant human collagen (rhCollagen) that features one or more curable moiety/ies or group(s), which is also referred to herein as curable rhCollagen.
By "curable" it is meant herein a material that is capable of undergoing curing, or hardening, as defined herein, when exposed to a suitable curing condition.
A curable material is typically hardened or cured by undergoing polymerization and/or cross-linking.
Curable materials are typically polymerizable materials, which undergo polymerization and/or cross-linking when exposed to a suitable curing condition or a suitable curing energy (a suitable energy source). Alternatively, curable materials are thermo-responsive materials, which solidify or harden upon exposure to a temperature change (e.g., heating or cooling). Optionally, curable materials are made of small particles (e.g., nanoparticles or nanoclays) which can undergo curing to form a hardened material. Further optionally, curable materials are biological materials which undergo a reaction to form a hardened or solid material upon a biological reaction (e.g., an enzymatically-catalyzed reaction).
In some of any of the embodiments described herein, a curable material is a photopolymerizable material, which polymerizes and/or undergoes cross-linking upon exposure to radiation, as described herein, and in some embodiments the curable material is a UV-curable material, which polymerizes or undergoes cross-linking upon exposure to UV-vis radiation, as described herein.
In some of any of the embodiments described herein, when a curable material is exposed to a curing condition (e.g., radiation, reagent), it polymerizes by any one, or combination, of chain elongation, entanglement and cross-linking.

The cross-linking can be chemical and/or physical.

In some of any of the embodiments described herein, a curable material can be a mono-functional curable material or a multi-functional curable material.

Herein, a mono-functional curable material comprises one curable group or moiety - a functional group or moiety that can undergo polymerization, entanglement and/or cross-linking when exposed to a curing condition (e.g., radiation, presence of calcium ions).

A multi-functional curable material comprises two or more, e.g., 2, 3, 4 or more, curable groups. Multi-functional curable materials can be, for example, di-functional, tri-functional or tetra-functional curable materials, which comprise 2, 3 or 4 curable groups, respectively.

By "curable group" it is meant herein a functional group that is capable of undergoing polymerization and/or cross-linking when exposed to a suitable curing condition.

By "curable collagen" it is meant herein a collagen as described herein in any of the respective embodiments (human recombinant collagen), which features one or more curable groups as defined herein. According to some of any of the embodiments described herein, the curable collagen is a multi-functional curable material that comprises a plurality of curable groups, as defined herein.

The terms "curable collagen", "curable rhCollagen" and "rhCollagen featuring one or more (or at least one) curable groups" are used herein interchangeably.

According to some of any of the embodiments described herein, the curable collagen comprises an amino acid sequence as described herein in any of the respective embodiments, and features one or more, preferably a plurality of, curable groups generated at least a portion of the amino acid residues forming the collagen, preferably by covalent attachment, directly or via a linker, of a compound that comprises a curable group to functional groups of the side chains of the amino acid residues. Alternatively, or in addition, curable groups can be generated at the N-terminus and/or C-terminus of one or more the units forming the collagen, for example, by covalent attachment, directly or via a linker, of a compound that comprises a curable group to a respective amine or carboxylate.

According to some of any of the embodiments described herein, at least a portion of the curable groups in a curable collagen as described herein are cross-linkable groups, which undergo cross-linking when exposed to a curing condition. Such a curable collagen is also referred to herein as a cross-linkable rhCollagen (e.g., rhCollagen produced by a plant as described herein).

In some embodiments, curable groups can undergo polymerization and/or cross-linking via free-radical mechanism.

Exemplary such curable groups include acrylic groups, including acrylate, methacrylate, acrylamide and methacrylamide groups. Other free-radical curable groups may include thiols, vinyl ethers and other groups that feature a reactive double bond.

In some embodiments, curable groups can undergo polymerization and/or cross-linking via other mechanisms, such as cationic polymerization, or (cationic or anionic) ring opening polymerization. Exemplary such curable groups include, but are not limited to, epoxy-containing groups, caprolactam, caprolactone, oxetane, and vinyl ether.

Other curable groups can include, for example, formation of amide bonds between functional carboxylate and amine group (each being a curable group that reacts with the other and can effect cross-linking); formation of urethane between isocyanate groups and hydroxyl groups via polycondensation in the presence of a catalyst and/or upon exposure to UV radiation; and formation of disulfide bonds between two thiols.

Any other curable groups are contemplated.

The curable groups in the curable collagen can be generated by means of chemical reactions between a material that comprises or can generate the curable group(s) when reacted with chemically-compatible functional groups present in the collagen, as described herein, either directly, or be means of a spacer or a linker, using chemistries well known in the art. For example, a material that comprises a curable group and a functional group can be reacted with a compatible functional group in the collagen, for example, a functional group in an amino acid side chain, such that the curable group is a substituent of the amino acid side chain.

In some embodiments, a compatible functional group is first generated within the collagen by chemical modification of inherent chemical groups of the collagen, and is then reacted with a material that comprises or generates a curable group upon the reaction.

Whenever a curable collagen comprises more than one curable groups, the curable groups can be the same of different.

According to some of any of the embodiments described herein, at least a portion, or all, of the curable groups in a curable collagen of the present embodiments are photopolymerizable groups (e.g., UV-curable groups) that are capable of undergoing polymerization and/or cross-linking upon exposure to irradiation as described herein.

According to some of any of the embodiments described herein the curable group is a photocurable or photopolymerizable group (e.g., an acrylate or methacrylate).

A curable collagen as described herein that features acrylic (e.g., methacrylate) groups is also referred to herein as acrylated or methacrylated or (meth)acrylated rhCollagen e.g., (meth)acrylated rhCollagen.

Alternatively, or in addition, the curable group is a thiol-containing group, which provides a disulfide bridge upon curing.

A curable rhCollagen as described herein that features thiol-containing groups is also referred to herein as thiolated rhCollagen.

Alternatively, or in addition, the curable group or moiety is cured upon undergoing a chemical reaction, such as glycation of conjugation (using coupling agents such as EDC).

According to some embodiments, the curable groups comprise an amine and a carboxyl group which form peptide bonds upon curing.

According to some of any of the embodiments described herein, at least a portion, or all, of the curable groups in a curable collagen of the present embodiments are acrylic groups, as defined herein, for example, methacrylic groups.

According to some of any of the embodiments described herein, an acrylic group such as methacrylamide can be generated by reacting an acrylate or methacrylate (e.g., acrylic acid, methacrylic acid, acrylic or methacrylic ester, acrylic or methacrylic anhydride) with an amine functional group (of, for example, lysine residues).

According to some of any of the embodiments of the present invention, the number of the curable groups in a curable collagen as described herein can determine the degree of curing (e.g., the degree of cross-linking) and can be manipulated in order to achieve a desired curing (e.g., cross-linking) degree.

The curing degree affects the mechanical and/or physical and/or biological properties of the hardened material and can be manipulated in order to provide the composite scaffold with desired properties

According to some of any of the embodiments described herein, the curable collagen features a plurality of acrylamide or methacrylamide curable groups generated by reacting with lysine residues as described herein.

According to some of any of the embodiments described herein, the curable collagen features a plurality of acrylamide or methacrylamide curable groups substituting the amine groups of lysine residues in the collagen.

In some embodiments, at least 50 %, or at least 60 %, or at least 70 %, of the lysine residues in the collagen are substituted by a methacrylamide or acrylamide group. In some embodiments, the curable collagen features from 70 % to 100 %, or from 80 % to 100 %, or from 90 % to 100 %, of its lysine residues substituted by a methacrylamide or acrylamide group, including any intermediate values and subranges therebetween.

The rhCollagen-containing modeling material formulation further comprises one or more additional materials, including one or more additional curable materials.

The printing media (the building material) comprises one or more additional materials, including, one or more additional curable materials.

According to some of any of the embodiments described herein, the additional materials are included in the rhCollagen-containing formulation.

Additional curable materials that can be included in the rhCollagen formulation can be any curable material as defined herein, and is preferably a biocompatible material.

The curable formulation further comprises a biocompatible synthetic material that features a curable moiety, which forms a biocompatible synthetic polymer upon hardening (e.g., upon exposure to a curing condition as described herein), and which is also referred to herein as a curable biocompatible synthetic polymer or as a curable synthetic polymer.

According to some of any of the embodiments described herein, the curable formulation comprises a biocompatible synthetic polymer that features one or more curable groups as described herein (e.g., polymerizable and/or cross-linkable groups), that is, a curable biocompatible synthetic polymer. Such a material is also referred to herein as a modified biocompatible synthetic polymer or as a modified derivative thereof or a photopolymerizable modified derivative thereof, or a curable derivative thereof, and typically provides, upon curing, a cross-linked biocompatible synthetic polymer, which is cross-linked with itself and/or with other components in the formulation (e.g., curable rhCollagen and/or cross-linking agents).

According to some of any of the embodiments described herein, the biocompatible synthetic polymer is or comprises polylactic acid (PLA), polyglycolic acid (PGA), polycaprolactone (PCL), poly (lactic-co-glycolic acid) (PLGA), polyethylene glycol (PEG), polyvinyl alcohol (PVA), poly(N-isopropylacrylamide) (PNIPAAm), poly-4-hydroxybutyrate (P4HB) or any copolymer thereof or a combination thereof.

According to some of any of the embodiments described herein, the curable formulation comprises a precursor that, upon exposure to a curing condition, polymerizes and/or cross-links to form the above-described biocompatible synthetic polymer.

According to some of any of the embodiments described herein, the curable formulation comprises one or more of a polylactic acid (PLA) that features a curable group, a polyglycolic acid (PGA) that features a curable group, a polycaprolactone (PCL) that features a curable group, a poly (lactic-co-glycolic acid) (PLGA) that features a curable group, a polyethylene glycol (PEG) that features a curable group, a polyvinyl alcohol (PVA) that features a curable group, a poly(N-isopropylacrylamide) (PNIPAAm), a poly-4-hydroxybutyrate (P4HB) that features a curable group, and a copolymer of any of the foregoing that features a curable group.

Each of the above-described polymers can feature one or more curable groups.

Each of the above-described polymers can feature one or more curable groups that form a part of its pristine structure or can be synthetically generated by attaching such a group directly or via a linker to one or more compatible functional groups of the polymer.

Synthetic polymers that feature one or more curable groups are also referred to herein interchangeably as modified synthetic polymers or a modified derivative thereof.

According to some of any of the embodiments described herein, the curable formulation comprises a modified PGA or a modified derivative thereof or a photopolymerizable modified derivative thereof, a modified PCL or a modified derivative thereof or a photopolymerizable modified derivative thereof, a modified PLGA or a modified derivative thereof or a photopolymerizable modified derivative thereof, a modified PEG or a modified derivative thereof or a photopolymerizable modified derivative thereof, a modified PVA or a modified derivative thereof or a photopolymerizable modified derivative thereof, PNIPAAm or a modified derivative thereof, or any combination thereof. Modifications include, but are not limited to, (meth)acrylation or thiolation of the biocompatible synthetic polymer. In some embodiments, modified PEG comprises poly(ethylene glycol) diacrylate (PEGDA).

In some of any of the embodiments described herein, the curable formulation comprises one or more multi-functional curable materials (monomeric or polymeric) that form a biocompatible synthetic polymer when hardened or which react with a biocompatible synthetic polymer present in the formulation. Such materials act, and are also referred to herein, as a cross-linker, or a cross-linker agent or a cross-linking agent. The cross-linking agent can interact with a synthetic polymer (e.g., a modified synthetic polymer) and/or with the curable collagen as described herein, to form a cross-linked polymeric network. An exemplary cross-linking agent comprises ethoxylated (15) trimethylolpropane triacrylate, such as marketed under the tradename SR9035. Similar multi-functional (e.g., di- or tri-functional cross-linking agents are contemplated. In some embodiments, the cross-linking agent is a multi-functional PEG (e.g., PEG-diacrylate or PEG tri-acrylate).

According to some of any of the embodiments described herein, the curable formulation comprises a biocompatible synthetic polymer, as described herein, which is capable of undergoing curing, e.g., by means of cross-linking, when exposed to a curing condition in the presence of other components in the formulation (e.g., a cross-linking agent as described herein and/or rhCollagen).

According to some of any of the embodiments described herein, the curable formulation comprises a curable rhCollagen as described herein in any of the respective embodiments and any combination thereof, and one or more biocompatible synthetic polymers that feature, independently, one or more curable groups, as described herein in any of the respective embodiments and any combination thereof. In some embodiments, the curable formulation further comprises a cross-linking agent as described herein.

Alternatively, the curable formulation comprises a curable rhCollagen as described herein in any of the respective embodiments and any combination thereof, and one or more biocompatible synthetic polymers, as described herein in any of the respective embodiments and any combination thereof, that is capable of undergoing curing by, for example, cross-linking, and further comprises a cross-linking agent as described herein that promotes curing of the synthetic polymers by cross-linking the polymer, or by cross-linking the polymer with one or more other components in the formulation (e.g., curable rhCollagen, other curable materials as described herein, and/or other polymers).

According to some of any of the embodiments described herein, each of the biocompatible synthetic polymers that feature one or more curable groups has, independently, an average molecular weight (Mw or Mn) that ranges from about 100 Daltons to about 100 kDa, including any intermediate values and subranges therebetween. In the some embodiments, the synthetic polymer has a Mn of from about 400 Da to about 100 kDa, or from about 400 Da to about 50 kDa, or from about 500 Da to about 50 kDa, or from about 100 Da to about 10 kDa, or from about 500 Da to about 10 kDa, or from about 1,000 Da to about 10 kDa, or from about 100 Da to about 5 kDa, or from about 1000 Da to about 5 kDa, or from about 2 kDa to about 10 kDa, or from about 5 kDa to about 10 kDa, including any intermediate values and subranges therebetween.

According to some of any of the embodiments described herein, the ratio of a curable rhCollagen (rhCollagen that feature a curable group) to a curable synthetic polymer (a biocompatible synthetic polymer that features a curable group and/or a biocompatible synthetic polymer that is otherwise capable of undergoing cross-linking) in the curable formulation can be from about 10:1 to about 1:10, including any intermediate values and subranges therebetween, and can be, for example, from about 1:0.5 to about 1:2.0, or from about 5:1 to about 1:5, or from about 2:1 to 1:2, or from about 1:1 to 1:10, or from about 1:1 to 1:5, or from about 1:1 to 2:1, or from about 1:1 to 3:2, or from about 3:2 to 2:1.

In some embodiments, the ratio of curable rhCollagen to curable biocompatible synthetic polymer is about 1:0.5, or 1:0.6, or 1:0.7, or 1:0.8, or 1:0.9, or 1:1, or 1:1.1, or 1:1.2, or 1:1.3, or 1:1.4, or 1:1. or 1:1.6, or 1:1.7, or 1:1.8, or 1:1.9, or 1:2.0.

According to some of any of the embodiments described herein, the curable formulation further comprises at least one extracellular matrix (ECM) component, for example, an ECM protein, including, but not limited to, fibrinogen,

26

collagen, fibronectin, vimentin, microtubule-associated protein 1D, Neurite outgrowth factor (NOF), bacterial cellulose (BC), laminin and gelatin. In some embodiments, an ECM component comprises (in addition to rhCollagen), fibronectin, hyaluronic acid (HA), heparin, elastin, or laminin, or any combination thereof, as described in detail herein.

In some of these embodiments, the ECM component features one or more curable groups as described herein (and is also referred to herein as a curable ECM component, or a modified ECM component or a cross-linkable ECM component or a polymerizable ECM component). The curable group can be covalently attached to functional groups of the ECM component, directly or via a linker.

For example, the formulation can further comprise one or more of HA, modified HA or a polymerizable modified derivative thereof, for example, (meth)acrylated or thiolated HA.

In some embodiments, a curable ECM component comprises a modified fibronectin (e.g., (meth)acrylated or thiolated fibronectin). In some embodiments, a curable ECM component comprises a modified heparin (e.g., (meth)acrylated or thiolated heparin). In some embodiments, a curable ECM component comprises modified elastin (e.g., (meth)acrylated or thiolated elastin). In some embodiments, a curable ECM component comprises modified laminin (e.g., (meth)acrylated or thiolated laminin). Optionally, the formulation can comprise an ECM component that is capable of undergoing curing, e.g., by means of cross-linking, in the presence of a cross-linking agent, as described herein in any of the respective embodiments. Such a curable ECM component can feature one or more curable groups as described herein, or not.

According to some of any of the embodiments described herein, the curable modeling formulation further comprises an integrin-binding material, for example, an RGD-containing material, including cyclic-RGD-containing materials. In some embodiments, the integrin-binding material, e.g., an RGD-containing material, is a curable material that is capable of undergoing polymerization and/or cross-linking when exposed to a curing condition as described herein. The curable RGD-containing material can feature curable groups or can be used with a cross-linking agent and is cross-linkable in the presence of the cross-linking agent.

In some of these embodiments, an integrin-binding material is curable, and features one or more curable moieties or groups.

Accordingly, in some embodiments, an integrin-binding material comprises a least one Arg-Gly-Asp (RGD) moiety, or a peptidomimetic thereof , and can optionally further include other amino acids, amino acid derivatives, or other chemical groups (e.g., alkylene chains) and/or one or more curable groups as described herein in any of the respective embodiments and any combination thereof.

In some embodiments, the RGD-containing material is an oligopeptide. The oligopeptide can be a cyclic oligopeptide (including, for example, monocyclic, bicyclic and tricyclic oligopeptides) or a linear oligopeptide, and can include, in addition to the Arg-Gly-Asp amino acid sequence, from 1 to 10 amino acids.

An exemplary oligopeptide is a cyclic peptide being or comprising c[Arg-Gly-Asp-Phe-Lys].

In some embodiments, the integrin-binding material comprises two or more Arg-Gly-Asp-containing moieties, wherein the moieties can be the same or different.

Exemplary Arg-Gly-Asp-containing materials include, but are not limited to c(RGDfk), RGD4C, and other RGD-containing cyclic peptides such as those described in Haubner et al. [J. Am. Chem. Soc. 1996, 118, 7881-7891] and Capello, et al. [J. Nucl. Med. 2004, 45(10), 1716-20] and in WO 97/06791 and U.S. Patent No. 5,773,412.

In some embodiments, the RGD-containing material can comprise two or more -Arg-Gly-Asp- moieties, being either linked to one another or being spaced by one or more amino acids or any other spacer, as defined herein.

In exemplary embodiments, the RGD-containing material comprises one or more cysteine residue(s), and in some exemplary embodiments it is RGD4C. Such an RGD-containing material can undergo cross-linking by means forming intermolecular disulfide bonds with its self and/or with other thiolated curable components in the formulation.

In some embodiments, any of the RGD-containing materials as described herein comprises one or more curable groups or moieties as described herein, for example, one or more (meth)acrylic groups, attached to functional group(s) within amino acid side chains and/or to the terminal amine or carboxylate, as described herein.

Alternatively, the RGD-containing material comprises one or more thiol groups, for example, thiol groups of a cysteine residue, which render it a curable material. Optionally, thiol groups can be attached to the RGD-containing material directly or via a linker.

Alternatively or in addition, any of the RGD-containing materials as described herein, whether comprising a curable group or moiety or nor, can be attached to a polymeric material, preferably a biocompatible synthetic polymer as described herein in any of the respective embodiments, or to a hydrogel forming material, or to an ECM component, as described herein in any of the respective embodiments. One or more RGD-containing moieties or materials can be attached to the polymeric backbone of these material, directly or via a linker, and can be attached to one or more of the terminal units and/or backbone units. The polymer can feature curable groups as described herein, or is capable of undergoing cross-linking in the presence of a cross-linking agent. Alternatively or in addition, the RGD-containing moieties feature one or more curable groups, as described herein (e.g., thiol and/or (meth)acrylic groups).

An exemplary RGD-containing material comprises one or more RGD-containing sequences as described herein, covalently attached to PEG, for example, to PEG-DA or PEG-TA, as described herein in any of the respective embodiments. According to some of any of the embodiments described herein, a curable moiety or group of the rhCollagen and a curable moiety or group of the synthetic polymer are curable when subjected to the same curing condition. In some of these embodiments, the curing condition comprises irradiation (illumination), and these curable moieties or groups are photocurable (photopolymerizable or light-polymerizable) moieties or groups.

According to some of any of the embodiments described herein, a curable moiety or group of the rhCollagen and a curable moiety or group of the synthetic polymer, and a curable moiety or group of the ECM component, if present, and a curable moiety or group of the integrin-binding material, if present, are curable when subjected to the same curing condition. In some of these embodiments, the curing condition comprises irradiation (illumination), and these curable moieties or groups are photocurable (photopolymerizable) moieties or groups.

According to some of any of the embodiments described herein, the modeling material formulation features a viscosity of no more than 2,000 centipoises, or no more than 1,500 centipoises, at a zero shear rate, at 37 °C, when determined using a rheometer (e.g., a Brookfield rheometer) according to methods well known in the art.

According to some of any of the embodiments described herein, the modeling material formulation features a viscosity of no more than 2000 centipoises, or no more than 1,500 centipoises, at a shear rate of 5 1/sec, at room temperature, when determined using a rheometer as described herein.

In some of any of the embodiments described herein, a concentration of the curable recombinant human collagen in the modeling material formulation containing same ranges from 0.5 mg/ml to 50 mg/ml, or from 0.5 mg/ml to 20 mg/ml, or from 1 mg/ml to 50 mg/ml, or from 1 mg/ml to 50 mg/ml, or from 1 mg/ml to 40 mg/ml, or from 1 mg/ml to 30 mg/ml, or from 2 mg/ml to 20 mg/ml, or from 5 mg/ml to 15 mg/ml, or from 1 mg/ml to 10 mg/ml, including any intermediate values and subranges therebetween.

In some of any of the embodiments described herein, a total concentration of the curable biocompatible synthetic polymer(s) in the modeling material formulation containing same ranges from 1 mg/ml to 500 mg/ml, or from 10 mg/ml to 500 mg/ml, or from 1 mg/ml to 100 mg/ml, or from 10 mg/ml to 100 mg/ml, or from 50 mg/ml to 500 mg/ml, or from 50 mg/ml to 300 mg/ml, or from 1 mg/ml to 20 mg/ml, or from 5 mg/ml to 50 mg/ml, including any intermediate values and subranges therebetween.

In some of any of the embodiments described herein, a total concentration of the curable ECM component(s) in the modeling material formulation containing same, if present, ranges from 0.01 mg/ml to 10 mg/ml, or from 0,01 mg/ml to 5 mg/ml, or from 0.01 mg/ml to 1 mg/ml, or from 0.05 mg/ml to 1 mg/ml, including any intermediate values and subranges therebetween.

In some of any of the embodiments described herein, a total concentration of the curable integrin-binding material, if present, in the modeling material formulation containing same is stoichiometric relative to the curable rhCollagen, and can range, for example, from 1 to 50, or from 1 to 40, or from 1 to 30, $\mu$M, including any intermediate values and subranges therebetween.

According to some of any of the embodiments described herein, a concentration of the curable recombinant human collagen in the modeling material formulation containing same ranges from 0.01 to 10 %, or from 0.01 to 5 %, 0.05 to 10 %, or from 0.05 to 5 %, or from 0.1 to 2 %, or from 0.1 to 1 %, or alternatively, from 10 to 50 %, or from 10 to 40 %, or from 10 to 30 %, or from 10 to 20 %, or from 20 to 40 %, or from 30 to 40 %, by weight, of the total weight of the formulation, including any intermediate values and subranges therebetween.

According to some of any of the embodiments described herein, a concentration of the curable biocompatible synthetic polymer (or a precursor curable material thereof) in the modeling material formulation containing same ranges from 0.1 to 10 %, or from 0.1 to 5 %, or from 0.1 to 2 %, or from 0.5 to 2 %, or from 1 to 60 %, or from 1 to 50 %, or from 10 to 60 %, or from 5 to 50 %, or from 10 to 40 %, or from 5 to 30 %, or from 10 to 20 %, or from 5 to 15 %, by weight, of the total weight of the formulation's components (not including a carrier as described hereinafter), including any intermediate values and subranges therebetween.

According to some of any of the embodiments described herein, a concentration of the ECM component featuring a curable group as described herein in any of the respective embodiments ranges from 0.001-0.1 %, by weight of the total weight of the formulation's components (not including a carrier as described hereinafter), including any intermediate values and subranges therebetween.

According to some of any of the embodiments described herein, a curable (modeling; bio-ink) formulation comprises:

Curable rhCollagen as described herein in any of the respective embodiments and any combination, for example, rhCollagen as described herein (e.g., plant-derived) featuring a plurality of (meth)acrylic groups as described herein;
A curable biocompatible synthetic polymer as described herein in any of the respective embodiments and any combination thereof, for example, one or more of a polylactic acid (PLA), a polyglycolic acid (PGA), a polycaprolactone (PCL), a poly (lactic-co-glycolic acid) (PLGA), a polyethylene glycol (PEG), a polyvinyl alcohol (PVA), a poly(N-isopropylacrylamide) (PNIPAAm), a Poly-4-hydroxybutyrate (P4HB) or any copolymer thereof, which features one or more curable groups (e.g., (meth)acrylic and/or thiol) groups, as described herein in any of the respective embodiments and any combination thereof;

A curable ECM component, which comprises an ECM component as described herein in any of the respective embodiments (e.g., one or more of hyaluronic acid, fibronectin, heparin, elastin, or laminin) which features one or more or more curable groups (e.g., (meth)acrylic or thiol) groups, as described herein in any of the respective embodiments and any combination thereof, and/or which is used in combination with a cross-linking agent, as described herein; and a curable RGD-containing material, as described herein in any of the respective embodiments.

In some of these embodiments, the formulation further comprises an aqueous carrier (e.g., water, optionally in combination with a water-soluble organic acid), and a concentration of the curable rhCollagen ranges from 1 to 10 mg/ml, a concentration of the curable synthetic polymer ranges from 50 to 300 mg/ml, a concentration of the ECM component ranges from 0 to 1 mg/ml; and the amount of the RGD-containing material is stoichiometrically adjusted to the rhCollagen.

Exemplary formulations are described in the Examples section that follows.

An exemplary formulation according to some embodiments of the present invention comprises from 0.1% to 1 % by weight curable rhCollagen, and from 5 to 15 % by weight curable synthetic polymer(s), as described herein, of the total weight of the formulation.

An exemplary formulation according to some embodiments of the present invention comprises from 0.1 to 0.5 % by weight curable rhCollagen, and from 10 to 15 % by weight curable synthetic polymer(s), as described herein, of the total weight of the formulation.

A curable biocompatible synthetic polymer in these exemplary formulations can be, for example, multi-functional (e.g., di-functional) PEG-acrylate (e.g., PEG-DA), which can be of one or more average Mn values, as described herein. For example, it can include, 1, 2, 3 or more PEG-DAs, each featuring a different Mn.

A concentration of the curable rhCollagen in a modeling material formulation containing same can affect the rheological properties of the formulation and of the hardened formulation obtained upon dispensing, and can be manipulated in accordance with AM methodology and conditions employed and desired properties of the final object or a portion thereof. Similarly, a concentration and type (e.g., amount of polymerizable groups, average Mn) of the curable biocompatible synthetic polymer (or a curable precursor thereof) can affect the rheological properties of the formulation and of the hardened formulation obtained upon dispensing, and can be manipulated in accordance with AM methodology and conditions employed and desired properties of the final object or a portion thereof.

According to the present embodiments, the building material comprises at least one modeling material formulation that comprises a curable recombinant human collagen, as described herein in any of the respective embodiments. Such a modeling material formulation is also referred to herein as rhCollagen-containing formulation.

According to some of any of the embodiments described herein, the curable formulation further comprises a carrier, and in some of these embodiments, the carrier is an aqueous carrier.

The aqueous carrier can be water, a buffer featuring pH in a range of from about 4 to about 10, or from about 6 to about 8, or from about 7 to about 7.4, a basic aqueous solution or an acidic aqueous solution.

The aqueous carrier can comprise salts and other water-soluble materials at varying concentrations. In some embodiments, a concentration of a salt in the carrier ranges from about 0.1 mM to about 0.2 M, or from about 0.1 mM to about 0.1 M, or from about 0.1 mM to about 100 mM, or from about 0.1 mM to about 50 mM, or from about 0.1 mM to about 20 mM, including an intermediate values and subranges therebetween.

In some embodiments, the aqueous carrier comprises salts at physiologically acceptable concentrations, such that the formulation features osmolarity around a physiological osmolarity.

In some embodiments the aqueous carrier comprises a phosphate salt, for example, a sodium phosphate monobasic ($NaH_2PO_4$) and/or a sodium phosphate dibasic (sodium hydrogen phosphate; $Na_2HPO_4$). In some embodiments, the total concentration of the phosphate salt(s) is about 0.1 M.

In some embodiments, the aqueous carrier comprises NaCl or any other physiologically acceptable salt.

In some embodiments, the aqueous carrier comprises a phosphate buffer and in some embodiments, the aqueous carrier comprises a phosphate buffer saline, which comprises sodium phosphate monobasic and/or sodium phosphate dibasic and NaCl.

The phosphate buffer saline (PBS) can be a commercially available PBS (e.g., DPBS) or a custom-made buffer featuring a desirable pH and/or osmolarity.

In exemplary embodiments, the aqueous carrier comprises a phosphate buffer that comprises a phosphate sodium salt as described herein at a concentration of about 0.1M and NaCl at a concentration of from about 0 mM to about 200 mM, including any intermediate value and subranges therebetween.

Any other buffers are also usable in the context of the present embodiments.

In some of any of the embodiments described herein, the aqueous carrier comprises an acid.

In some embodiments, a concentration of the acid is lower than 100 mM, and can be, for example, of from 0.1 mM to 50 mM, or from 0.1 mM to 30 mM, or from 0.1 mM to 40 mM, or from 0.1 mM to 30 mM, or from 1 to 30 mM, or from 10 to 30 Mm, including any intermediate values and subranges therebetween.

In some embodiments, a concentration of the acid ranges from 0.01 to 0.1, or from 0.01 to 0.05, % by weight, including any intermediate values and subranges therebetween.

The acid can be an inorganic acid (e.g., HCl) or an organic acid, preferably which is water soluble at the above-indicated concentrations (e.g., acetic acid or picric acid).

Non-curable materials, other than the biological materials as described herein, can also be included in one or more modeling formulations as described herein and can be, for example, materials that impart a certain property to the formulation or to the hardened formulation and to the composite scaffold formed thereby. Such a property can be a physical property (e.g., an optical property such as transparency or opacity, color, a spectral property, heat resistance, electrical property and the like), or a mechanical or rheological property such as viscosity, elasticity, storage modulus, loss modulus, stiffness, hardness, and the like.

Exemplary non-curable materials include thixotropic agents, reinforcing agents, toughening agents, fillers, colorants, pigments, dyes, etc.

According to some of any of the embodiments described herein the formulation features a neutral pH (e.g., from about 6 to about 8).

According to some of any of the embodiments described herein the formulation features viscosity parameters essentially as described herein.

In embodiments where two or more modeling material formulations are used, two of more formulations are rhCollagen containing formulations as described herein, which differ from one another by the presence, type and/or concentration of an additional material that is included therein. For example, one formulation can comprise curable rhCollagen, and another formulation can comprise curable rhCollagen and one or more of the additional curable materials (e.g., a curable synthetic polymer) as described herein. For example, one formulation can comprise curable rhCollagen and one additional curable material, and another formulation can comprise curable rhCollagen and another additional curable material as described herein. For example, one formulation can comprise curable rhCollagen and one additional curable material, and another formulation can comprise curable rhCollagen and a non-curable material as described herein. Any other combinations are contemplated.

In some of any of the embodiments described herein, all the curable materials in the building material are cured under the same curing condition. In some embodiments, all curable materials are photocurable.

In some of any of the embodiments described herein, a modeling material formulation that comprises a curable material further comprises an agent that promotes curing or hardening of the curable material when exposed to a curing condition.

The concentration of the agent can be determined in accordance with the concentration of the curable material and the desired degree of curing (e.g., desired cross-linking degree).

When the curable materials are photocurable materials, the agent is a photoinitiator. The photoinitiator is selected in accordance with the curing mechanism (e.g., free-radical, cationic, etc.).

A free-radical photoinitiator may be any compound that produces a free radical on exposure to radiation such as ultraviolet or visible radiation and thereby initiates a polymerization reaction. Non-limiting examples of suitable photo-initiators include benzophenones (aromatic ketones) such as benzophenone, methyl benzophenone, Michler's ketone and xanthones; acylphosphine oxide type photo-initiators such as 2,4,6-trimethylbenzolydiphenyl phosphine oxide (TMPO), 2,4,6-trimethylbenzoylethoxyphenyl phosphine oxide (TEPO), Lithium phenyl-2,4,6-trimethylbenzoylphosphinate and bisacylphosphine oxides (BAPO's); benzoins and bezoin alkyl ethers such as benzoin, benzoin methyl ether and benzoin isopropyl ether and the like.

Exemplary photoinitiators include, but are not limited to, those of the Irgacure® family, riboflavin, rose Bengal, and more.

A free-radical photo-initiator may be used alone or in combination with a co-initiator. Co-initiators are used with initiators that need a second molecule to produce a radical that is active in the photocurable free-radical systems. Benzophenone is an example of a photoinitiator that requires a second molecule, such as an amine, to produce a free radical. After absorbing radiation, benzophenone reacts with a ternary amine by hydrogen abstraction, to generate an alpha-amino radical which initiates polymerization of acrylates. Non-limiting example of a class of co-initiators are alkanolamines such as triethylamine, methyldiethanolamine and triethanolamine.

Suitable cationic photoinitiators include, for example, compounds which form aprotic acids or Bronsted acids upon exposure to ultraviolet and/or visible light sufficient to initiate polymerization. The photoinitiator used may be a single compound, a mixture of two or more active compounds, or a combination of two or more different compounds, i.e. co-initiators. Non-limiting examples of suitable cationic photoinitiators include aryldiazonium salts, diaryliodonium salts, triarylsulphonium salts, triarylselenonium salts and the like. An exemplary cationic photoinitiator is a mixture of triarylsolfonium hexafluoroantimonate salts.

Non-limiting examples of suitable cationic photoinitiators include P-(octyloxyphenyl) phenyliodonium hexafluoroantimonate UVACURE 1600 from Cytec Company (USA), iodonium (4-methylphenyl)(4-(2-methylpropyl)phenyl)-hexafluorophosphate known as Irgacure 250 or Irgacure 270 available from Ciba Speciality Chemicals (Switzerland), mixed arylsulfonium hexafluoroantimonate salts known as UVI 6976 and 6992 available from Lambson Fine Chemicals (England), diaryliodonium hexafluoroantimonate known as PC 2506 available from Polyset Company (USA), (tolylcumyl) iodonium tetrakis (pentafluorophenyl) borate known as Rhodorsil® Photoinitiator 2074 available from Bluestar Silicones (USA), iodonium bis(4-dodecylphenyl)-(OC-6-11)-hexafluoro antimonate known as Tego PC 1466 from Evonik Industries AG (Germany).

A concentration of the photoinitiator in the curable formulation can range from 0.1 to 3 %, or from 0.1 to 2 %, or from 0.5 to 2.5 %, or from 0.1 to 2 %, or from 0.5 to 1.5 %, by weight, of the total weight of the formulation.

***Chemical composition of the composite scaffold:***

The 3D bio-printed composite scaffold comprises a recombinant human collagen (rhCollagen) and a biocompatible synthetic polymer.

A skilled artisan would appreciate that the term "composite" or "composite scaffold" may encompass a scaffold comprising a combination of materials. In some embodiments, a composite scaffold is degradable. In some embodiments, a composite scaffold is biodegradable. In some embodiments, a composite scaffold is biocompatible. In some embodiments, a composite scaffold is partially degradable. In some embodiments, a composite scaffold is a mechanically degradable scaffold. In some embodiments, a composite scaffold comprises components which are biodegradable and components which are mechanically degradable. An artisan would appreciate that biocompatible components of a composite scaffold may be mechanically broken up into small pieces over a period of time which would then gradually become resorbed or metabolized in the body.

The composite scaffold comprises collagen. The collagen comprises rhCollagen. In some embodiments, collagen comprises a plant-derived rhCollagen. In some embodiments, collagen comprises a cross-linked curable rhCollagen as described herein which underwent cross-linking. In some embodiments, the collagen comprises a cross-linked, plant-derived recombinant human collagen, as described in detail herein.

The composite scaffold comprises a curable biocompatible synthetic polymer as described herein which underwent polymerization and/or cross-linking.

A skilled artisan would appreciate that the term "biocompatible" may encompass materials which are compatible with a biological tissue or organ without eliciting toxicity, an immune reaction, an injury or the like.

A skilled artisan would appreciate that the term "synthetic polymer" may encompass polymers that are not naturally occurring and/or that are not obtained from a naturally occurring substance, and are produced synthetically by chemical synthesis.

According to some of any of the embodiments described herein, the chemical composition of the composite scaffold is a result of subjecting a curable formulation as described herein in any of the respective embodiments and any combination thereof to a curing condition.

According to some of any of the embodiments described herein, all the curable groups or moieties in the components of the curable formulation are photo-curable (e.g., light-curable, photopolymerizable, UV-curable) and the chemical composition of the composite scaffold is a result of subjecting a curable formulation as described herein in any of the respective embodiments and any combination thereof to irradiation (illumination, e.g., UV-vis irradiation).

Without being bound by any particular theory, it is assumed that when forming the composite scaffold, as described herein in any of the respective embodiments, the curable materials, when exposed to a curing condition, cross-link to one another, to provide a hydrogel network, as described herein.

A skilled artisan would appreciate that the terms "cross-linked", "cross-linking", or "cross-linkable" refer to joining together of at least two molecules by a chemical interaction, including formation of a covalent bond, formation of hydrogen bonds, hydrophobic, hydrophilic, ionic or electrostatic interaction. A skilled artisan would further appreciate that there are various methods of cross-linking. In some embodiments, methods involve photo-reactive molecules and photo-induced reactions, by, for example, illumination with an infrared, UV, or white light source, which create cross-linked molecules. An artisan would appreciate that while crosslinking is used to enhance the stability and strength of the matrices and scaffolds comprising crosslinked molecules described herein, the crosslinking does not compromise the characteristic of the matrices and scaffolds to degrade or become resorbed or metabolized in the body over a period of time.

According to some of any of the embodiments described herein, when exposed to a curing condition (e.g., irradiation), the curable materials undergo cross-linking and/or polymerization, each alone (with the same curable component) and/or one with another, so as to form a network of cross-linked materials, optionally in a form of a hydrogel as described herein.

In some embodiments a composite scaffold comprises a cross-linked plant-derived human collagen and two different synthetic polymers (e.g., cross-linked polymers), three different synthetic polymers, four different synthetic polymers, five different synthetic polymers, six different synthetic polymers or seven different synthetic polymers. In some embodiments, a composite scaffold comprises at least two different synthetic polymers, at least three different synthetic polymers, at least four different synthetic polymers, at least five different synthetic polymers, at least six different synthetic polymers or at least seven different synthetic polymers. Two or more of the cross-linked rhCollagen and cross-linked polymers can be cross-linked to one another to form a cross-linked network as described herein.

The range and specific ratios of rhCollagen to a biocompatible synthetic polymer are as described hereinabove and in accordance with the ratios provided for a curable formulation as described herein in any of the respective embodiments.

In some embodiments, a composite scaffold comprises a crosslinked biocompatible synthetic polymer. In some embodiments, a crosslinked biocompatible synthetic polymer comprises crosslinked PLA, crosslinked PGA, crosslinked PCL, crosslinked PLGA, crosslinked PEG, crosslinked PEGDA, crosslinked PVA,, cross-linked Poly-4-hydroxybutyrate (P4HB), cross-linked PNIPAAm, or any combination thereof.

In some embodiments, a composite scaffold comprises (cross-linked) rhCollagen, a (e.g., cross-linked) synthetic polymer and at least one extracellular matrix (ECM) component, for example, a cross-linked ECM component. In some embodiments, an ECM component comprises in addition to rhCollagen, fibronectin, HA, heparin, elastin, or laminin, or any combination thereof, as described in detail herein. In some embodiments, HA comprises cross-linked HA. In some embodiments, heparin comprises cross-linked heparin. In some embodiments, elastin comprises cross-linked HA. In some embodiments, laminin comprises cross-linked laminin.

In some embodiments, a composite scaffold comprises two different ECM components, three different ECM components, four different ECM components, or five different ECM components. In some embodiments, a composite scaffold comprises at least two different ECM components, at least three different ECM components, at least four different ECM components, or at least five different ECM components. The rhCollagen, one or more biocompatible synthetic polymer(s) and one or more ECM component(s) can be cross-linked, such that at least two, at least three, or all of these materials are cross-linked to one another to form a network as described herein.

In some embodiments, collagen comprises cross-linked collagen. In some embodiments, rhCollagen comprises cross-linked rhCollagen. In some embodiments, a curable (e.g., (meth)acrylated) rhCollagen cross-links under the curing condition (e.g., radiation/illumination) only to itself. In some embodiments, a curable (e.g., (meth)acrylated) rhCollagen cross-links under the curing condition (e.g., irradiation/illumination) to another type of curable rhCollagen (e.g., thiolated rhCollagen). In some embodiments, a curable (e.g., (meth)acrylated and/or thiolated) rhCollagen cross-links under a curing condition to any other curable material in the curable formulation, as described herein in any of the respective embodiments (e.g., (meth)acrylated/thiolated curable components). In some embodiments, curable (e.g., (meth)acrylated) rhCollagen cross-links under a curing condition (e.g., irradiation/illumination) to a curable (e.g., (meth)acrylated) HA. In some embodiments, curable (e.g., thiolated) rhCollagen cross-links under a curing condition (e.g., irradiation/illumination) to thiolated HA. In some embodiments, a curable (e.g., (meth)acrylated) rhCollagen cross-links under a curing condition to a curable (e.g., (meth)acrylated) PVA. In some embodiments, a curable (e.g., thiolated) rhCollagen cross-links under a curing condition (e.g., irradiation/illumination) to a curable (e.g., thiolated) PVA. In some embodiments, a curable (e.g., (meth)acrylated) rhCollagen cross-links under a curing condition (e.g., irradiation/illumination) to a curable (e.g., (meth)acrylated) PEG. In some embodiments, a curable (e.g., thiolated) rhCollagen cross-links under a curing condition (e.g., irradiation/illumination) to a curable (e.g., thiolated) PEG.

In some embodiments, a (meth)acrylated rhCollagen cross-links under a curing condition (e.g., irradiation/illumination) to PEG-DA. In some embodiments, thiolated rhCollagen cross-links under a curing condition (e.g., irradiation/illumination) to PEG-DA. In some embodiments, (meth)acrylate rhCollagen cross-links under a curing condition (e.g., irradiation/illumination) to methacrylated or thiolated PLA. In some embodiments, thiolated rhCollagen cross-links under a curing condition (e.g., irradiation/illumination) to methacrylated or thiolated PLA. In some embodiments, methacrylate rhCollagen cross-links under a curing condition (e.g., irradiation/illumination) to methacrylated or thiolated PGA. In some embodiments, thiolated rhCollagen cross-links under a curing condition (e.g., irradiation/illumination) to methacrylated or thiolated PGA. In some embodiments, methacrylate rhCollagen cross-links under a curing condition (e.g., irradiation/illumination) to methacrylated or thiolated PCL. In some embodiments, thiolated rhCollagen cross-links under a curing condition (e.g., irradiation/illumination) to methacrylated or thiolated PCL. In some embodiments, methacrylate rhCollagen cross-links under a curing condition (e.g., irradiation/illumination) to methacrylated or thiolated PLGA. In some embodiments, thiolated rhCollagen cross-links under a curing condition (e.g., irradiation/illumination) to methacrylated or thiolated PLGA. In some embodiments, methacrylate rhCollagen cross-links under a curing condition (e.g., irradiation/illumination) to methacrylated or thiolated PNIPAAm. In some embodiments, thiolated rhCollagen cross-links under a curing condition (e.g., irradiation/illumination) to methacrylated or thiolated PNIPAAm.

In some embodiments, methacrylate rhCollagen cross-links under a curing condition (e.g., irradiation/illumination) to methacrylated OC. In some embodiments, methacrylate rhCollagen cross-links under a curing condition (e.g., irradiation/illumination) to thiolated OC. In some embodiments, methacrylate rhCollagen cross-links under a curing condition (e.g., irradiation/illumination) to N-(2-Hydroxyethyl)acrylamide (HEAA). In some embodiments, thiolated rhCollagen cross-links under a curing condition (e.g., irradiation/illumination) to N-(2-Hydroxyethyl)acrylamide (HEAA).

*A degradable Implant:*

According to an aspect of some embodiments of the present invention there is provided a soft tissue implant which comprises a composite scaffold as described herein in any of the respective embodiments and any combination thereof.

A skilled artisan would appreciate that the terms "implant", "implantable" may encompass any material which can be completely or partially, permanently or transiently, implanted, inserted, embedded, and/or grafted into a subject's (e.g., an animal or a human, preferably a mammal) body, including, for example, attached to a tissue, muscle, organ or any other part of the subject's body.

According to some of any of the respective embodiments, the soft tissue implant is a biocompatible, degradable implant, as these terms are defined herein throughout.

A skilled artisan would appreciate that the term "degradable", which is interchangeable with the terms "resorbable", may encompass the ability to be degraded or metabolized in a subject, over a period of time when implanted within a subject. In some embodiments, an implant described herein comprises a biodegradable implant. In some embodiments, an implant described herein comprises a mechanically degraded implant. In some embodiments, an implant described herein comprises both a biodegradable and a mechanically degradable implant. In some embodiments, an implant described herein comprises an implant that is naturally degradable.

According to some embodiments, an "implant" is a medical device manufactured to replace a missing biological structure, to support a damaged biological structure, and/or to enhance an existing biological structure. In some embodiments, an implant according to the present embodiments is an implant for the reconstruction of body tissue and/or the restoration of the function of a tissue or organ.

Examples of implants include, but are not limited to, a breast implant, e.g. for breast reconstruction after mastectomy or for breast augmentation, a salivary gland implant for the reconstruction of salivary gland functions, and a pancreas implant for the restoration of pancreatic island function (i.e. secretion of insulin and/or glucagon). Additional examples are provided hereinbelow. According to some of any of the respective embodiments, the soft tissue implant is a biocompatible, degradable implant, as these terms are defined herein throughout.

In some embodiments, an implant as described herein may be gradually degraded over about a 1-36-month time period. In some embodiments, an implant is degraded within about a 3-36-month time period. In some embodiments, an implant is degraded within about a 1-24-month time period. In some embodiments, an implant is degraded within about a 1-12-month period. In some embodiments, an implant is degraded within about a 12-24-month period. In some embodiments, an implant is degraded within about a 24-36-month period. In some embodiments, an implant is degraded within about 1 month. In some embodiments, an implant is degraded within about 3 months. In some embodiments, an implant is degraded within about 6 months. In some embodiments, an implant is degraded within about 12 months. In some embodiments, an implant is degraded within about 24 months. In some embodiments, an implant is degraded within about 36 months. In some embodiments, an implant is degraded within less-than a 1-month time period. In some embodiments, an implant is degraded within less-than a 3-month time period. In some embodiments, an implant is degraded within less-than a 6-month time period. In some embodiments, an implant is degraded within less-than a 12-month time period. In some embodiments, an implant is degraded within less-than a 24-month time period. In some embodiments, an implant is degraded within less-than a 36-month time period.

In some embodiments, an implant comprising a composite scaffold as described herein degrades over time following implant into a subject. In some embodiments, an implant comprising a composite scaffold is bioresorbable. In some embodiments, the implants described herein are designed to degrade or break up over time, where the scaffold is replaced by newly formed tissue. In some embodiments, implants comprise biodegradable or naturally-dissolving implants. In some embodiments, implants comprising composite scaffolds are manufactured from materials that dissolve or become absorbed in the body. In some embodiments, an implants degrade over a period of time. In some embodiments, degradation comprises gradual degradation over 1, 3, 6, 12, 24, or 36 months.

In some embodiments, a 3D bio-printed degradable implant is degraded by up to 36 months from implanting. In some embodiments, a 3D bio-printed degradable an implant is degraded by up to 24 months from implanting. In some embodiments, a 3D bio-printed degradable an implant is degraded by up to 12 months from implanting. In some embodiments, a 3D bio-printed degradable an implant is degraded by between 24 months to 36 months from implanting. In some embodiments, a 3D bio-printed degradable an implant is degraded by between 12 months to 24 months from implanting. In some embodiments, a 3D bio-printed degradable an implant is degraded by between 6 months to 18 months from implanting. In some embodiments, a 3D bio-printed degradable an implant is degraded by between 3 months to 12 months from implanting. In some embodiments, a 3D bio-printed degradable an implant is degraded by between 1 months to 12 months from implanting. In some embodiment, a 3D bio-printed degradable an implant is degraded within about 3 months from implanting, about 6 months from implanting, about 7 months from implanting, about 8 months from implanting, about 9 months from implanting, about 10 months from implanting, about 11 months from implanting, about 12 months from implanting, about 13 months from implanting, about 14 months from implanting, about 15 months from implanting, about 16 months from implanting, about 17 months from implanting, about 18 months from implanting, about 19 months from implanting, about 20 months from implanting, about 21 months from implanting, about 22 months from implanting, about 23 months from implanting, about 24 months from implanting, about 25 months from implanting, about 26 months from implanting, about 27 months from implanting, about 28 months from implanting, about 29 months from implanting, about 30 months from implanting, about 31 months from implanting, about 32 months from implanting, or about 33 months from implanting, about 34 months from implanting, about 35 months from implanting, or about 36 months from implanting.

In some embodiments, a 3D bio-printed degradable implant as disclosed herein may be implanted in a subject in need. In some embodiments, a 3D bio-printed degradable implant is used for replacing or reconstructing a breast tissue. In some embodiments, a 3D bio-printed degradable implant is used is subjects undergoing mastectomy requiring breast reconstruction. In some embodiments, a 3D bio-printed degradable implant is used in cosmetic, plastic, and reconstructive surgical procedures resulting from surgery, disease or trauma. In some embodiments, the 3D bio-printed degradable implant gradually degrades over time. In some of these embodiments, the 3D bio-printed degradable implant is replaced by newly formed tissue.

According to some of any of the embodiments described herein, the soft tissue implant is a breast implant.

A skilled artisan would appreciate that the term "breast implant" may encompass an implant inserted under or within the breast tissue or under the pectoral muscle for breast augmentation, reconstruction or replacement.

According to some of any of the embodiments described herein, the soft tissue is a facial (e.g., nose, ear, chin, cheek, eye, lip, etc.) tissue, a neck tissue, a muscle tissue, a joint tissue, a jaw tissue, a buttock tissue, a hand tissue, a chest tissue, a brain tissue, a hepatic tissue, a cartilage, a connective tissue, a cardiac tissue, a pulmonary tissue, a gonadal tissue, and the soft tissue implant is for being implanted, as described herein, in one or more of these tissues and/or in one or more oral cavities comprising one or more of these tissues, and/or in or in close proximity to one or more bodily organs that comprise one or more of these tissues.

According to some of any of the embodiments described herein, the soft tissue implant is an implant of the salivary gland, a pancreas implant, a bone implant, an implant to reconstruct an anterior cruciate ligament tear, a craniofacial reconstruction implant, a maxillofacial reconstruction implant, a complex jaw surgery implant, a post tumor-resection reconstruction implant, an implant for tissue reconstruction after removal of a melanoma, an implant for tissue reconstruction after removal of a head and neck cancer, an ear implant, a nose implant, a chest wall reconstruction implant, an orthopedic surgery implant, a cartilage reconstruction implant and a delayed burn reconstruction implant. According to some of any of the embodiments described herein, the soft tissue implant is for implantation under or within a soft tissue as described herein, for augmentation, reconstruction, replacement and/or regeneration of a soft tissue or a bodily organ comprising same.

The soft tissue implant as described herein further comprises a matrix, as described herein in any of the respective embodiments. The matrix fills at least a portion of the inner cavity of the composite structure.

The matrix comprises rhCollagen, as described herein in any of the respective embodiments, optionally one or more ECM component(s), as described herein in any of the respective embodiments, in addition to the rhCollagen, optionally an integrin-binding material, as described herein in any of the respective embodiments and any combination thereof, and further optionally a biological material such as, for example, cells, cellular components and/or an adipose tissue or a fat extract, as described in further detail hereinbelow.

According to some of any of the embodiments described herein, the soft tissue implant is prepared by additive manufacturing a composite scaffold, e.g., bioprinting, as described herein in any of the respective embodiments and any combination thereof.

A soft tissue implant as described herein can be prepared by forming a composite scaffold, as described herein, by dispensing at least one curable formulation as described herein in any of the respective embodiments to sequentially form a plurality of layers in the configured pattern of the scaffold, as described herein in any of the respective embodiments and any combination thereof.

For at least a portion of these layers, the dispensing is of a formulation that comprises a recombinant human collagen featuring at least one curable group, a synthetic polymer featuring at least one curable group, and optionally an ECM component featuring a curable group and/or an integrin-binding material featuring at least one curable material, as described herein in any of the respective embodiments and any combination thereof.

Preparing the implant further comprises injecting to at least within the inner cavity of the scaffold via the injection port of the scaffold, a matrix as described herein in any of the respective embodiments and any combination thereof.

In some embodiments, a method of preparing a 3D bio-printed degradable implant comprises, subsequent to preparing the bio-printed composite scaffold, a step of maintaining the composite scaffold or the composite scaffold which comprises tissue matric as described herein in a sterile culture medium. In some embodiments, maintaining the scaffold in sterile medium, such as sterile Phosphate-buffered saline (PBS) or Dulbecco's Modified Eagle Medium (DMEM) medium, may encompass maintaining the bio-printed 3D scaffold in an environment where more than 99% of living microorganisms are removed. A skilled artisan would appreciate that the term "sterile medium" may encompass a medium substantially free of living or microorganisms.

In some embodiments, the methods of preparing an implant comprises a sterilization step. In some embodiments, the sterilization comprises use of Ethylene Oxide (EtO) sterilization. In this case, the bio-printed scaffold is dried and exposed to EtO per standard procedures.

In some embodiments, a method of producing an implant comprises bioprinting the composite scaffold, and loading into the inner cavity of the scaffold a matrix as described herein, wherein the bio-printing and/or the loading are performed under sterile conditions.

An exemplary method of preparing an implant as described herein in any of the respective embodiments and any combination thereof comprises:

Bioprinting the composite scaffold according to the embodiments described herein, preferably under sterile conditions;
Optionally drying the scaffold (e.g., removing liquids by e.g., lyophilization);
Optionally sterilizing the scaffold as described herein; and Loading a matrix as described herein to the inner cavity of the scaffold preferably via one or more injection ports, as described herein in any of the respective embodiments and any combination thereof, and preferably under sterile conditions.

*Matrix:*

In some embodiments, a scaffold comprises a matrix at least within the inner cavity, where the matrix comprises rhCollagen optionally in combination with one or more additional ECM components and cells or adipose tissue, or a combination thereof. In some embodiments, a scaffold comprises a matrix as described in detail herein. In some embodiments, an ECM component comprises HA, fibronectin, heparin, elastin, or laminin, or any combination thereof. In some embodiments, a matrix comprises cells. In some embodiments, cells comprise pericytes, adipose derived stem cells, pre-adipocytes, endothelial cells, progenitor cells, hematopoietic cells, or adipocytes, or any combination thereof. In some embodiments, cells comprise a stromal vascular fraction (SVF) isolated from fat tissue. In some embodiments, a matrix comprises adipose tissue, e.g., a minimally processed adipose tissue. In some embodiments, a matrix comprises a fat extract, e.g., a minimally processed fat extract. In some embodiments, the volume of the matrix is from about 5 ml to about 300 ml.

The matrix comprises rhCollagen, as described herein in any of the respective embodiments and any combination thereof. The rhCollagen does not include curable groups or moieties as described herein attached thereto.

In some of any of the embodiments described herein, the rhCollagen is a cross-linked fibrillar collagen as described herein, a rhCollagen-derived particles as described herein (e.g., in a form of rhCollagen and/or rhGelatin nanoparticles), and any mixture thereof.

In some of these embodiments, the matrix comprises cross-linked fibrillar collagen as described herein and a particulate rhCollagen and/or rhGelatin (e.g., in a form of nanoparticles). In some of these embodiments, a weight ratio between the cross-linked fibrillar collagen as described herein and the particulate rhCollagen and/or rhGelatin (e.g., in a form of nanoparticles is from about 5:1 to about 5:1, including any intermediate values and subranges therebetween. In some of these embodiments, the ratio ranges from 3:1 to 1:3, or from 2:1 to 1:2, or from 2:1 to 1:1, or from 3:2 to 2:1, or from 3:2 to 1:1, including any intermediate values and subranges therebetween.

In some of any of the embodiments described herein, the rhCollagen is or comprises a cross-linked fibrillar collagen as described herein.

According to some of any of the embodiments described herein, the matrix further comprises one or more ECM components as described herein in any of the respective embodiments and any combination thereof, including, for example, hyaluronic acid, fibronectin, heparin, elastin, or laminin, or any combination thereof.

According to some of these embodiments, the ECM component does not have one or more curable groups or moieties attached thereto, as described herein in any of the respective embodiments. According to some of these embodiments, the ECM components is a cross-linkable ECM component and the matrix may optionally further comprise a cross-linking agent, as described herein in any of the respective embodiments. In some embodiments, the ECM component is a curable ECM component, as described herein in any of the respective embodiments and any combination thereof.

According to some of these embodiments, the ECM component is cross-linked, e.g., by means a cross-linking agent and/or via inherent functional groups in its chemical structure.

According to some of any of the embodiments described herein, the matrix further comprises one or more integrin-binding materials as described herein in any of the respective embodiments and any combination thereof, including, for example, an RGD-containing material, for example, RGD4C.

According to some of these embodiments, the RGD-containing material does not have one or more curable groups or moieties attached thereto, as described herein in any of the respective embodiments. According to some of these embodiments, the RGD-containing material is cross-linkable, for example, it contains one or more cysteine residues which can form disulfide bonds. In some embodiments, the matrix may optionally further comprise a cross-linking agent, as described herein in any of the respective embodiments. In some embodiments, the RGD-containing material is a curable RGD-containing material, as described herein in any of the respective embodiments and any combination thereof.

In some embodiments, an ECM component comprises hyaluronic acid (HA), fibronectin, heparin, elastin, or laminin, or any combination thereof. Collagen comprises a recombinant human collagen (rhCollagen). In some embodiments, collagen comprises a plant-derived rhCollagen. In some embodiments, rhCollagen comprises a cross-linkable, plant-derived human collagen. In some embodiments, rhCollagen comprises a plant-derived human collagen, as described in detail herein.

In some embodiments, an ECM component comprises a fragmented ECM component. In some embodiments, an ECM component comprises hyaluronic acid (HA). In some embodiments, HA comprises modified HA. Addition of methacrylate groups to hyaluronic acid (HA) results in hyaluronic acid-methacrylate (HAMA or MA-HA) which is photocurable. In some embodiments, HA comprises modified HA or a photopolymerizable modified derivative thereof. In some embodiments, HA comprises methacrylated HA. In some embodiments, HA comprises thiolated HA. In some embodiments, HA comprises crosslinked HA.

In some embodiments, an ECM component comprises modified fibronectin. In some embodiments, an ECM component comprises modified heparin. In some embodiments, an ECM component comprises modified elastin. In some embodiments, an ECM component comprises modified laminin.

In some embodiments, a matrix further comprises a crosslinking agent. In some embodiments, a crosslinking agent comprises a photocurable crosslinker. In some embodiments, a crosslinking agent comprises SR9035 (Ethoxylated (15) trimethylolpropane triacrylate). In some embodiments, a crosslinker agent comprises 4-arm PEG-thiol, 8-arm PEG-thiol, 4-arm PEG-acrylate, or 8-arm PEG-acrylate.

In some embodiments, the cross-linking agent is not necessarily photocurable and is capable of cross-linking an ECM component or any other component on the matrix when subjected to suitable reaction conditions.

In some embodiments, an ECM component comprises fibronectin or a functional fragment thereof. In some embodiments, an ECM component comprises heparin or a functional fragment thereof. In some embodiments, an ECM component comprises elastin or a functional fragment thereof. In some embodiments, an ECM component comprises laminin or a functional fragment thereof. A skilled artisan would appreciate that laminin surrounds adipocytes and is important in adipogenesis of preadipocytes and lipogenesis of adipocytes.

In some embodiments, a matrix comprises two different ECM components, three different ECM components, four different ECM components, or five different ECM components. In some embodiments, a matrix comprises at least two different ECM components, at least three different ECM components, at least four different ECM components, or at least five different ECM components.

In some embodiments, an ECM component is decellularized (as described, e.g., in US Pub. 2019/0022017). In some embodiments, ECM components are prepared from desirable cells and native decellularized ECM. Native tissue is washed in PBS to remove all residual blood. The tissue is sliced and incubated in hyper-hypotonic solutions of NaCl. Slices are then incubated with trypsin-EDTA 0.05 % for 24 hours (repeated twice). For chemical removal, tissue is then washed several times with triton X-100+ammonium hydroxide solutions and finally several washing cycles of 48 hours with phosphate buffered saline (PBS) until no residue of foam is obtained. ECM is sterilized by washing for two hours with ethanol (70%) and following two washes with double distilled water. Lyophilized ECM and protease enzyme (e.g. pepsin or/and collagenase or/and trypsin) at different concentration and biological activities are mixed in 15 ml of 0.05 M to 0.2M HCl and kept at a constant stirring for 48 h at room temperature (25 °C). The resultant viscous solution of digested ECM solution has a pH of approximately 3.0-4.0. The activity of the enzyme is irreversibly inactivated according to the type of enzyme, for example, for pepsin the pH will be raised to 7.4. The solubilized matrix still retains ECM proteins and peptide fragments therefore, the matrix retains its biochemical components, as is necessary for cell-matrix interactions. ECM are considered completely solubilized, when no particles are detected in solution.

Adipose tissue ECM contains large and complex proteins that exhibit distinct domains, and whose sequences and arrangements are highly conserved. Collagen is a key structural protein in the ECM and is the most abundant protein in the ECM. Adipose tissue ECM regulates and promotes cell differentiation, proliferation, survival and importantly, physiological functions. In some embodiments, ECM components comprise adipose tissue ECM. In some embodiments, ECM components comprise allogeneic adipose tissue ECM. In some embodiments, ECM components comprise autologous adipose tissue ECM. In some embodiments, adipose tissue ECM comprises collagen I-VII, laminin, fibronectin, elastin and glycosaminoglycan (GAG).

According to some of any of the embodiments described herein, the matrix comprises rhCollagen, and optionally another ECM component and/or an integrin-binding material, as described herein, optionally one or more cross-linking agents, and may further comprise cellular components, cells and/or an adipose tissue.

According to some of any of the embodiments described herein, the matrix comprises rhCollagen, and optionally another ECM component and/or an integrin-binding material, as described herein, and at one of these components is cross-linked. In some embodiments, at least two of these components are cross-linked to one another. In some embodiments, the ECM component is cross-linked by means of a cross-linking agent.

Such matrices are injected into a composite scaffold as described herein.

The matrix comprises rhCollagen, alone or in combination with an additional ECM component, and cells and/or adipose tissue.

The matrix as described herein is an injectable matrix.

In some embodiments, a matrix comprises rhCollagen which does not include curable groups component as described herein in any of the respective embodiments and cells. In some embodiments, cells comprise pericytes, adipose derived stem cells, pre-adipocytes, endothelial cells, progenitor cells, hematopoietic cells, or adipocytes, or any combination thereof.

In some embodiments, a matrix comprises pericytes. A skilled artisan would appreciate that the term "pericyte" may encompass spatially isolated contractile cells that wrap around the endothelial cells that line the body's blood vessels, such as capillaries.

In some embodiments, a matrix comprises adipose derived stem cells. In some embodiments, a matrix comprises adipocytes. In some embodiments, a matrix comprises pre-adipocytes. A skilled artisan would appreciate that the term "adipocytes," also known as lipocytes or fat cells, may encompass cells containing lipid droplets.

In some embodiments, a matrix comprises endothelial cells. A skilled artisan would further appreciate that the term "endothelial cells" may encompass cells of the endothelium, which line the surfaces of body cavities, such as, blood or lymph vessels or capillaries.

In some embodiments, a matrix comprises hematopoietic cells. A skilled artisan would appreciate that the term "hematopoietic cells" may encompass hematopoietic stem cells and hematopoietic progenitor cells which are able to differentiate into blood cells, erythrocytes.

In some embodiments, a matrix comprises progenitor cells. A skilled artisan would appreciate that the term "progenitor cells" may encompass unipotent cells that are committed to differentiate into a specific type of cell, and which have limited or no ability to self-renew.

In some embodiments, cells comprise autologous cells. In some embodiments, cells comprise allogeneic cells. Thus, in some embodiments, cells can be obtained from a subject, and the matrix is administered to the same subject. In some other embodiments, cells may be obtained from a compatible donor (for example, a subject with a matching blood type).

Cells and cellular components, autologous or allogeneic, can be obtained using methods well known and recognized in the art for extracting and preparing the cells herein disclosed, for example, using the method schematically illustrated in FIG. 9.

In some further embodiments, a matrix comprises an adipose tissue. A skilled artisan would appreciate that the term "adipose tissue" may encompass connective tissue composed of multiple cell types including adipocytes and microvascular cells. In some embodiments, adipose tissue comprises, among others, stem cells and endothelial precursor cells. A skilled artisan would appreciate that the term "stem cell" may encompass a multipotent cell with the potential to differentiate into a variety of cell types and has the ability to self-renew.

In some embodiments, adipose tissue comprises a fat extract. A skilled artisan would appreciate that the term "fat extract" refers to fat, including connective tissue that stores fat. In some embodiments, fat extract, fat cells, and/or tissue may be obtained, for example, by liposuction, lipoaspiration, and/or direct excision. In some embodiments, fat extract or adipose tissue is extracted from various areas of the body, including, for example, the abdomen, thighs, buttocks, arms and neck.

In some embodiments, adipose tissue, or fat extract is homogenized. In some embodiments, the fat extract comprises homogenized fat extract. In some embodiments, the fat extract comprises minimally processed fat extract. In some embodiments, the fat extract comprises minimally processed homogenized fat extract, as described herein and demonstrated in the Examples section that follows. In some embodiments, adipose tissue or fat extract is mechanically chopped, e.g., broken into pieces. In some embodiments, adipose tissue or fat extract is broken into pieces of between about $0.5mm^2$ to $5mm^2$. In some embodiments, adipose tissue or fat extract is chopped or broken into pieces to allow injection thereof into an implant scaffold or soft tissue.

A skilled artisan would appreciate that the term "homogenized" may encompass making substantially similar in size and composition.

In some embodiments, the adipose tissue as described herein in any of the respective embodiments comprises autologous adipose tissue. In some embodiments, the adipose tissue as described herein in any of the respective embodiments comprises allogeneic adipose tissue. In some embodiments, adipose tissue can be obtained from a subject, and administered to the same subject. In some other embodiments, adipose tissue may be obtained from a compatible donor (for example, a subject with matching blood type). A skilled artisan would be knowledgeable of the available methods for extracting and preparing the adipose tissue, such as the method herein disclosed.

In some embodiments, cells comprise a stromal vascular fraction (SVF) isolated from a fat tissue (e.g., an autologous fat tissue). In some embodiments, a matrix comprises an SVF isolated from a fat tissue (e.g., an autologous fat tissue). In some embodiments, an SVF comprises pericytes, adipose derived stem cells, pre-adipocytes, endothelial and progenitor cells, and haemopoietic cells including monocytes and macrophages. In some embodiments, an SVF is implanted as part of the matrix. An SVF placed within a 3D collagen scaffold has the capacity to reorganize into 3D organoids *in-vitro* and develop capillary networks, suggesting that SVF cells promote vascular network formation and angiogenesis. An artisan would appreciate that there are various methods to extract the SVF. In one embodiment, a method of SVF cellular extraction is described in Example 1 and is schematically illustrated in FIG. 9. In some embodiments, SVF can be obtained from autologous or heterologous subjects.

In some embodiments, an adipose tissue comprises a fat extract isolated from a fat tissue (e.g., an autologous fat tissue) which is minimally processed (e.g., is not subjected to enzymatic processes). In some of these embodiments, the fat extract is a homogenized fat extract.

In some embodiments, a matrix comprises rhCollagen and at least one further extracellular matrix (ECM) component, and cells or adipose tissue, or a combination of cells and adipose tissue, as described herein in any of the respective embodiments.

In some embodiments, the ratio of ECM components to cells or adipose tissue in the matrix is about 5:95, 10:90, 15:85, 20:80, 25:75, 30:70, 40:60, 45:55, 50:50, 55:45, 60:40, 65:35, 70:30, 75:25, 80:20, 85:15, 90:10, or 95:5. In some embodiments, the ratio of ECM components to cells or adipose tissue is 5:95, 10:90, 15:85, 20:80, 25:75, 30:70, 40:60, 45:55, 50:50, 55:45, 60:40, 65:35, 70:30, 75:25, 80:20, 85:15, 90:10, or 95:5.

In some embodiments, a matrix comprises at least one extracellular matrix (ECM) component, and cells or adipose tissue, or a combination thereof, wherein the ECM component comprises rhCollagen, optionally in combination with HA, fibronectin, heparin, elastin and/or laminin, or any combination thereof, the cells comprise pericytes, adipose derived stem cells, pre-adipocytes, endothelial cells, progenitor cells, hematopoietic cells, or adipocytes, or any combination thereof, or the cells comprise a stromal vascular fraction (SVF) isolated from fat tissue.

A skilled artisan would appreciate that the terms "injecting" or "injectable" in some embodiments comprises any *in-vivo* insertion or introduction of a matrix into a scaffold, as described herein, or in other embodiments, comprises *in vitro* insertion or introduction of a matrix into a scaffold. In some embodiments, a matrix is injected into a scaffold prior to implanting in a subject. In some embodiments, a matrix is injected into a scaffold post implanting in a subject. In some embodiments, use of a matrix described herein is for tissue regeneration. In some embodiments, use of a matrix described herein is for fat tissue regeneration.

### Methods of Use of a Matrix:

A matrix, as described herein, may be used in methods of preparing and/or using a soft tissue implant as described herein in any of the respective embodiments and any combination thereof. In some embodiments, a method of preparing a degradable soft tissue implant comprises a step of injecting a matrix into at least the inner cavity of the scaffold as described herein in detail.

The matrix is injected into an inner cavity of a soft tissue implant scaffold, as described herein.

According to the invention, a matrix is injected into an implant, for example, into an inner cavity of a composite scaffold as described in detail herein. In some embodiments, a matrix is injected into a scaffold prior to implanting in a subject. In some embodiments, a matrix is injected into a scaffold post implanting in a subject. The matrix is injected into a scaffold through an injection port. The injection port of a scaffold has a diameter which allows injection of a matrix as described herein. The injection port has a diameter which allows injection of ECM components, cells, or tissues, as described herein.

The matrix features a viscosity that renders it injectable, for example, injectable through a medical device as described herein in any of the respective embodiments.

In some embodiments, a matrix as described herein has a viscosity in a range of from 5 to 20, or from 10 to 20 or from 15 to 20 Pa/second.

### Methods of Use of Degradable soft tissue Implants

According to some of any of the embodiments of the present invention there are provided methods of implanting a 3D bio-printed biocompatible, degradable breast implant comprising a composite scaffold as described herein in any of the respective embodiments and any combination thereof into a subject in need, comprising the steps of (a) implanting the scaffold in the subject, (b) injecting a matrix into the scaffold, using at least one injection port of the scaffold, and (c) optionally repeating step (b), where the implanted scaffold gradually degrades over time, and the degrading scaffold is replaced by newly formed tissue.

According to some of any of the embodiments of the present invention there are provided methods of replacing or reconstructing or generating or augmenting or repairing or healing a soft tissue (e.g., breast tissue) in a subject in need, effected by (a) implanting an implant as described herein in any of the respective embodiments in a site to be treated in a subject in need thereof, (b) injecting a matrix into the scaffold, using at least one injection port of the scaffold, and (c) optionally repeating step (b), where the implanted scaffold gradually degrades over time, and the degrading scaffold is replaced by newly formed tissue. According to an aspect of some embodiments of the present invention there is provided a bio-printed soft tissue implant as described herein in any of the respective embodiments and any combination thereof, for use in augmenting and/or reconstructing and/or regenerating and/or repairing and/or healing a soft tissue in a subject in need thereof, which comprises:

implanting the scaffold in a bodily organ or cavity where augmenting and/or reconstructing and/or regenerating and/or repairing and/or healing the soft tissue is desirable; and

injecting a matrix as described herein in any of the respective embodiments to at least within the inner cavity of the scaffold, by means of an injection port of the scaffold.

Injecting the matrix can be performed prior to or subsequent to the implanting.

According to an aspect of some embodiments of the present invention there is provided a method of augmenting and/or reconstructing and/or regenerating and/or repairing and/or healing a soft tissue in a subject in need thereof, which comprises:

implanting the scaffold in a bodily organ or cavity where augmenting and/or reconstructing and/or regenerating and/or repairing and/or healing the soft tissue is desirable; and

injecting a matrix as described herein in any of the respective embodiments to at least within the inner cavity of the scaffold, preferably by means of an injection port of the scaffold.

Injecting the matrix can be performed prior to or subsequent to the implanting.

According to an aspect of some embodiments of the present invention there is provided a use of a soft tissue implant as described herein in any of the respective embodiments and any combination thereof, as a medical device for augmenting and/or reconstructing and/or regenerating and/or repairing and/or healing a soft tissue in a subject in need thereof, by implanting the scaffold in a bodily organ or cavity where augmenting and/or reconstructing and/or regenerating and/or repairing and/or healing the soft tissue is desirable; and injecting a matrix as described herein in any of the respective embodiments to at least within the inner cavity of the scaffold, preferably by mans of an injection port of the scaffold.

Injecting the matrix can be performed prior to or subsequent to the implanting.

According to some embodiments, injecting the matrix is performed subsequent to the implanting, and is optionally performed repetitively, as described herein.

According to some embodiments, when a matrix is injected to the scaffold, a volume of the matrix is from about 5 ml to about 300 ml, as described herein in any of the respective embodiments.

According to some of any of the embodiments described herein, following the implanting, the printed vascular network path is anastomosed with at least one of the subject's blood vessels, as described herein.

The details of soft tissue implants comprising composite scaffolds are as provided above. Those sections are incorporated herein in full. Any embodiment of a soft tissue (e.g., breast) implant comprising a composite scaffold as described herein may be used in the methods for implanting a soft tissue (e.g., breast) implant, and in the methods of use for replacing or reconstructing a soft tissue.

In some embodiments, a composite scaffold as described herein is bio-printed in a size and shape that corresponds to the treated subject's anatomy. These parameters can be obtained, for example, from imaging data, such that the method can further comprise acquiring an imaging data for a size and shape of the scaffold, and bioprinting the scaffold in accordance with the imaging data.

In some embodiments of a method of use of a soft tissue implant, a matrix is loaded into the scaffold prior to a step of implanting a soft tissue implant comprising a composite scaffold in a subject, where injection is through at least one injection port. In some embodiments of a method of use of a soft tissue implant, a matrix is loaded into the scaffold prior to a step of implanting a soft tissue implant comprising a composite scaffold in a subject. In some embodiments of a method of use of a soft tissue implant, a scaffold is implanted in a subject without loading a matrix onto the scaffold. In some embodiments of a method of use of a soft tissue implant, a scaffold is implanted in a subject without a matrix for allowing vascularization of the implant. In some embodiments of a method of use of a soft tissue implant, a scaffold is implanted in a subject without loading a matrix onto the scaffold and a matrix is loaded into the scaffold about 1 week to 24 weeks post implanting of the soft tissue implant. In some embodiments of a method of use of a soft tissue implant, a matrix is injected into the scaffold, using at least one injection port of the scaffold, after the step of implanting the scaffold in a subject. The ECM components, cells, and or adipose tissue comprised in a matrix provide a basis for new tissue formation. In some embodiments, concurrent with the degradation or bio resorption or combination thereof of the composite scaffold, cells present within the matrix may proliferate and spread within the porous regions of the scaffold forming new tissue. In some embodiments, concurrent with the degradation or bio resorption or combination thereof of the composite scaffold, cells within tissue present within the matrix may proliferate and spread within the porous regions of the scaffold forming new tissue. In some embodiments, concurrent with the degradation or bio resorption or combination thereof of the composite scaffold, the ECM components within the matrix may provide 3D structural support for the formation of new tissue, for example but not limited to for cell adhesion.

In some embodiments of a method of use of a soft tissue implant, following implanting, the composite scaffold degrades over time, and may be replaced by newly formed tissue. In some embodiments of a method of use of a soft tissue implant, following implanting the composite scaffold degrades over time, and may be replaced by components of the matrix and newly formed tissue. In some embodiments, a soft tissue implant degrades over a period of time. In some embodiments, following a method of use of a soft tissue implant, the implant gradually degrades over 1, 3, 6, 12, 24, or 36 months, as described above for soft tissue implants.

In some embodiments of a method of use of a soft tissue implant, a composite scaffold is degraded by up to 36 months from implanting. In some embodiments of a method of use of a soft tissue implant, a composite scaffold is degraded by up to 24 months from implanting. In some embodiments of a method of use of a soft tissue implant, a composite scaffold is degraded by up to 12 months from implanting. In some embodiments of a method of use of a soft tissue implant, a composite scaffold is degraded by between 12 months to 36 months from implanting. In some embodiments of a method of use of a soft tissue implant, a composite scaffold is degraded by between 12 months to 24 months from implanting. In some embodiments of a method of use of a soft tissue implant, a composite scaffold is degraded by between 6 months to 18 months from implanting. In some embodiments of a method of use of a soft tissue implant, a composite scaffold is degraded by between 1 months to 36 months from implanting. In some embodiments of a method of use of a soft tissue implant, a composite scaffold is degraded by between 3 months to 24 months from implanting.

In some embodiments of a method of use of a soft tissue implant, a 3D bio-printed biocompatible degradable soft tissue implant composite scaffold is degraded within about 6 months from implanting, about 7 months from implanting, about 8 months from implanting, about 9 months from implanting, about 10 months from implanting, about 11 months from implanting, about 12 months from implanting, about 13 months from implanting, about 14 months from implanting, about 15 months from implanting, about 16 months from implanting, about 17 months from implanting, about 18 months from implanting, about 19 months from implanting, about 20 months from implanting, about 21 months from implanting, about 22 months from implanting, about 23 months from implanting, about 24 months from implanting, about 25 months from implanting, about 26 months from implanting, about 27 months from implanting, about 28 months from implanting, about 29 months from implanting, about 30 months from implanting, about 31 months from implanting, about 32 months from implanting, or about 33 months from implanting, about 34 months from implanting, about 35 months from implanting, or about 36 months from implanting in a subject.

In some embodiments of a method of use of a soft tissue implant, the step of injecting a matrix into the scaffold is repeated. In some embodiments of a method of use of a soft tissue implant, a matrix is repeatedly injected into a scaffold post implanting in a subject. In some embodiments of a method of use of a soft tissue implant, a matrix is injected into a scaffold at least once. In some embodiments of a method of use of a soft tissue implant, a matrix is injected into a scaffold at once, twice, three, four, five, six, seven, eight, nine, or ten times.

In some embodiments of a method of use of a soft tissue implant, the step of injecting a matrix into the scaffold is performed between 1 week to 24 weeks post implanting in a subject.

In some embodiments of a method of use of a soft tissue implant, the step of injecting a matrix into the scaffold is performed by up to 24 weeks from implanting. In some embodiments of a method of use of a soft tissue implant, the step of injecting a matrix into the scaffold is performed by up to 12 weeks from implanting. In some embodiments of a method of use of a soft tissue implant, the step of injecting a matrix into the scaffold is performed by up to 12 weeks from implanting. In some embodiments of a method of use of a soft tissue implant, the step of injecting a matrix into the scaffold is performed by between 1 week to 24 weeks from implanting. In some embodiments of a method of use of a soft tissue implant, the step of injecting a matrix into the scaffold is performed by between 12 weeks to 24 weeks from implanting. In some embodiments of a method of use of a soft tissue implant, the step of injecting a matrix into the scaffold is performed by between 6 weeks to 18 weeks from implanting. In some embodiments of a method of use of a soft tissue implant, the step of injecting a matrix into the scaffold is performed by between 1 week to 12 weeks from implanting. In some embodiments of a method of use of a soft tissue implant, the step of injecting a matrix into the scaffold is performed by between 3 weeks to 24 weeks from implanting.

In some embodiments of a method of use of a soft tissue implant, the step of injecting a matrix into the scaffold is performed within about 1 week from implanting, about 2 weeks from implanting, about 3 weeks from implanting, about 4 weeks from implanting, about 5 weeks from implanting, about 6 weeks from implanting, about 7 weeks from implanting, about 8 weeks from implanting, about 9 weeks from implanting, about 10 weeks from implanting, about 11 weeks from implanting, about 12 weeks from implanting, about 13 weeks from implanting, about 14 weeks from implanting, about 15 weeks from implanting, about 16 weeks from implanting, about 17 weeks from implanting, about 18 weeks from implanting, about 19 weeks from implanting, about 20 weeks from implanting, about 21 weeks from implanting, about 22 weeks from implanting, about 23 weeks from implanting, or about 24 weeks from implanting in a subject.

In some embodiments of a method of implanting a soft tissue implant, a matrix can be administered via localized injection, including catheter administration, systemic injection, localized injection, intravenous injection, or parenteral administration. In some embodiments of a method of use of a soft tissue implant, devices suitable for injection of a matrix comprise needles, cannulas, pointed plastic tip applicators, reservoirs, stents, plungers, release systems and syringes.

In some embodiments, the method of implanting a 3D bio-printed degradable soft tissue implant comprises a scaffold pre-loading step prior to the implanting step (a), where the scaffold is pre-loaded with a matrix comprising at least one ECM component, and cells or adipose tissue, or a combination thereof.

In some embodiments of a method of implanting a soft tissue implant, a scaffold is pre-loaded with a matrix, at least within the inner cavity of the scaffold. In some embodiments of a method of implanting a soft tissue implant, a matrix comprises rhCollagen which does not include curable groups optionally in combination with at least one futher extracellular matrix (ECM) component and cells or adipose tissue, or a combination thereof. In some embodiments of a method of implanting a soft tissue implant, a scaffold comprises a matrix as described in detail herein. In some embodiments of a method of implanting a soft tissue implant, an ECM component comprises rhCollagen, and optionally HA, fibronectin, heparin, elastin, or laminin, or any combination thereof. In some embodiments, HA comprises modified HA or a photopolymerizable modified derivative thereof. In some embodiments, HA comprises methacrylated or thiolated HA, as described herein in detail.

In some embodiments of a method of use of a soft tissue implant, a matrix comprises cells. In some embodiments of a method of implanting a soft tissue implant, cells comprise pericytes, adipose derived stem cells, pre-adipocytes, endothelial cells, progenitor cells, hematopoietic cells, or adipocytes, or any combination thereof. In some embodiments of a method of implanting a soft tissue implant, cells comprise a stromal vascular fraction (SVF) isolated from fat tissue. In some embodiments of a method of implanting a soft tissue implant, a matrix comprises adipose tissue. In some embodiments of a method of implanting a soft tissue implant, adipose tissue comprises homogenized fat extract.

In some embodiments of a method of use of a soft tissue implant comprising a composite scaffold, the volume of the pre-loaded matrix is as described herein in any of the respective embodiments.

Details of the composite scaffold and matrix have been provided above. Those details and embodiments thereof, are incorporated herein in full, wherein methods of use of a soft tissue implant include the embodiments of soft tissue implants and matrices described herein.

In some embodiments of a method of implanting a soft tissue implant described herein, the method comprises a step of anastomosis of at least one of the printed vascular network paths with at least one of the subject's blood vessels. A skilled artisan would appreciate that the term "anastomosis" may encompass the surgical joining of the subject's blood vessels with the soft tissue implant or scaffold described herein. In some embodiments, the subject's blood vessels are joined to the scaffold via at least one of the printed vascular network paths. In some embodiments, the subject's blood vessels are joined through the printed vascular network paths such that cells and tissue within the inner cavity of the scaffold receive a constant supply of oxygen and nutrients. In some embodiments, vascular network paths are designed to allow anastomosis to the subject's blood vessels through a surgical procedure.

In some embodiments of a method of implanting a soft tissue implant described herein, the method comprises a step of anastomosis of the printed vascular network path with at least one of the subject's blood vessels.

In some embodiments of a method of implanting a soft tissue implant described herein, the soft tissue implant comprises a composite scaffold comprising rhCollagen and a biocompatible synthetic polymer into a subject in need, comprising the steps of (a) implanting the scaffold in the subject, where the scaffold comprises a porous lattice, an inner cavity within the scaffold and at least one injection port that connects the inner cavity with the outer most surface of the scaffold, where the injection port is sized to permit insertion of a cannula for cell or tissue injection, (b) injecting a matrix into the scaffold, using at least one injection port of the scaffold, the matrix comprising rhCollagen which does not include curable groups and optionally at least one further extracellular matrix (ECM) component and cells or adipose tissue, or a combination thereof, and (c) optionally repeating step (b), where the implanted scaffold gradually degrades over time, and the degrading scaffold is replaced by newly formed tissue.

In some embodiments of a method of implanting a soft tissue implant described herein, the soft tissue implant comprises a scaffold pre-loading step prior to the implanting step (a), where the scaffold is pre-loaded with a matrix comprising at least one ECM component and cells or adipose tissue, or a combination thereof, where the ECM component comprises rhCollagen, and optionally HA. fibronectin, heparin, elastin and laminin, or any combination thereof, and the cells comprise pericytes, adipose derived stem cells, pre-adipocytes, endothelial cells, progenitor cells, hematopoietic cells, or adipocytes, or any combination thereof, or the cells comprise a stromal vascular fraction (SVF) isolated from fat tissue. In some embodiments of a method of implanting a soft tissue implant, adipose tissue comprises homogenized fat extract.

In some embodiments of a method of implanting a soft tissue implant described herein, implanting promotes vascularization of the implant. In some embodiments of a method of implanting a soft tissue implant described herein, the scaffold comprises implanted cells and tissue. In some embodiments of a method of implanting a soft tissue implant described herein, the scaffolds are biodegradable, such that the implanted scaffold gradually degrades over time and is eventually replaced by newly formed tissue. In some embodiments of a method of implanting a soft tissue implant described herein, the scaffold comprises rhCollagen and optionally further ECM components which promote cell proliferation, cell differentiation and tissue growth. The ability to achieve appropriate vascularization is critical for any successful transplantation procedure. Any delay in supply of oxygen and nutrients and the removal of waste may cause damage to the implanted cells and tissues.

*Applications:*

Any of the curable formulations, composite scaffolds, matrices and implants as described herein are for use in any of the methods as described herein, for augmenting, reconstructing, repairing and/or regenerating a soft tissue in a subject in need thereof.

When a matrix as described herein is used per se (without an implant) according to the present embodiments, it is also regarded as a soft tissue filler, for example, for use in applications that require filling and/or augmenting and/or repairing a soft tissue as described herein

"Tissue reconstruction" encompasses to the repair or replacement of portions of tissues or whole tissues within the body or to the repair or replacement of portions of organs or whole organs. An implant or matrix for tissue reconstruction is designed to aid the process of tissue reconstruction. It can for example take over the role of the supporting connective tissue within an organ or body part. In case of an implant comprising a three-dimensional scaffold made of a biodegradable material, the implant may temporarily take over the role of the supporting connective tissue.

In some embodiments, an implant as described herein is for generating prevascularized connective tissue as recipient site for cell/tissue transplantation, preferably for transplantation of free fat grafts.

In some embodiments, the methods and uses as described herein are usable in treating scars and deformities following trauma or surgical procedure.

In some embodiments, the methods and uses as described herein are usable in treating soft tissue depression.

In some embodiments, the methods and uses as described herein are usable in treating congenital deformities.

In some embodiments, the methods and uses as described herein are usable in treating Poland syndrome.

In some embodiments, the methods and uses as described herein are usable in treating Romberg syndrome.

In some embodiments, the methods and uses as described herein are usable in treating pectus excavatum.

In some embodiments, the methods and uses as described herein are usable in treating structural asymmetry, such as breast asymmetry or buttock asymmetry or facial asymmetry.

In some embodiments, the methods and uses as described herein are usable in post-mastectomy and post-lumpectomy breast reconstruction.

In some embodiments, the methods and uses as described herein are usable in treating lipodystrophy.

In some embodiments, the methods and uses as described herein are usable in tissue augmentation (breast, buttock).

In some embodiments, the methods and uses as described herein are usable in treating any condition that requires cavity fill and improved body contouring.

*Kits:*

According to an aspect of some embodiments of the present invention there is provided a kit which comprises:

A curable formulation or curable formulations for forming as composite scaffold as described in any of the respective embodiments and any combination thereof; and

a matrix formulation for forming a matrix to be injected into an inner cavity of the scaffold, as described in any of the respective embodiments and any combination thereof.

In some embodiments, the formulations are packaged individually within the kit. In some embodiments the packages are light-impermeable and/or air-impermeable.

In some embodiments, the kit is identified for use in preparing a soft tissue implant as described herein in any of the respective embodiments and can include written instructions how to use the formulations, in according with the embodiments as described herein.

According to an aspect of some embodiments of the present invention there is provided a kit which comprises:

A composite scaffold as described in any of the respective embodiments and any combination thereof; and

a matrix formulation for forming a matrix to be injected into an inner cavity of the scaffold, as described in any of the respective embodiments and any combination thereof.

In some embodiments, the scaffold and the formulation are packaged individually within the kit. In some embodiments the packages are light-impermeable and/or air-impermeable.

In some embodiments, the kit is identified for use in preparing a soft tissue implant as described herein in any of the respective embodiments and can include written instructions to combine the formulation and the scaffold, in accordance with any of the respective embodiments as described herein.

In some embodiments, the kit is identified for use in preparing a soft tissue implant as described herein in any of the respective embodiments and can include written instructions how to use the formulations, in according with the embodiments as described herein.

In some embodiments, the kit is identified for use as a soft tissue filler, as described herein in any of the respective embodiments.

Herein throughout, according to some embodiments, whenever an ECM component is described, including a curable ECM component, and including collagen, rhCollagen, as described in any of the respective embodiments and any combination thereof, the ECM component encompasses also fragments and degradation products thereof.

As used herein the term "about" refers to ± 10 % or ± 5 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

As used herein, the term "subject" encompasses an animal, preferably a mammal, more preferably a human being. The term "subject" "individual", or "patient" are used herein interchangeably. In one embodiment, in any of the methods and uses as described herein, the subject comprises a human subject. In some embodiments, the subject is male. In some embodiments, the subject is female.

Various embodiments and aspects of the present invention as delineated hereinabove and as claimed in the claims section below find experimental support in the following examples.

## EXAMPLES

Reference is now made to the following examples, which together with the above descriptions illustrate some embodiments of the invention in a non-limiting fashion.

### *EXAMPLE 1*

***Preparation of 3D-Bioprinted, Degradable Breast Implants***

***Implant Design and Preparation:***

As an exemplary breast implant that may be vascularized and comprises a scaffold that gradually degrades over 3-36 months was designed and practiced. Generally, a scaffold was formed by 3D-printing using a curable formulation comprising a curable rhCollagen-based material and was optionally loaded thereafter with a matrix comprising extracellular matrix components and/or autologous fat cells (e.g., fat extract or SVF in an rhCollagen based matrix), as generally depicted in FIGs. 7 and 10.

*Scaffold preparation:*

The 3D breast degradable implant scaffold was bioprinted (FIG. 7; Upper left object) using LabFab desktop printer (3D Systems)], with a curable formulation as described herein, that comprises methacrylated rhCollagen, as described herein in any of the respective embodiments. A non-examplary formulation included methacrylated rhCollagen (0.5 %), poly(ethylene glycol) diacrylate 700 (PEG-DA average Mn=700; 0.5-2 %), Ethoxylated (15) trimethylolpropane triacrylate (marketed as SR9035; 1.0-2.5 %), and N-(2-Hydroxyethyl)acrylamide (HEAA; 12.0-40.0 %) and optionally an ECM component and/or an RGD-containing material as described herein, all in an aqueous carrier (e.g., water).

An additional exemplary formulation included methacrylated rhCollagen (0.1-1 %), a synthetic polymer featuring a curable group as described herein in any of the respective embodiments (5-30 %), an ECM component featuring a curable group as described herein in any of the respective embodiments, for example, (meth)acrylated or thiolated fibronectin and/or (meth)acrylated or thiolated heparin (0.001-0.1 %) , and an integrin-binding material featuring a curable group (e.g., a cysteine-containing RGD material and/or RGD-containing material attached to PEG-DA as described herein); at about a stoichiometric amount relative to the rhCollagen).

An exemplary 3D-printed scaffold was generally shaped as a dome, having dimensions of 10 mm x 10 mm x 6 mm, and comprised pores featuring dimensions of about 0.5 mm x 0.5 mm.

FIGs. 8A-8C show the printed 3D scaffold from a cross-section (FIG. 8A), top view (FIG. 8B), and side view (FIG. 8C), wherein the pore dimensions were 500 microns (micrometers). The scaffold was designed to feature at least one (e.g., two) injection port(s) and a vascular network in its interior space, and printed vascular network paths around the scaffold edge, as depicted, for example, in FIG. 10 and described herein.

Additional designs of exemplary 3D-printed scaffolds generally shaped as a dome are presented in FIGs. 14A-B and 15, as described herein.

*Loading materials/matrix:*

An injectable matrix according to the present invention comprises rhCollagen as described herein, optionally in combination with one or more additional ECM components selected from hyaluronic acid, fibronectin, heparin, elastin, or laminin, and optionally further comprises a fat extract (e.g., an autologous fat extract), as described herein in the context of cells and/or adipose tissue. An autologous fat extract such as autologous stromal vascular fraction (SVF) (used in *in vitro* studies) or an autologous homogenized fat extract (used in *in vivo* studies) is optionally prepared and mixed with the matrix. In some embodiments, the autologous homogenized fat extract is minimally processed, as described in further detail hereinafter.

*Preparation of an autologous fat extract:*

As autologous stromal vascular fraction is prepared as generally depicted in FIG. 9. Briefly, autologous fat tissue was transferred to a 50 ml conical tube in ice cold PBS. The fat was washed by vigorously shaking the capped conical tube, and then transferred to a fresh tube. This washing step was repeated until the solution was clear. The fat tissue was then cut and trimmed into small pieces and transferred to a 50-ml centrifuge tube. 0.1 % collagenase solution (1 mg/ml) was added to the sample, and the tissue was incubated for 60 minutes at 37 °C under gentle shaking (60 cycles/minute). The digestion was neutralized by adding complete medium (CM) (aMEM with 10 % fetal bovine serum and P/S) to the tube, and then mixing by inversion. Materials were collected by centrifugation following which three layers were distinctly visible in the tubes. The uppermost layer contained fat and oil. The middle layer is an aqueous layer, which appeared red/clear. The stromal vesicular fraction (SVF) was present as a brownish pellet at the bottom of the tube, and was separated from the other layers. The SVF was re-suspended in PBS to remove collagenase, centrifuged at 700 x g for 5 minutes at room temperature, and again re-suspended in PBS and collected by centrifugation. The SVF pellet was re-suspended in 5 ml of PBS, filtered to filter out larger tissue particles, pipetted onto a 100 $\mu$m mesh filter placed on top of a new 50 ml centrifuge tube and allowed to filter by gravity flow. The filter was rinsed with 5 ml PBS and centrifuged at 280 x g for 5 minutes at room temperature. The supernatant was removed, and the pellet re-suspended in appropriate volume of CM. The obtained solution consists essentially of a single-cell suspension of SVF cells. The Trypan Blue exclusion method was used to count live cells.

An autologous homogenized fat extract was prepared in a similar manner to that shown in FIG. 9 and accompanying description, but the isolation procedure was stopped at mechanical mincing/homogenization of the fat pad, without enzymatic digestion, to thereby obtain a minimally processed extract.

*Matrix Loading:*

Fat extract was mixed with cross-linked fibrillar rhCollagen (e.g., EDC X-linked fibrillar rhCollagen such as X-Fb-rhCol-EDC20 as described in Example 4 below) to provide about 500 microliters in total of the fat extract and an rhCollagen-containing matrix for injection into the scaffold, as shown in FIG. 10. Loading of the scaffold was performed using a 1-ml syringe, with a 18G needle syringe.

In an exemplary matrix, a weight ratio of the rhCollagen and the fat extract was 1:3.

Using this methodology, a biocompatible, degradable scaffold was produced using 3D printing.

### Characterization:

Mechanical properties of implants prepared according to some embodiments of the present invention are determined using methods known and acceptable in the art, as exemplified in Background art FIGs. 1A-6, adopted from Brandon et al. (2019) Bioengineering (Basel).6(2):43.

Background Art FIGs. 1A-B show an example of a compression test between two parallel plates used for characterizing implant mechanical properties; Background Art FIG. 2 shows an exemplary graphic presentation of an analysis testing load as a function of projection strain. Background Art FIG. 3 shows an exemplary graphic presentation of an analysis testing load as a function of diametric strain; Background Art FIG. 4 shows an exemplary graphic presentation of an analysis testing load as a function of areal strain; Background Art FIG. 5 shows the set up to analyze localized strain at very low compressive loads ("Pinch" tests); and Background Art FIG. 6 shows an exemplary graphic presentation of results obtained in "Pinch" tests.

Further details are set forth in Example 3 hereinbelow.

### EXAMPLE 2

### Animal Model Studies

An exemplary general animal study is depicted in FIG. 11. 3D bio-printed, degradable breast implants are implanted into a rat model under different conditions, for example scaffolds with and without pre-loading or post injection of a matrix (SVF or fat extract in a rhCollagen based matrix). The implanted breast implants are then evaluated for safety and status at different time points.

More specifically, 3D bioprinted degradable scaffolds are printed using recombinant human collagen (rhCollagen) combined with biocompatible synthetic polymers as described herein. In one embodiment, the scaffold is generally shaped as a dome and includes injection ports, an inner cavity, and printed vascular network paths. The 3D bio-printed, degradable scaffolds is sterilized with EtO prior to being implanted.

    i. 3D bio-printed, degradable scaffolds are implanted in the backs of rats. 2-4 different implant configurations are evaluated, wherein each rat receives a single scaffold manipulated to match one of the configurations. The four possible configurations are: Empty 3D bio-printed scaffold - without injecting the internal matrix or homogenous fat cells;

    ii. 3D bio-printed scaffold injected with matrix but without the homogenous fat cells;

    iii. 3D bio-printed scaffold injected with matrix loaded with homogenous fat cells; and

    iv. Injection of the matrix and the homogenous fat cells without the 3D bio-printed scaffold.

Injection of matrix with or without homogenous fat cells occurs prior to or post implanting (see, FIG. 11).

The effect of the implantation on the rat's general health is evaluated, as well as its degradation over time and regeneration of tissue and cell distribution throughout the scaffold, using, for example, histological measurements and/or imaging.

*Preliminary study:*

A pilot animal study in a subcutaneous rat model was conducted. rhCollagen based 3D printed scaffold was loaded (by injection) with a filler comprising crosslinked fibrillar rhCollagen-EDC20 with or without a fat extract and the loaded scaffold was implanted in subcutaneous pockets created on a back of a rat, as depicted in FIGs. 12A-C.

The subcutaneous model in rats is a common model for estimating biocompatibility and regeneration potential of implants combined with artificial scaffold for soft tissue augmentation.

*Study Objectives:*

The primary objective of the study was to assess in a subcutaneous rat model the ability of the implant and the soft tissue filler matrix to sustain viability of fat extract and induce new fat tissue regeneration at the site of implantation. It included histology evaluation to assess tissue integration and tissue regeneration over time and fully functional neo-vascular network formation.

The primary endpoint was signs of tissue regeneration and tissue integration as well as vascularization of the implant/injectable soft filler site.

The secondary endpoint was the volume retention of the injectable soft fillers over time.

*Test articles and materials:*

The scaffold was preparing using a formulation comprised of methacrylated rhCollagen (0.5 %), PEGDA (0.5-2 %), SR9035 (1.0-2.5 %), HEAA (12.0-40.0 %), as described herein (see, Example 1, FIGs. 7 and 10). The obtained 3D-printed implants were sterilized with ETO.

20mM EDC X-linked fibrillar rhCollagen - X-Fb-rhCol-EDC20 was used as injectable filler.

The filler was divided into syringes, lyophilized and sterilized with ETO.

Fat extract for injection was isolated and prepared as described in Example 1 hereinabove at the day of the experiment. The compositions of the test articles are detailed in Table 1.

**Table 1**

| Implant type | Test group | Test article | Cellular component | Final rhCollagen composition | Final HA composition | Fat:filler ratio |
|---|---|---|---|---|---|---|
| | | | | **Matrix composition** | | |
| Acellular implant | Group A | #1 | N/A | 5 mg/ml | N/A | N/A |
| Full implant | Group B | #2 | Fat extract | 5 mg/ml | N/A | 3:1 |

*Study design:*

The experimental model was a subcutaneous implantation into the back of Sprague Dawley rats. Each of the Groups A and B was implanted with the 3D printed implant in 2 opposite subcutaneous pockets.

Overall, 6 animals were used for all the experimental groups. Animals were sacrificed 4 weeks following implantation/injection.

Additional 2 rats were sacrificed for fat graft isolation for implants of group B.

*Histology assessment:*

At sacrifice points, implantation sites were exposed and assessed macroscopically. The implants were excised, fixed in 4 % PFA and was subjected to histopathological assessment.

*Animal test system:*

Species/Strain: Hsd:Sprague Dawley®™SD®™
Source: Harlan Laboratories Israel, Ltd.
Sex: Female
Total No. of Animals: 6 for study + 2 for fat harvesting

Age: 10 weeks

Body Weight: about 230 grams at study initiation. Weight variation of animals at the time of treatment initiation did not exceed $\pm$ 20 % of the mean weight.

Animals Health: The health status of the animals used in this study is examined on arrival. Only animals in good health are acclimatized to laboratory conditions and are used in the study.

Acclimation: 7 days.

Housing: Animals were housed in IVC cages in dedicated HVAC (heat, Ventilation, Air Conditioning animal facility at temperature of 22 $\pm$ 2 °C and RH (Relative Humidity) of 55$\pm$15 %. Temperature and humidity were monitored continuously.

Diet and Water: Animals were provided ad libitum a commercial rodent diet (Harlan Teklad TRM Ra/Mouse Diet) and allowed free access to autoclaved water, supplied to each cage via polysulfone bottles with stainless steel sipper-tubes.

Environment: The facility had no exposure to outside light and was maintained on automatic alternating cycles of 12 hours light and 12 hours dark.

Identification: Each cage contained the study name, animal number and relevant details regarding treatment group.

Termination: At termination of the study animals were euthanized by $CO_2$ asphyxiation. Study procedure.

### Fat graft preparation:

At the day of the experiment fat tissue was collected from 5 rats by SIA. The fat was be kept sterile and transported on ice. Fat extract for injection was prepared in the biological safety hood. The fat tissue was divided into two 5-ml syringes and homogenized with additional syringe. After homogenization the syringes were centrifuged at 1000 g for 1 minute. Phase separation was clearly observed. The upper oily fraction was easily removed. Remained fat extract was mixed and divided into syringes, 3 ml/syringe to be mixed with rhCollagen filler (3:1).

### Analgesia and Anesthesia:

Animals were sedated using Isoflurane 2 % mixed in 3% $O_2$, shaved and disinfected with 70 % Ethanol.

### Implantation procedure:

Animals of group A and B were sedated and shaved, and a single midline incision was made over the spine of the rats. Subcutaneous pockets were created on both flanks and one scaffold was placed in each pocket. Prior to implantation the scaffolds were injected with rhCol-based matrix with (group B) and without (group A) fat extract.

Implant design and dimensions are as shown in FIGs 8A-D and described herein. Approximately 500 microliters of matrix was injected into the dome through one of the designated pores (see, FIG. 10) using 18G needle, as shown in FIG. 12A. Following the implantation (FIG. 12B), the wound was closed with clamps, as shown in FIG. 12C.

Each animal was treated with a maximum of 5 mg of rhCollagen.

### Post-operative Care:

All animals were observed for morbidity and mortality twice daily throughout the entire study period.

### Organ/Tissue Collection & Fixation:

At sacrifice point, implantation/injection sites were exposed and assessed macroscopically. The blebs and the implants were excised with the overlying skin, and fixed in 4 % PFA.

### Histological measurements:

The harvested/fixed samples were subjected to staining and histological assessment.

Paraffin blocks were sectioned at approximately 4 microns thickness. The sections were put on glass slides and stained with Hematoxylin & Eosin (H&E) for general histology and with Masson Trichrome (MT) for fibrosis.

Photographs were taken using microscope (Olympus BX60, serial NO. 7D04032) at objective magnifications of X1.25 and X10 and microscope's Camera (Olympus DP73, serial NO. OH05504). Photographs acquisition was performed only on pathological changes and of representative animals.

All slides were examined by one pathologist.

Grading system for histological evaluation (from ISO 10993 Part 6) was selected for a semi-qualitative evaluation.

### Results:

Implants from both groups remained intact structure following 4 weeks implantation. Large vasculature was observed in close proximity to the implants due to highly vascular implant sights

The major parameters extracted from the histological measurements are summarized in FIG. 13A. Inflammation score was calculated as a sum of scores for all inflammation cells evaluated (Polymorphonuclear cells (PMC), Lymphocytes, Plasma cells, Macrophages & Giant cells).

Representative histological images of both implant types are shown in FIGs. 13B-E.

As can be seen in FIGs. 13A-E, high tissue ingrowth was achieved inside the scaffold and between the strands in both groups. Tissue integration was low in both groups, possibly due to encapsulation and fibrosis. Necrosis was observed only at one single implantation site in group B, probably due to sample contamination. Fatty infiltrate was mild and insignificant. In terms of inflammation, 3D scaffold combined with rhCollagen alone (group A) displayed lower inflammation rates as indicated by the lower presence of mononuclear inflammatory cells.

Overall, this study demonstrated that the 3D printed scaffold showed promising results regarding tissue ingrowth and regeneration without major adverse tissue reactions in all study groups.

Specifically, the 3D printed scaffolds remained intact following 4 weeks of implantation; and good tissue ingrowth was achieved within the inner compartment and the printed strands in both groups.

### *EXAMPLE 3*

### *Implant Characterization and Design*

Even though the breast implants described herein are designed to degrade over time, and the implant inner cavity is replaced by newly formed tissue, there remains an extended timeframe when the implant resides within a subject prior to and during the degradation period. The mechanical properties of the implant play an important role in providing strength and durability of the degradable implants described herein, and in promoting tissue regeneration to replace the degradable implant with time.

Ideally, methodologies used for analyzing the mechanical properties of a scaffold/implant should simulate *in vivo* conditions or conditions as close as possible to *in vivo,* for an indicated tissue (a tissue to be augmented by the scaffold implant). Similarly, the scaffold should feature mechanical properties that efficiently promote tissue growth.

In some embodiments, mechanical properties to analyze include but are not limited to multi-dimensional strains and tangent moduli, shape stability, implant mobility, and fatigue failure characteristics. This information may provide a basis to show durability of an implant during compression *in vivo.* Information showing dimensional strains may be useful, as it describes the geometric or shape changes that may occur in response to compressive loading. Tangent modulus is useful in describing the behavior of materials that have been stressed beyond the elastic region. The tangent modulus quantifies the change that occurs as a material begins to yield to stresses and strains by either "softening" or "hardening". For example, one skilled in the art may use the tangent modulus to quantify the buckling failure of breast implants under expected "normal" stress conditions.

In some embodiments, mechanical properties of the scaffold may be analyzed using methods known in the art at this time. An example of methods for evaluation includes the methods described in Brandon et al., (2019) "New Evaluation Procedure for Multi-Dimensional Mechanical Strains and Tangent Moduli of Breast Implants: IDEAL IMPLANT® Structured Breast Implant Compared to Silicone Gel Implants" Bioengineering, 6:43. Brandon *et al., (ibid)* describes a series of mechanical analysis characterizing the mechanical properties of implants tested therein, for example, compression testing between two plates (FIGs. 1A and 1B) and localized strain ("pinching") at very low compressive loads (FIG. 5). The mechanical analysis described in Brandon et al., 2019 *(supra)* were carried out under both static and dynamic conditions, and with and without lubricant to test the difference with and without frictional forces, as described therein.

The multi-dimensional strain analysis of an implant is performed, for example but not limited to, by the method shown in FIGs. 1A-1B, which can provide results to determine projection strain, diametric strain, and areal strain.

Strain is the percent change in dimension of the implant in response to load, in this case compressive loads. The lower strain values correspond to greater shape stability of the implant. Using FIGs. 1A and 1B as a guide and the equations below, projection strain, diametric strain, and areal strain may be calculated.

$$Projection\ Strain = \in_H = -\frac{H - H_0}{H_0} \times 100\%$$

$$Diametric\ Strain = \epsilon_D = \frac{D - D_0}{D_0} \times 100\%$$

$$Areal\ Strain = \epsilon_A = \frac{A - A_0}{A_0} \times 100\%$$

$H_0$= Initial projection (space between plates); $D_0$ - Initial Diameter; $A_0$ - Initial Surface Area.

The results of compression testing in Brandon et al., 2019 (*supra*), are presented in FIGs. 2-4, and relate to the "high cyclic, low cyclic and occasional loads that a breast implant may be subjected to over its lifetime" (Brandon et al., 2019 *supra*). The dashed versus the solid lines show the difference between the dry and lubricated measurements. The friction provided by the plate used during the testing could influence the predictions for cyclic fatigue and strength.

While compression testing as shown in FIGs 1A and 1B measure the compression force over the total implant or large portion of the implant, the "pinch" testing implemented as shown in FIG. 5, tests the resistance of an implant to localized pinching or squeezing at the perimeter of an implant. Localized resistance demonstrates the free deformation response of an implant and simulates the tactile and palpable "softness" properties of an implant.

The results of the localized lateral compressive forces on breast implants are shown in FIG. 6, which presents the relative trends of different implants, with and without lubricant (dashed lines) in view of tactility, palpability, and softness.

In some embodiments, a degradable breast implant comprising a composite scaffold has a shape and mechanical properties that are minimally affected by compressive loading. In some embodiments, a degradable breast implant comprising a scaffold has a shape and mechanical properties that are minimally affected by localized compressive loads ("pinching", localized "squeezing"). In some embodiments, a degradable breast implant comprising a scaffold possesses a feel similar to a female breast. In some embodiments, a degradable breast implant comprising a scaffold possesses a softness similar to a female breast.

In some embodiments, the degradable implants disclosed herein have a projection strain, diametric strain, and areal strain within +/- 20% range of commercially available respective implants (e.g., breast implants).

In some embodiments, the degradable breast implants as disclosed herein have a projection strain, diametric strain, and/or areal strain each independently within +/- 20% range of the respective properties reported for an ideal breast implant as reported in Brandon et al., 2019 (*supra*).

### EXAMPLE 4

### *Filler compositions*

The following describes an exemplary soft tissue filler composition according to some embodiments of the present invention.

| Component | Example | Concentration |
|---|---|---|
| rhCollagen | As crosslinked fibrillar rhCollagen | 5 - 40 mg/ml |
| Nano particles (NPs) | As rhCollagen NPs or heat treated rhCollagen (rhGellatin) NPs | 2.5 - 20 mg/ml |
| ECM component | Hyaluronic acid, fibronectin, heparin, elastin, or laminin, or any combination thereof | 0.01 - 5 mg/ml |
| RGD-containing material (e.g., Cys-containing) | Stoichiometrically adjusted to rhCollagen | 1 - 120 mM |

An exemplary cross-linked fibrillar rhCollagen was prepared as followed:

### *Fibrillogenesis and washing buffers preparation:*

A stock solution of a fibrillogenesis buffer (FB) was prepared by adding 1,000 ml double distilled water (DDW) to 23 grams of Sodium phosphate dibasic (SPD), and thereafter filtering the resulting buffer through a 0.22 $\mu$m filter. pH was then adjusted to 11.2 by addition of 10N NaOH, and the obtained buffer was stored at 4 °C until used.

The washing buffer was prepared by mixing 100 ml of the FB, 9 ml HCl and 891 ml (by weight) DDW. pH was adjusted to 7.24 by addition of 10N NaOH, and the obtained buffer was stored at 4 °C until used.

### Fibrillogenesis of Collagen:

9 parts of thCollagen (about 3.1 mg/ml) were combined with 1 part of FB and the mixture was gently stirred for one hour at room temperature.

### X-linking of fibrillar collagen:

20 mM EDC were added to the fibrillar rhCollagen and the resulting mixture was gently stirred for two hours at room temperature, protected from light.

The mixture was thereafter centrifuged for 25 minutes at 4 °C, 10,000 rpm. The supernatant was discarded, an equivalent volume of the washing buffer was added, and the obtained mixture was shaken and centrifuged. This procedure was repeated three times. The obtained X-linked fibrillar rhCollagen was stored at 4 °C.

### In vitro Assay:

Aqueous formulations containing X-Fb Coll-EDC20 (5 mg/ml) and a formulation containing X-Fb Coll-EDC20 (5 mg/ml) mixed with HA Crosslinked with 20 % BDDE (1,4-butanediol diglycidyl) (10 mg/ml) were used.

The formulations were lyophilized and sterilized with ETO.

Prior to experiment the formulation were re-constituted with 1XDPBS.

The formulations were homogenized between two syringes.

100 $\mu$l of each formulation was placed in transwells in a 24-well plate.

1 ml of DMEM medium was added to the external part of the well.

P#8 nHDF cells were used.

100 $\mu$l of 100K cells/ml cell suspension was placed on top of each transwell.

100 $\mu$l of 100K cells/ml of cells seeded on top of the membrane of empty transwells without formulations were used as control.

Plates were incubated at 37 °C for 1 or 5 days.

At each timepoint WST assay was used to evaluate cell viability and proliferation. Cells with different concentrations for WST assay calibration curve were also seeded.

The obtained data is presented in FIG. 16. As can be seen, addition of rhCollagen-based fillers to the inserts exhibited beneficial effect on the cell growth.

An exemplary process of preparing rhCollagen-derived nanoparticles as described herein is as follows: rhCollagen is treated at 70 °C for 20 minutes to transform into rhGelatin. The obtained rhGelatin (3 grams) is dissolved in 200 ml DI water at 40 °C with vigorous stirring. Once the gelatin is dissolved, the pH of the solution is adjusted to pH 2.5, using concentrated HCl (10 M or higher). Acetone (600 ml) is thereafter added at a rate of 5 ml/minute. The amount of acetone can be adjusted as needed, until a milky white mixture is obtained. Then 1 ml of 25 % glutaraldehyde solution is added, and the obtained mixture is stirred overnight while being illuminated, at room temperature. The resulting reaction mixture is ultracentrifuged at 15,000 g for 15 minutes, or until the obtained nanoparticles precipitate into a pellet.

The pellet is then re-dispersed in 50 ml (or more) of 75 % acetone in water solution to wash the nanoparticles, optionally along with sonication, to assure the formation of a thorough dispersion. The centrifugation and re-dispersion is repeated 3 times.

Then, the solution is transferred to a 200 ml round bottom flask and the acetone is removed by evaporation or distillation, until the volume is reduced by about 75 %. The pH of the obtained solution is adjusted to neutral using NaOH, and the solution is transferred to a conical tube, and lyophilized until dried. The obtained particles are stored in an air/water tight container at room temperature until used.

## Claims

1. A three-dimensional (3D) biocompatible, degradable soft tissue implant comprising a bio-printed composite scaffold being composed of:

   a collagen which comprises cured recombinant human collagen (rhCollagen) and a biocompatible synthetic polymer,
   wherein the scaffold comprises:

a porous wall;
an inner cavity at least partially enclosed within the porous wall; and
at least one injection port that connects the inner cavity with an outer most surface of the scaffold, wherein said injection port has an opening sized to permit insertion of an injection device through said port, wherein the implant further comprises an injectable matrix which comprises rhCollagen which does not include curable groups within the inner cavity of the scaffold.

2. The soft tissue implant of claim 1, wherein said scaffold further comprises at least one printed vascular network path that connects the outer most surface of the scaffold with the inner cavity of the scaffold, wherein said vascular network path is sized to permit entry of vascular cells and tissues.

3. The soft tissue implant of claim 1 or 2, wherein said inner cavity comprises from 2 to 30 chambers, and wherein optionally, at least two of said chambers are interconnected to one another.

4. The soft tissue implant of any one of claims 1 to 3, wherein said bio-printed composite scaffold is formed by bio-printing a curable formulation in a configured pattern which corresponds to a desired shape and dimension of the soft tissue implant, wherein said biocompatible synthetic polymer features a curable moiety.

5. The soft tissue implant of any one of claims 1 to 4, wherein said rhCollagen comprises a plant-derived recombinant human collagen.

6. The soft tissue implant of any one of claims 1 to 5, wherein said scaffold further comprises at least one extracellular matrix (ECM) component and/or an integrin-binding material.

7. The soft tissue implant of any one of claims 1 to 6, wherein said matrix further comprises

at least one extracellular matrix (ECM) component; and
cells or adipose tissue,
and optionally further comprises an integrin-binding material.

8. The soft tissue implant of claim 7, wherein said cells comprise a stromal vascular fraction (SVF) isolated from a fat tissue and/or a minimally processed extract from a fat tissue.

9. The soft tissue implant of any one of claims 1-8, comprising a smooth surface.

10. A method of preparing the implant of any one of claims 1 to 8, the method comprising sequentially forming a plurality of layers in said configured pattern of said scaffold,

wherein at least a portion of said layers, is formed of a formulation that comprises said recombinant human collagen featuring at least one curable group, a synthetic polymer featuring at least one curable group, and optionally an ECM component featuring a curable group and/or an integrin-binding material featuring at least one curable material,
wherein the method optionally further comprises injecting the matrix into at least within said inner cavity of said scaffold, via said injection port,.

11. A kit comprising:

at least one curable formulation for forming a scaffold comprising a collagen which comprises a curable recombinant human collagen (rhCollagen) and a biocompatible synthetic polymer that optionally features a curable group; and
an injectable matrix formulation for forming a matrix of the scaffold which consists of rhCollagen which does not include curable groups, the kit being identified for use in preparing a bioprinted soft tissue implant.

12. The soft tissue implant of any one of claims 1 to 8, for use in augmenting and/or reconstructing and/or regenerating a soft tissue in a subject in need thereof.

**Patentansprüche**

1. Dreidimensionales (3D) biokompatibles, abbaubares Weichgewebeimplantat, umfassend ein biologisch bedrucktes Verbundgerüst, bestehend aus:

   einem Kollagen, das gehärtetes rekombinantes menschliches Kollagen (rhCollagen) und ein biokompatibles synthetisches Polymer umfasst,
   wobei das Gerüst Folgendes umfasst:

   eine poröse Wand;
   einen inneren Hohlraum, der zumindest teilweise in der porösen Wand eingeschlossen ist; und
   mindestens einen Injektionsanschluss, der den inneren Hohlraum mit einer äußersten Oberfläche des Gerüsts verbindet, wobei der Injektionsanschluss eine Öffnung aufweist, die so bemessen ist, dass sie das Einführen einer Injektionsvorrichtung durch den Anschluss ermöglicht,
   wobei das Implantat ferner eine injizierbare Matrix umfasst, die rhCollagen umfasst, das keine härtbaren Gruppen innerhalb des inneren Hohlraums des Gerüsts einschließt.

2. Weichgewebeimplantat nach Anspruch 1, wobei das Gerüst ferner mindestens einen bedruckten Gefäßnetzwerkpfad umfasst, der die äußerste Oberfläche des Gerüsts mit dem inneren Hohlraum des Gerüsts verbindet, wobei der Gefäßnetzwerkpfad so bemessen ist, dass er den Eintritt von Gefäßzellen und -geweben ermöglicht.

3. Weichgewebeimplantat nach Anspruch 1 oder 2, wobei der innere Hohlraum 2 bis 30 Kammern umfasst und wobei optional mindestens zwei der Kammern miteinander verbunden sind.

4. Weichgewebeimplantat nach einem der Ansprüche 1 bis 3, wobei das biologisch bedruckte Verbundgerüst durch biologisches Drucken einer härtbaren Formulierung in einem konfigurierten Muster gebildet wird, das einer gewünschten Form und Abmessung des Weichgewebeimplantats entspricht, wobei das biokompatible synthetische Polymer eine härtbare Einheit aufweist.

5. Weichgewebeimplantat nach einem der Ansprüche 1 bis 4, wobei das rhCollagen ein von Pflanzen stammendes rekombinantes menschliches Kollagen umfasst.

6. Weichgewebeimplantat nach einem der Ansprüche 1 bis 5, wobei das Gerüst ferner mindestens eine Komponente der extrazellulären Matrix (ECM) und/oder ein Integrin-bindendes Material umfasst.

7. Weichgewebeimplantat nach einem der Ansprüche 1 bis 6, wobei die Matrix ferner mindestens eine Komponente der extrazellulären Matrix (ECM); und

   Zellen oder Fettgewebe umfasst,
   und optional ferner ein Integrin-bindendes Material umfasst.

8. Weichgewebeimplantat nach Anspruch 7, wobei die Zellen eine stromale Gefäßfraktion (SVF), die aus einem Fettgewebe isoliert ist, und/oder einen minimal verarbeiteten Extrakt aus einem Fettgewebe umfassen.

9. Weichgewebeimplantat nach einem der Ansprüche 1-8, umfassend eine glatte Oberfläche.

10. Verfahren zur Herstellung des Implantats nach einem der Ansprüche 1 bis 8, wobei das Verfahren das sequentielle Bilden einer Vielzahl von Schichten in dem konfigurierten Muster des Gerüsts umfasst,

    wobei mindestens ein Abschnitt der Schichten aus einer Formulierung gebildet ist, die das rekombinante menschliche Kollagen mit mindestens einer härtbaren Gruppe, ein synthetisches Polymer mit mindestens einer härtbaren Gruppe und optional eine ECM-Komponente mit einer härtbaren Gruppe und/oder ein Integrin-bindendes Material mit mindestens einem härtbaren Material umfasst,
    wobei das Verfahren optional ferner das Injizieren der Matrix in mindestens den inneren Hohlraum des Gerüsts über den Injektionsanschluss umfasst.

11. Kit, umfassend:

mindestens eine härtbare Formulierung zum Bilden eines Gerüsts, umfassend ein Kollagen, das ein härtbares rekombinantes menschliches Kollagen (rhCollagen) und ein biokompatibles synthetisches Polymer umfasst, das optional eine härtbare Gruppe aufweist; und

eine injizierbare Matrixformulierung zum Bilden einer Matrix des Gerüsts, die aus rhCollagen besteht, das keine härtbaren Gruppen einschließt, wobei der Kit zur Verwendung bei der Herstellung eines biologisch bedruckten Weichgewebeimplantats identifiziert wird.

**12.** Weichgewebeimplantat nach einem der Ansprüche 1 bis 8 zur Verwendung bei der Verstärkung und/oder Rekonstruktion und/oder Regeneration eines Weichgewebes bei einem Subjekt, der dies benötigt.

**Revendications**

**1.** Implant tridimensionnel (3D) biocompatible, dégradable de tissu mou, comprenant un échafaudage composite bio-imprimé étant composé :

d'un collagène qui comprend du collagène humain recombiné durci (rhCollagène) et un polymère synthétique biocompatible,
dans lequel l'échafaudage comprend :

une paroi poreuse ;
une cavité interne au moins partiellement renfermée dans la paroi poreuse ; et
au moins un orifice d'injection qui relie la cavité interne à une surface la plus extérieure de l'échafaudage, dans lequel ledit orifice d'injection présente une ouverture dimensionnée pour permettre l'insertion d'un dispositif d'injection à travers ledit orifice,
dans lequel l'implant comprend en outre une matrice injectable qui comprend du rhCollagène qui n'inclut pas de groupes durcissables dans la cavité interne de l'échafaudage.

**2.** Implant de tissu mou selon la revendication 1, dans lequel ledit échafaudage comprend en outre au moins une voie de réseau vasculaire imprimée qui relie la surface la plus extérieure de l'échafaudage à la cavité interne de l'échafaudage, dans lequel ladite voie de réseau vasculaire est dimensionnée pour permettre l'entrée de cellules et de tissus vasculaires.

**3.** Implant de tissu mou selon la revendication 1 ou 2, dans lequel ladite cavité interne comprend de 2 à 30 chambres, et dans lequel, éventuellement, au moins deux desdites chambres sont interconnectées l'une à l'autre.

**4.** Implant de tissu mou selon l'une quelconque des revendications 1 à 3, dans lequel ledit échafaudage composite bio-imprimé est formé par bio-impression d'une formulation durcissable dans un motif configuré qui correspond à une forme et à une dimension souhaitées de l'implant de tissu mou, dans lequel ledit polymère synthétique biocompatible présente un fragment durcissable.

**5.** Implant de tissu mou selon l'une quelconque des revendications 1 à 4, dans lequel ledit rhCollagène comprend un collagène humain recombiné dérivé d'une plante.

**6.** Implant de tissu mou selon l'une quelconque des revendications 1 à 5, dans lequel ledit échafaudage comprend en outre au moins un composant de matrice extracellulaire (ECM) et/ou un matériau de liaison à l'intégrine.

**7.** Implant de tissu mou selon l'une quelconque des revendications 1 à 6, dans lequel ladite matrice comprend en outre au moins un composant de la matrice extracellulaire (ECM), et

des cellules ou du tissu adipeux,
et éventuellement comprend en outre un matériau de liaison à l'intégrine.

**8.** Implant de tissu mou selon la revendication 7, dans lequel lesdites cellules comprennent une fraction vasculaire stromale (SVF) isolée à partir d'un tissu adipeux et/ou un extrait ayant subi un traitement minimal à partir d'un tissu adipeux.

**9.** Implant de tissu mou selon l'une quelconque des revendications 1-8, comprenant une surface lisse.

**10.** Procédé de préparation de l'implant selon l'une quelconque des revendications 1 à 8, le procédé comprenant la formation séquentielle d'une pluralité de couches dans ledit motif configuré dudit échafaudage, dans lequel au moins une partie desdites couches est formée d'une formulation qui comprend ledit collagène humain recombiné présentant au moins un groupe durcissable, un polymère synthétique présentant au moins un groupe durcissable et, éventuellement, un composant ECM présentant un groupe durcissable et/ou un matériau de liaison à l'intégrine présentant au moins un matériau durcissable,

dans lequel le procédé comprend éventuellement en outre l'injection de la matrice au moins dans ladite cavité interne dudit échafaudage, via ledit orifice d'injection.

**11.** Kit, comprenant :

au moins une formulation durcissable pour former un échafaudage comprenant un collagène qui comprend un collagène humain recombiné durcissable (rhCollagène) et un polymère synthétique biocompatible qui présente éventuellement un groupe durcissable ; et

une formulation de matrice injectable pour former une matrice de l'échafaudage qui consiste en du rhCollagène qui n'inclut pas de groupes durcissables, le kit étant identifié pour être utilisé dans la préparation d'un implant de tissu mou bio-imprimé.

**12.** Implant de tissu mou selon l'une quelconque des revendications 1 à 8, destiné à être utilisé pour augmenter et/ou reconstruire et/ou régénérer un tissu mou chez un sujet qui en a besoin.

FIG. 1A
(Background Art)

FIG. 1B
(Background Art)

FIG. 2
(Background Art)

FIG. 3
(Background Art)

FIG. 4

(Background Art)

FIG. 5
(Background Art)

FIG. 6
(Background Art)

Breast scaffold printing

Loading matrix with ECM components + autologous fat cells

Implantation and vascularization

Implant replacement by newly formed tissue

FIG. 7

Cross-section

FIG. 8A

Top view

FIG. 8B

Side view

FIG. 8C

FIG. 8D

Adult rat ⟹ Fat pad isolation ⟹ Mechanical mincing of the fat pad ⟹ Enzymatic digestion

Incubation

Removal of the supernatant fat layer

Centrifugation

Components of SVF pellet ⟸ SVF pellet

Pericytes

Adipose Derived Stem Cells

Pre-Adipocytes

Endothelial & Progenitor Cells

Haematopoetic Cells Monocytes/Macrophages

FIG. 9

EP 4 126 088 B1

FIG. 10

Mixture of SVF
in rhCollagen
based matrix

3D printed
dome shape
scaffold

Scaffold
implantation and
matrix injection

Implant harvest

Histological evaluation
for safety and efficacy

FIG. 11

FIG. 12A

Implant harvest

Histological evaluation
for safety and efficacy

FIG. 12B

FIG. 12C

FIG. 13A

| GROUP A: loaded with - Xlinked-Fibrillar-rhCollagen-EDC20 | | GROUP B: loaded with - Homogenized Fat: [Xlinked-Fibrillar-rhCollagen-EDC20] (3:1) | |
|---|---|---|---|
| Animal 1-R Group A X1.25 H&E | Animal 1-R Group A X10 MT | Animal 5-L Group B X1.25 H&E | Animal 5-L Group B X10 MT |
| Animal 1-R Group A X10 H&E | Animal 1-R Group A X10 MT | Animal 5-L Group B X10 H&E | Animal 5-L Group B X10 MT |

FIG. 13B

FIG. 13C

FIG. 13D

H&E x10                                    MT x10

FIG. 13E

## FIG. 14A

## FIG. 14B

## FIG. 14C

## FIG. 14D

10 mm

12

10

5 mm

Side views

12

10

Top view

12

10

16

10 mm

10 mm

FIG. 15C

FIG. 15B

FIG. 15A

# WST-1 Assay

FIG. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62992998
- WO 2006035442 A
- WO 2009053985 A
- WO 2011064773 A
- WO 2013093921 A
- WO 2014147622 A
- WO 2018225076 A
- WO 2019211854 A
- US 5591444 A
- US 7723108 B
- US 7745105 B
- US 7919112 B
- US 8025869 B
- US 8038665 B
- US 8066691 B
- US 8124120 B
- US 8142815 B
- US 8192487 B
- US 8435600 B
- US 8546142 B
- US 8702684 B
- US 8641775 B
- US 8642735 B
- US 8697059 B
- US 8992551 B
- US 8758781 B
- US 8778333 B
- US 8778909 B
- US 8871267 B
- US 9101692 B
- US 9074190 B
- US 9173975 B
- US 9150668 B
- US 9289533 B
- US 9956317 B
- US 9681941 B
- US 9752138 B
- US 9744260 B
- US 9782517 B
- US 9801976 B
- US 9901440 B
- US 9913705 B
- US 9956072 B
- US 10011820 B
- US 10258588 B
- US 10117822 B
- US 10039633 B
- US 10300169 B
- US 10335190 B
- US 10327884 B
- US 10449034 B
- US 10471181 B
- DE 102011121982
- RU 2675019
- EP 2995278 A
- EP 3013379 A
- EP 2231061 A
- EP 1280562 A
- EP 3247413 A
- EP 1814606 A
- EP 2550028 A
- EP 2841115 A
- EP 1734894 A
- EP 3191020 A
- EP 1546307 A
- EP 3357519 A
- US 20170274052
- US 20110274666 A
- US 20180064854 A
- US 20200078411 A
- US 20200030495 A
- US 20190321158 A
- US 20180177917 A
- US 20190184064 A
- US 20170071725 A
- US 20200030496 A
- US 20190060516 A
- US 20200016191 A
- US 20190374457 A
- US 20180193522 A
- US 20180098836 A
- US 20170224896 A
- US 20190134265 A
- US 20170087273 A
- US 20180289860 A
- US 20180015204 A
- US 20200268503 A
- US 20210030528 A
- WO 9706791 A
- US 5773412 A
- US 20190022017 A

**Non-patent literature cited in the description**

- **HÖLMICH et al.** *Untreated Silicone Breast Implant Rupture Plastic and Reconstructive Surgery.*, 2004, vol. 114 (1), 204-214
- **KATZIN et al.** *Pathology of Lymph Nodes from Patients with Breast Implants: A Histologic and Spectroscopic Evaluation American Journal of Surgical Pathology.*, 2005, vol. 29 (4), 506-11
- **SWERDLOW et al.** The 2016 revision of the World Health Organization classification of lymphoid neoplasms.. *Blood*, 2016, vol. 127 (20), 2375-2390
- **KADLER K.** *Birth Defects Res C Embryo Today.*, 2004, vol. 72, 1-11
- **KADLER KE** ; **BALDOCK C** ; **BELLA J** ; **BOOT-HANDFORD RP**. *J Cell Sci.*, 2007, vol. 120, 1955-1958
- **KREGER ST.** *Biopolymers.*, 2010, vol. 93 (8), 690-707
- **ATALA A**. *Nature Biotechnology*, 2014, vol. 32 (8)
- **MILLER JS** ; **BURDICK J.** *ACS Biomater. Sci. Eng.*, 2016, vol. 2, 1658-1661
- **ISAACSON et al.** *Experimental Eye Research*, 2018, vol. 173, 188-193
- **DRZEWIECKI et al.** A thermoreversible, photocrosslinkable collagen bio-ink for free-form fabrication of scaffolds for regenerative medicine. *Technology*, 2017
- **DRZEWIECKI, K. E. et al.** *Langmuir*, 2014, vol. 30, 11204-11211
- **GAUDET, I. D.** ; **SHREIBER, D. I.** *Biointerphases*, 2012, vol. 7, 25
- **STEIN H**. *Biomacromolecules*, 2009, vol. 10, 2640-5
- **CHHAYA MOHIT P. et al.** Transformation of Breast Reconstruction via Additive Biomanufacturing. *SCIENTIFIC REPORTS*, 01 September 2016, vol. 6 (1), 28030, https://www.nature.com/articles/s-rep28030.pdf
- **BRANDON et al.** *Bioengineering (Basel).*, 2019, vol. 6 (2), 43
- **BRANDON et al.** *Bioengineering (Basel)*, 2019, vol. 6 (2), 43
- **HAUBNER et al.** *J. Am. Chem. Soc.*, 1996, vol. 118, 7881-7891
- **CAPELLO et al.** *J. Nucl. Med.*, 2004, vol. 45 (10), 1716-20
- **BRANDON et al.** New Evaluation Procedure for Multi-Dimensional Mechanical Strains and Tangent Moduli of Breast Implants: IDEAL IMPLANT® Structured Breast Implant Compared to Silicone Gel Implants. *Bioengineering*, 2019, vol. 6, 43